# EUROPEAN PATENT APPLICATION

(11) **EP 1 295 893 A1**
(43) Date of publication of application: **26.03.2003**
(21) Application number: 00953510.5
(22) Date of filing: 18.08.2000
(51) Int. Cl.: C07J 17/00

(54) **SKIN TISSUE REGENERATION PROMOTERS COMPRISING GINSENOSIDE Rb1**

(30) Priority: 31.05.2000 JP 2000163026
(71) Applicant: JAPAN SCIENCE AND TECHNOLOGY CORPORATION, Kawaguchi-shi, Saitama 332-0012 (JP)
(72) Inventor: SAKANAKA, Masahiro, Onsen-gun, Ehime 791-0204 (JP); HASHIMOTO, Koji, Onsen-gun, Ehime 791-0314 (JP); TANAKA, Junya, Onsen-gun, Ehime 791-0203 (JP); NAKATA, Kimihiko, Iyo-shi, Ehime 799-3111 (JP)
(74) Representative: Cresswell, Thomas Anthony
(86) International application number: JP0005554
(87) International publication number: WO01092289

(57) **Abstract**

Efficacious intravenous preparations, skin preparations for external use, mucosal preparations for external use or cosmetics comprising ginsenosides, in particular, ginsenoside Rb or its derivative which are useful as skin tissue regeneration/reconstruction promoters or wound healing promoters; and fertilizer additives comprising ginsenosides, in particular, ginsenoside Rb or its derivative which are useful as plant tissue regeneration/reconstruction promoters. These intravenous preparations, skin preparations for external use, mucosal preparations for external use or cosmetics are useful particularly in promoting the tissue regeneration/reconstruction in incised wounds, open wounds, bite wounds and missing of skin or mucosa or in promoting would healing. The above fertilizer additives are useful particularly in water culturing and raising crops.

## Description

### Technical Field

The present invention relates to a pharmaceutical composition(s) or a veterinary drug composition(s) for prevention, treatment or therapy of organic diseases with histopathological changes of the organic, living or viable tissues, more particularly for diseases caused by damage, injury, trauma or deficit of skin tissues or mucosal tissues, and further more particularly for promotion of regeneration or reconstruction of the skin tissues or the mucosal tissues, or promotion of wound healing, comprising ginsenosides such as ginsenoside Rb₁, metabolites thereof or salts thereof. The present invention further relates to ginsenosides, metabolites thereof or salts thereof useful as promoters of skin tissue regeneration, reconstruction and/or wound healing.

The present invention also relates to a preparation(s) for intravenous administration or a preparation(s) for external use of skin or for topical application to skin comprising a composition(s) for prevention, treatment or therapy of the said aiseases. The present invention further relates to the use of ginsenosides such as ginsenoside Rb₁ or metabolites thereof as a leading compound for exploring novel useful compounds for prevention, treatment or therapy of skin tissue diseases or for exploring promotors of skin tissue regeneration or wound healing.

The present invention relates to a composition(s) for external use of skin or for topical application to skin such as a composition(s) for cosmetics and a composition(s) for hair growth, hair raising and hair nourishment useful for prevention, treatment and/or improvement of senile symptoms of the skin comprising ginsenosides such as ginsenoside Rb₁, metabolites thereof or salts thereof.

The present invention further relates to a composition(s) for promoting regeneration, generation or reconstruction of plant or animal tissues, more particularly a composition(s) for growth regulation comprising ginsenosides such as ginsenoside Rb₁, metabolites thereof or salts thereof. The composition(s) for growth regulation of the present invention is useful as a fertilizer composition(s) and feed composition(s).

Further, the present invention relates to a method for exploring substances for promoting regeneration, generation, rooting, budding, growth, differentiation or reconstruction of living, vital or viable tissues comprising assaying protective actions of intracerebroventricularly administered substances to be tested on brain cells or nerve cells.

### Background Art

It is known that damage or deficit of skin and mucosa, such as injury, wound, morsus, scald, burn, congelation, radiation injury, ultraviolet irradiation, electric injury, traumatic injury, skin ulcer, bedsore and bullous skin diseases, causes degenerative exfoliation, necrosis, apoptosis or apoptosis-like cell death of skin tissue-composing cells or mucosal tissue-composing cells. As for effective methods for prevention or therapy of diseases caused by mechanical or physical damage or defect of the skin tissue or the mucosal tissues, administration of a drug(s), which can rapidly regenerate and/or reconstruct the degenerated and defected skin tissues, mucosal tissues and composing cells thereof and promote wound healing, is considered.

Regeneration and/or reconstruction of tissues will be simply explained by exemplifying the skin tissue in the following. Generally, if a part of the skin tissues is eliminated or defected by disease or traumatic injury, epidermal cells surviving in the periphery of the defected skin tissue divide, proliferate and move to the defected skin region. In this specification, this phenomenon is defined, in the narrow sense, as regeneration of epidermal cells or epidermal tissues. Thereafter, the regenerative epidermal cells, which have moved or are moving to the defected skin region, are adhered each other to terminate division and proliferation, as a result, the epidermal tissues are reconstructed. This phenomenon is, in this specification, defined as a reconstruction of epidermal cells or epidermal tissues (epidermis). As like this, the vital phenomenon, in which regeneration and reconstruction of the epidermal cells or the epidermal tissues (epidermis) occur, is generally called epithelization or epidermization. Most of the epidermal cells in the epidermal tissues are epidermal keratinocytes, cornified cells, keratinized cells or hornified cells. Although numbers of cells are few, admixing with the epidermal keratinocytes, cornified cells, keratinized cells and the hornified cells, there are melanocytes (melanin pigment-generating cells), Merkel cells (cells involved in skin sensation), Langerhans cells (cells involved in the skin immune system, especially antigen presentation to lymphocytes), stem cells (cells which can differentiate to all cell species), cells of sweat glands differentiated from the epidermal cells, cells of sebaceous glands differentiated from the epidermal cells, cells of hair follicles differentiated from the epidermal cells, etc. In response to the epidermization or epithelization, in case that the skin tissues are injured or in case of skin diseases, these cell species are also incorporated into the epidermal tissues (or designated as the epidermis), or they move to the deep regions of the skin (dermis and subcutaneous tissue) while maintaining connections with the epidermal tissues to form appendages of the skin (sweat glands, sebaceous glands, hair follicles, etc.), through the complex processes such as division, proliferation, adhesion or differentiation. In order to regenerate and/or reconstruct the skin tissues, all the epidermis-derived cells and the appendages of the skin such as melanocytes, Merkel cells, Langerhans cells, stem cells, sweat glands or cells of sweat glands, sebaceous glands or cells of sebaceous glands, hair follicles or cells of hair follicles, etc. should be regenerated and reconstructed. On the other hand, in the phenomena of regeneration and/or reconstruction of the dermis and the subcutaneous tissue, the fibroblasts and the blood vessels play the central role. The fibroblasts, like the epidermal cells, generate and excrete (regenerate) collagen fibers, elastic fibers, reticular fibers and various extracellular matrix components. In addition, if these excreted components are not reconstructed very regularly and systematically up to the condition close to the healthy dermis and subcutaneous tissue, namely if the fibroblasts, collagen fibers, elastic fibers, reticular fibers and various extracellular matrix components in the dermis and subcutaneous tissue can not certainly and rapidly be regenerated and/or reconstructed, the regeneration and/or reconstruction of the skin tissues can not be completed. In other words, in the phenomena of regeneration and reconstruction of the dermis and the subcutaneous tissue, roles which the fibroblasts play are extremely important. Consequently, suppressing division, proliferation, movement and/or differentiation of the fibroblasts, or suppressing generation of collagen fibers, elastic fibers, reticular fibers and/or various extracellular matrices should be avoided for smoothly proceeding the regeneration and/or reconstruction of the skin tissues. With regard to the important role of blood vessels, when the regeneration and reconstruction of skin tissues are actively performed, the regeneration and/or generation of blood vessels (including vascular endothelial cells, vascular smooth muscle cells and fibroblasts in vascular tunica media and vascular tunica externa) ruptured or broken by skin injuries should actively occur, but when the regeneration and reconstruction of the skin tissue are completed, the reconstruction of blood vessels should be completed by the action of regulatory mechanisms wherein the excess blood vessels are degenerated and the necessary blood vessels remain. In addition, in case of damage, injury or disease of the skin, the peripheral nerves and the corpuscular nerve receptors (Merkel's corpuscle, Pacini's corpuscle, etc.), which are distributed in the skin with damage, injury or disease, are cut or destroyed, however, it is important from the standpoint of maintaining function of the regenerated skin tissue to simultaneously regenerate and/or reconstruct these nervous tissues.

As explained hereinabove, the vital phenomenon such like regeneration or reconstruction of the skin tissue can not proceed successfully, unless the above-described extremely complex vital phenomenon can proceed regularly well in the order. Examples of molecular group involved in the vital phenomena are EGF, TGF-β₁, TGF-α, FGF, VEGF, PDGF-BB, TGF-β₁, PDGF-AB, IGF, KGF, PDGF, TGF-β₂, TGF-β₆, FGF-2, U-PA, t-PA, integrins, adhesion factors, etc. (Singer, A. J. and Clark, R. A. F. New Engl. J. Med., 341, 738-746, 1999). It is said that blood cell components or plasma components extravascularly leaked out in cases of damage or injuries to the skin tissue play important roles.

Consequently, as described hereinabove, the vital phenomena of regeneration and reconstruction of the skin tissue are very complex and the involved cell species, blood vessels, nerves and molecular groups are immeasurable. It has been thought to be impossible that such complex vital phenomena are regulated and controlled by one compound to promote the regeneration and/or reconstruction of tissue rapidly and exactly. It has been known that local spreading or local spraying of basic fibroblast growth factor (bFGF) or platelet-derived growth factor (PDGF) could promote partial regeneration and/or reconstruction of the skin tissue to exhibit effect and efficacy for skin ulcer and bedsore (decubitus), but these effects could not be satisfactory from the clinical standpoint (Singer, A. J. and Clark, R. A. F. New Engl. J. Med., 341, 738-746, 1999). The above-mentioned peptide factors (bFGF, PDGF) are the pharmaceutical compositions which can only be applicable with local spreading and local spraying, and no effectiveness or efficacy can be expected by the systemic administration such as intravenous administration. Consequently, in order to promote regeneration and/or reconstruction of the skin tissue, mucosal tissues, extremities, visceral organs of head, neck, abdomen, and thorax and extracutaneous tissues with damage, injury or defect, an invention relevant to intravenously administrable skin tissue regeneration promoters and to a pharmaceutical composition(s) or a veterinary drug composition(s) for promoting regeneration of the visceral organs or tissues is required. Further, a superior composition(s) or preparation(s) for external use of skin or topical application to skin, a superior composition(s) or preperation(s) for external or topical application to mucosa for prevention, treatment or therapy of the above-mentioned diseases or a superior cosmetic composition(s) or a health-promoting drug composition(s) for prevention, improvement or treatment of senile symptoms of skin or mucosa (dermatrophia, shinkage or atrophy of the skin, easy infectivity, vulnerability to infection, slackening, loosening, flabbiness, dandruff, scurf, depilation, alopecia, poliosis, gray hair, itching, dry skin, roughness, oligosteatosis, asteatosis, ablation or exfoliation of keratinocytes, cornified cells, keratinized cells, hornified cells or the stratum corneum, rhagade, crack, chapped, ephelis, spot, blotch, wrinkle, line, furrow, freckle, pigmentation, dryness, etc. or shrinkage or atrophy of mucosa, chapped, rhagade, crack, aplasia, poor regeneration, dryness, etc.) is also required. Further, in addition to the pharmaceutical composition(s) for promoting regeneration or reconstruction of animal tissues hereinbefore, a composition(s) for growth-regulation or a fertilizer additive(s) for the promotion of rooting, budding, growth, differentiation, regeneration, generation or reconstruction of plant tissues is also essentially required for cultivation of the crops, hydroponic culture, cultivation of vegetables, cultivation of fruits, growing mushroom, cultivation of natural plants, preservation of fresh flower, cultivation of tobacco, cultivation of medicinal plants, improvement of plants and cultivation of tea-leaves.

Ginsenoside Rb₁ is a compound represented by the following formula: 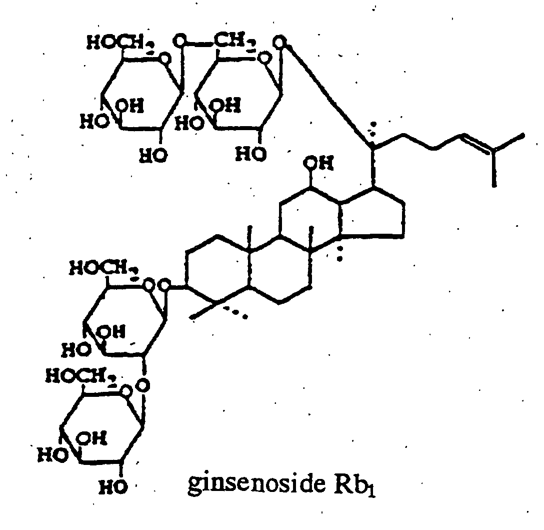 and ginsenoside Rb₁ is a known compound by Shibata et al. (Shibata, S. et al., Economic and medicinal plant research, world Scientific, Philadelphia, pp 217-284, 1985).

In Japanese Patent Appln. No. Hei 10-365560 (Brain cell or nerve cell-protective agents comprising ginsenoside Rb₁), one of inventors of the present invention (Sakanaka) invented that intravenous continuous administration of low dosages of ginsenoside Rb₁ reduced a volume of cerebral infarct lesion to about 1/4 of the non-administered group as a result of inhibiting apoptosis-like nerve cell death. Namely, Sakanaka et al. invented that in case of the extracellular fluid concentrations of ginsenoside Rb₁ in lesioned tissue at 1 ng/ml or less, preferably 10 pg/ml or less, more preferably 100 fg/ml or less, the expression of a cell death-suppressing gene product Bcl-x_{L} is promoted and apoptosis or apoptosis-like cell death is suppressed to exhibit a cytoprotective action. However, in the invention hereinabove, it was not elucidated whether or not low concentrations of ginsenoside Rb₁ promoted the regeneration of once dead cells or degenerated and eliminated tissues. Further, a phenomenon, in which tissues degenerated and eliminated due to traumatic injury or damage are recovered to nearly normal condition as a result of division, proliferation, migration, differentiation, etc. of the living or viable cells present in the eliminated tissue penumbra, is called the tissue regeneration and/or reconstruction. It has not been paid attention whether or not intravenous continuous administration or local administration to lesion of ginsenoside Rb₁ at low concentrations and low dosages can promote the tissue regeneration or reconstruction.

In U.S. Patent 5,663,160, it is described that a high extracellular fluid concentration of ginsenoside Rb₁ (100 µg/ml) can promote division and proliferation of skin epidermal keratinocytes or hornified cells and is effective for hair growth and hair nourishment, protection of skin, humectant action for skin, regeneration of epidermis and suppression of wrinkle. In WO 99/07338, it is described that ginsenoside Rb₁ at the extracellular concentration of 10 µg/ml can promote production of elastin in the skin fibroblast and is effective for suppression of skin wrinkle. In the above-mentioned U.S. Patent 5,663,160 and WO 99/07338, it is described that when ginsenoside Rb₁ is admixed at the concentrations of 0.001% or more by weight (i.e. concentrations at 10 µg/ml or 10 µg/g or more) in the cosmetics or in the agents for external use on skin and is administered externally to the skin, it is effective for hair growth and hair nourishment, protection of skin, humectant action for skin, regeneration of epidermis and suppression of wrinkle. However, according to the experimental results of the inventor of the present invention (Sakanaka), as described in JP Appln. No. Hei 10-365560, PCT/JP99/02550, high concentrations of ginsenoside Rb₁ do not always provide preferable effects on cells, but rather possible to provide detrimental effects on cells. Especially, as described hereinbefore, admixing ginsenoside Rb₁ at the high concentration and applying externally to the skin for long-term are not preferable due to the possibility of appearing adverse effects.

We (the present inventors) have performed the experiments that the extracellular fluid concentration of ginsenosides, especially ginsenoside Rb₁, in the skin tissue is adjusted at 1 ng/ml or less, preferably 10 pg/ml or less, more preferably 100 fg/ml or less, and among ginsenosides, especially ginsenoside Rb₁ is administered intravenously and continuously or applied externally to the skin at the low dosage levels. As a result, we have found that ginsenoside Rb₁ at the low concentrations or low doses exhibits a superior action for promoting skin tissue regeneration and/or reconstruction or a superior action for promoting wound healing, which could not have been anticipated at all, and thus we completed the present invention. More particularly, we have invented that administration of ginsenoside Rb₁ in low dose or low concentration could regenerate and reconstruct all of skin epithelium (epidermis), corium, dermis, stratum papillare of dermis, subcutaneous tissue, connective tissue, sweat glands, sebaceous glands, hair follicles, hair papilla, blood vessels, peripheral nerves, epidermal cells, epidermal keratinocytes, keratic cells, keratinized cells, cornified cells, hornified cells, Merkel's cell, melanocytes, Langerhans cells, stem cells, mesenchymal cells, fibroblasts, sweat gland cells, hair follicular cells, vascular endothelial cells, vascular smooth muscle cells, etc. and could regenerate and reconstruct extracellular matrices, collagen fibers, elastic fibers, reticular fibers, etc. to nearly healthy conditions. It was found that low dosages and low concentrations of ginsenosides, especially ginsenoside Rb₁, exhibit effectivess and efficacy, through promoting the regeneration and/or reconstruction of skin tissue, for diseases caused by damage, injuries and defect of skin tissue or all diseases causing histopathological changes of skin (wound, scald, burn, radiation injury, frostbite, Pernio, chilblain, ultraviolet injury, electric injury, traumatic injury, skin ulcer, bedsore, decubitus, contact dermatitis, bullous dermatitis, atopic dermatitis, xeroderma, diabetic skin ulcer, autosensitive dermatitis, erythroderma, exfoliative dermatitis, bullous epidermolysis, hypersensitivity to light, photosensitivity, progressive pigmentary disease (Schamberg's disease), strophulus, sting, insect bite, prurigo, erythema multiforme, erythema annulare, erythema nodosum, pemphigus, pemphigoid, dermatitis herpetiformis, palmoplantar pustulosis, psoriasis, lichen planus, ichthyosis, lichen pilaris, xanthomatosis, cutaneous amyloidosis, herpes simplex, viral wart, molluscum contagiosum, pyoderma, skin tuberculosis, atypical mycobacteriosis of the skin, tinea, trichophytide, trichophytosis, cutaneous or oral candidiasis, scabies, pediculosis pubis, phthiriasis, syphilis, keloid, hypertrophic scar, hemangioma, lymphoma, nevus, vitiligo vulgaris, freckle, ephelis (ephelides), chloasma, melanoderma, pompholyx, miliaria, acne vulgaris, rosedrop (rosacea), rosedrop(rosacea)-like dermatitis, oral mucosal injury, stomatitis, perioral dermatitis, senile symptoms of skin, alopecia, periungual disease, unguis incarnatus, etc).

We (the present inventors) have further performed the experiments that the extracellular fluid concentrations of ginsenosides, especially ginsenoside Rb₁, metabolites thereof or salts thereof in lesioned tissue are adjusted and maintained at the low levels as described hereinbefore, and among ginsenosides, especially ginsenoside Rb₁ is applied externally onto the oral mucosa. As a result, we have found that ginsenoside Rb₁ at the low concentrations facilitates regeneration and/or reconstruction of the mouth mucosal tissue, wound healing, ormorsus healing, which have never been anticipated, and thus we completed the present invention. More particularly, we have found that the external or topical administration of low dosages or low levels of ginsenosides, especially ginsenoside Rb₁, to mucosal tissues could cause regeneration or reconstruction of oral mucosal epithelium, lamina propria, salivary glands, mucous glands, mixed glands, connective tissues, muscular tissues, blood vessels, peripheral nerves, epithelial cells, glandular cells, myoepithelial cells, fibroblasts, stem cells, mesenchymal cells, vascular endothelial cells, vascular smooth muscle cells, muscle cells, etc., and further promote regeneration or reconstruction of extracellular matrices, collagen fibers, elastic fibers, reticular fibers, etc. to the conditions close to those of healthy tissues. Namely, it has been found that low doses or low concentrations of ginsenosides, especially ginsenoside Rb₁, exhibit preventive, ameliorating or therapeutic effects and efficacy for diseases caused by injuries to mucosa or defect in mucosa, especially mouth mucosa, or for all diseases causing histopathological changes of mucosa, especially mouth mucosa (caries, pulpitis, periodontal disease, marginal periodontitis, stomatitis, glossitis, recurrent aphthous stomatitis, oral aphtha, halitosis, mouth odor, oral dysesthesia, oral abnormal sensation, odontogenic infection, oral mucosal morsus, tongue morsus, oral mucosal scald, oral mucosal burn, oral mucosal injury, oral mucosal ulcer, etc.) through promoting the regeneration and/or reconstruction of the mucosal tissues.

We (the present inventors) have also found that ginseng, an extract(s) thereof, components thereof or metabolites thereof could promote generation, regeneration, rooting, budding, growth, differentiation or reconstruction of not only the animal tissues but also plant tissues or all plant cells, and completed the present invention. More particularly, we have found that a crude saponin fraction(s) of ginseng or ginsenosides, especially ginsenoside Rb₁, of ginseng are useful as a promoter(s) or a fertilizer additive(s) for rooting, budding, growth, differentiation, regeneration or generation in propagation by cutting or hydroponics of plant tissues such as stems or branches of pothos. Namely, we have found that ginseng, extract(s) thereof, components thereof or metabolites thereof can be used for cultivation, growth or preservation of plants, preservation of fresh flower, hydroponics, cultivation of farm products, growth of farm products, cultivation of vegetables, cultivation and growth of fruits, improvement or amelioration of plants, cultivation and growth of tobacco, or cultivation and growth of tea-leaves.

Further, we (the present inventors) have also found that dihydroginsenoside Rb₁, which is one of novel chemical derivatives of ginsenosides and has been described in PCT/JP00/04102 (Brain cell or nerve cell protecting agents comprising ginseng), exhibits, as like ginsenoside Rb₁, not only a nerve cell-protective action but also superior actions for promoting skin tissue regeneration and reconstruction or wound healing. Namely, it has been found that the above-described effects, efficacy and usages of ginsenoside Rb₁ can be applied to ginsenoside derivatives, especially dihydroginsenoside Rb₁.

### Disclosure of the Invention

An object of the present invention is to provide a drug(s), which can be administered intravenously and continuously or can be applied externally to the local region of skin after degeneration, elimination or morphological changes of the skin tissues or the other organs or tissues due to injuries or defect of skin, i.e. wound, scald, burn, frostbite, pernio, chilblain, radiation injuries, ultraviolet irradiation, electrical injuries, traumatic injuries, ulcer, decubitus, bedsore, or due to diseases of skin or other organs causing histopathological changes, and which can exhibit superior actions for promoting tissue regeneration and/or reconstruction or wound healing. More particularly, the present invention provides a pharmaceutical composition(s) or a veterinary drug composition(s) for prevention, treatment or therapy of organic disease causing histopathological changes of the organic, living, vital or viable tissues, a composition(s) for skin external use or topical application to skin such as cosmetic composition or health-promoting drug composition, a composition(s) for external or topical application to mucosa, or a composition(s) for growth regulation of animals or plants, comprising low dosages; low doses or low concentrations of ginsenosides, metabolites thereof or salts thereof. In the present invention, the health-promoting drugs or health drugs include not only a drug for health in the narrow sense but also health foods, health beverage, health beverage foods, etc.

The present invention also provides a method for exploring or screening effective components or compounds for prevention, treatment or therapy of diseases of skin tissue or mucosal tissues by using ginsenosides or metabolites thereof as a leading compound(s), or use of ginsenosides or metabolites thereof for that purpose. The present invention further provides a method for exploring or screening substances for promoting regeneration, generation, rooting, budding, growth, differentiation or reconstruction of the organic, living, vital or viable tissues comprising assaying protective actions of brain cells or nerve cells by administering substances to be tested into the animal cerebroventricles.

The present invention relates to a pharmaceutical composition(s) or a veterinary drug composition(s) for prevention, treatment or therapy of organic diseases causing histopathological changes of the organic, living, vital or viable tissues comprising ginsenosides such as ginsenoside Rb₁, metabolites thereof or salts thereof. More particularly, the present invention relates to the pharmaceutical composition(s) or the veterinary drug composition(s) hereinabove wherein a content of ginsenosides such as ginsenoside Rb₁, metabolites thereof or salts thereof is less than 0.001% by weight in the whole composition or based on the total weight of the composition. Namely, the present invention relates to the pharmaceutical composition(s) or the veterinary drug composition(s) for prevention, treatment or therapy of organic diseases causing histopathological changes of the organic, living, vital or viable tissues wherein the content of ginsenosides, metabolites thereof or salts thereof is less than 0.001% by weight in the whole composition. More preferably, the present invention relates to the pharmaceutical composition(s) or the veterinary drug composition(s) wherein the extracellular fluid concentrations of ginsenosides, metabolites thereof or salts thereof in lesioned tissues are adjusted to 1 ng/ml or less, or more preferably, the extracellular fluid concentrations in lesioned tissues are 0.01 - 100 fg/ml or 1 - 10000 fg/ml.

The preferable preparations for administration of the pharmaceutical composition(s) or the veterinary drug composition(s) of the present invention are the preparations for intravenous administration such as the preparation(s) for a single intravenous administration or the preparation(s) for continuous intravenous administration, the preparations for external or topical use on mucosa, or the preparations for external or topical use on skin.

The present invention further relates to a composition(s) for external or topical application to skin or a composition(s) for external or topical application to mucosa comprising or consisting essentially of ginsenosides, metabolites thereof or salts thereof at concentrations less than 0.001% by weight in the composition. The composition(s) for external use or topical application onto skin or the composition(s) for external use or topical application onto mucosa of the present invention is applied directly or indirectly to the skin tissue or the mucosal tissues as a cosmetic composition(s), a composition(s) for external use or topical application onto skin for chemical peeling, a health drug composition(s) or a composition(s) for hair growth and hair nourishment. More particularly, the present invention relates to the composition(s) for external use or topical application onto skin or the composition(s) for external use or topical application onto mucosa at the low dosages, doses or concentrations hereinbefore wherein a content of ginsenosides such as ginsenoside Rb₁, metabolites thereof or salts thereof is less than 0.001% by weight, preferably the extracellular fluid concentrations of ginsenosides, metabolites thereof or salts thereof in the skin tissue or the mucosal tissues are 1 ng/ml or less, and more preferably the said concentrations are 0.01 - 100 fg/ml or 1 - 10,000 fg/ml.

The present invention further relates to a composition(s) for growth-regulation for promoting generation, regeneration, growth, reconstruction, differentiation, stock, preservation, nourishment or cultivation of tissues or cells of plants or animals comprising containing ginsenosides, metabolites thereof or salts thereof. The composition(s) for growth-regulation of the present invention is used as a composition for growth promotion or a composition of fertilizer such as rooting or budding promoters for plants, or is used as a growth promoter composition or feed composition such as rooting or growth promoter for animals. More particularly, the present invention relates to the composition(s) for growth-regulation at the low dosage, low doses or low concentration hereinbefore wherein a content of ginsenosides such as ginsenoside Rb₁, metabolites thereof or salts thereof is less than 0.001% by weight.

Further, the present invention relates to a method for exploring or screening active compositions or compounds for prevention, treatment or therapy of diseases of skin tissues or mucosal tissues comprising applying ginsenosides or metabolites thereof as leading compounds, and use of ginsenosides or metabolites thereof as a leading compound(s) for exploring or screening effective components or compounds for prevention, treatment or therapy of diseases of skin or mucosa. The present invention also relates to a pharmaceutical composition(s) or a veterinary drug composition(s) for prevention, treatment or therapy of diseases of skin tissues or mucosal tissues comprising containing substances explored by the methods hereinabove.

Further the present invention relates to a method for exploring or screening substances for promoting regeneration, generation, rooting, budding, growth, differentiation or reconstruction of the organic, living, vital or viable tissues comprising assaying protective actions of intracerebroventricularly administered substances to be tested on brain cells or nerve cells. The present invention also relates to a pharmaceutical composition(s) or a veterinary drug composition(s) for promoting regeneration, generation, growth, differentiation or reconstruction of the organic, living, vital or viable tissues comprising containing a compound(s) or salt thereof which exhibited protective action on brain cells or nerve cells by intracerebroventricular administration performed by the said method.

The present invention relates to a method for mass production of ginsenosides or metabolites thereof comprising using cultured cells or plant strains which can produce ginsenosides.

Examples of the organic, living, vital or viable tissues of the present invention are the tissues of organisms such as human, animals, plants, microorganisms, etc., for example the external tissues of organisms such as skin tissue or mucosal tissues, the visceral tissues of peritoneal and thoracic regions such as liver, kidneys, spleen, pancreas, lungs, digestive organs such as intestine and stomach, urinary organs such as urinary bladder and genital organs such as uterus and testis, and tissues of head and neck, tissues of bone, joint, ligament, muscle, blood vessel, nerve, etc. These organic, living, vital or viable tissues are preferably in the form of in vivo state but can be in the form of ex vivo state such as for organ transplantation.

The histopathological changes of the organic, living, vital or viable tissues of the present invention are the conditions, in which the above-described organic, living, vital or viable normal tissues are pathologically and histologically changed, for example injury, wound, traumatic injury, trauma, wound or defect. Origins or sources causing these histopathological changes are not specifically limited, and can be any causes including physical forces from outside, excision, transection or suture in surgical treatments and operations, pathological changes such as peptic ulcerative lesion.

The pharmaceutical composition(s) or the veterinary drug composition(s) of the present invention is used for prevention, treatment or therapy of organic diseases causing histopathological changes of the vital, living or viable tissues, and characterized by promoting regeneration and/or reconstruction of cells or tissues of the organic, living, vital or viable tissues causing histopathological changes. Consequently, the specific feature of the pharmaceutical composition(s) or the veterinary drug composition(s) of the present invention is to promote cure of organic diseases through regeneration and/or reconstruction of the organic, living, vital or viable tissues or cells thereof with histopathological changes.

The pharmaceutical composition(s) or the veterinary drug composition(s) of the present invention has the specific feature of preferably low dosage, low dose or low concentration wherein a content of ginsenosides, metabolites thereof or salts thereof is less than 0.001% by weight, more precisely 0.0001% by weight or less, 0.00001% by weight or less, 0.000001% by weight or less, 0.0000001% by weight or less, or 0.00000001% by weight or less in the whole composition or based on the total weight of the composition. In use of such low dosages, low doses or low concentrations, the extracellular fluid concentrations of effective components of ginsenosides, metabolites thereof or salts thereof are maintained at 1 ng/ml or less, preferably 0.01 - 100 fg/ml or 1 - 10000 fg/ml. The present invention has the specific feature of finding out a new action of the effective components at such the low concentrations.

"Ginsenosides" of the present invention can be a compound(s) so called ginsenoside such as ginsenoside Rb₁, a component of ginseng, natural product(s) such as ginseng or extract(s) thereof containing the same, extract(s), fractional component(s) or purified component(s), further it can be a compound(s), derived from naturally occurring ginsenoside compounds such as ginsenoside Rb₁ through chemical modification by the use of chemical means.

"Ginsenosides" or naturally occurring ginsenoside compounds are exemplified as follows.

Ginsenoside Ro (chikusetsusaponin V; saponin A), ginsenoside Ra₁, ginsenoside Ra₂, ginsenoside Rb₁; saponin D, ginsenoside Rb₂, ginsenoside Rb₃, ginsenoside Rc, ginsenoside Rd, ginsenoside Re; ginsenoside Ra_{3;} notoginsenoside R_{4;} kinkenoside R_{1;} ginsenoside Rs_{1;} ginsenoside Rs_{2;} 20s-ginsenoside Rg_{3;} 20-glucoginsenoside Rf; notoginsenoside R_{1;} ginsenoside Rf; 20R- ginsenoside Rg_{2;} 20R-ginsenoside Rh_{1;} ginsenoside Rf, ginsenoside Rg_{1;} ginsenoside Rg_{2;} chikusetsusaponin I; ginsenoside Rg_{3;} ginsenoside Rh_{1;} ginsenoside Rh_{2;} maronylginsenoside Rb_{1;} maronylginsenoside Rb_{2;} maronylginsenoside Rc; maronylginsenoside Rd; chikusetsusaponin Ia; chikusetsusaponin Ib; chikusetsusaponin III; chikusetsusaponin IV; saponin B; chikusetsusaponin IVa; saponin C; protopanaxadiol, protopanaxatriol, oleanolic acid, etc. or stereoisomers thereof. In the present invention, since these ginsenosides have similar chemical structure in each other and thus are thought to have common effects, efficacy and usages, each of them can be used in a single form, or can be used simultaneously by combining with different plural numbers of ginsenosides.

Examples of natural product such as ginseng or extract(s) thereof, extract, fraction components, or purified components containing ginsenosides can be the natural product(s) which contain relatively a large amount(s) of the ginsenoside compound(s) hereinbefore. These can be the natural product(s) itself, the extract(s) which is prepared by extraction and concentration of components containing the ginsenoside compound(s), the extract(s) or the tablet(s) which is prepared in the form of liquid or solid state from the extract, further the fraction(s) containing the ginsenoside compound(s) which is purified and separated from the extract(s) such as saponin fraction, or the purified emulsion of the ginsenoside compound(s) which is prepared by purifying ginsenoside compound-containing fractions.

Examples of natural product such as ginseng or extract(s) thereof, extract, fraction component or purified fraction containing the ginsenoside compound(s) are medicinal ginseng, ginseng extract or crude saponin fraction of ginseng.

The compounds derived from naturally occurring ginsenoside compounds such as ginsenoside Rb₁ by means of chemical modification are prepared from chemical structures of the above-mentioned natural ginsenosides by the following means of modification. (Hereinafter, in the present specification, these compounds are referred to as "ginsenosides derivatives", "ginsenoside derivatives" or "ginsenosides' derivatives".)

Namely, (1) a double bond in the side carbon chain bound to steroid-like skeleton or structure (dammarane skeleton or structure) is reduced (so called dihydroginsenosides); (2) hydroxyl group of ginsenosides is acetylated; (3) in addition to acetylation, a double bond in the side carbon chain is converted to single bond and optional functional group such as a hydroxyl(s) and a hydroxyl group(s) is bonded; (4) in addition to acetylation, a double bond in the side chain is cleaved and terminal thereof is converted to aldehyde; (5) in addition to acetylation, optional functional group such as alkyl or aryl is bonded to the side chain terminal; (6) in addition to acetylation, a double bond in the side chain is cleaved and bonded with carboxyl; (7) a double bond in the side chain is cleaved and bonded with carboxyl; (8) methyl on one side in the side chain terminal is substituted to hydrogen atom and methyl on the other side is substituted by optional functional group such as alkyl or aryl; (9) a double bond in the side chain is converted to a single bond and optional functional group such as a hydroxyl(s) and a hydroxyl group(s) is bonded; and (10) any compound having fundamental skeletal structure of protopanaxadiol, protopanaxatriol, damaran, dammarane, oleanolic acid or reduced compound thereof. Derivatives of the above-described ginsenosides (especially, protopanaxadiol-based saponins and protopanaxatriol-based saponins) are described in PCT/JP00/04102 (Brain cell or nerve cell-protecting agents comprising ginseng). Further, in PCT/JP00/04102, neuroprotective action, a method of preparation and NMR chart of one of the above-mentioned ginsenosides derivatives, dihydroginsenoside Rb₁ are described.

With regard to oleanolic acid such as ginsenoside Ro (chikusetsusaponin V) which has a slightly different chemical structure among ginsenosides, a compound(s) prepared by chemical modification of the following means can be mentioned: (1) reduction of a double bond in the chemical structure of steroid-like skeleton or aglycone of ginsenosides (oleanolic acid) (so called dihydroginsenosides); (2) substitution of hydrogen atom in the reduced position of (1) to any of functional group (for example, hydroxyl, alkyl, aryl, etc.); (3) esterification of carboxyl; (4) acetylation of hydroxyl; and (5) combination of any two or more methods for modification (1) - (4). Since the above-described ginsenosides derivatives or stereoisomers thereof have similar chemical structures and are thought to have common effects, efficacy and usages, they can be used in the single form or in combination with different plural ginsenoside derivatives or ginsenosides simultaneously.

The metabolic products of ginsenosides of the present invention are compounds produced as a result of metabolism of ginsenosides of the present invention in vivo, and the effective component(s) of the present invention is not limited in the above-mentioned ginsenosides, but is the metabolic product(s) in vivo as well as the compound(s) which can achieve an object(s) of the present invention.

### Brief Description of Drawings

Fig. 1 is a bright-field photomicrograph in place of drawing showing the effect of intravenously administered ginsenoside Rb₁ on incised wound. A: a case of administering ginsenoside Rb₁; B: a case of administering physiological saline.
Fig. 2 is a bright-field photomicrograph in place of drawing showing the therapeutic effect of intravenously administered ginsenoside Rb₁ on open wound. A: a case of administering ginsenoside Rb₁; B: a case of administering physiological saline.
Fig. 3 is a bright-field photomicrograph in place of drawing showing the effect of intravenously pre-administered ginsenoside Rb₁ on open wound. A: a case of administering ginsenoside Rb₁; B: a case of administering physiological saline.
Fig. 4 is a drawing showing a part of chemical derivatives prepared by utilizing ginsenoside Rb₁ as a leading compound.
Fig. 5 is a photograph in place of drawing showing a trivial therapeutic effect of ginsenoside Rb₁ at a low concentration (0.001% by weight) administered extracutaneously, topically or locally to open wound.
Fig. 6 is a photograph in place of drawing showing the effects of ginsenoside Rb₁ at low concentrations (0.0001% by weight, 0.00001% by weight and 0.000001% by weight,) administered extracutaneously, topically or locally to open wound.
Fig. 7 is a photograph in place of drawing showing the effect of ginsenoside Rb₁ at a concentration of 10⁻⁵% by weight administered extramucosally, topically or locally to morsus of human oral mucosa.
Fig. 8 is a photograph in place of drawing showing the effect of ginsenoside Rb₁ at a concentration of 10⁻⁵% by weight administered extramucosally, topically or locally to morsus of human oral mucosa.
Fig. 9 is a photograph in place of drawing showing the effect(s) of ginsenoside Rb₁ (100 fg/ml) on generation, regeneration and/or rooting of root of pothos on day 13 after treatment with ginsenoside Rb₁.
Fig. 10 is a photograph in place of drawing showing the effect(s) of ginsenoside Rb₁ (100 fg/ml) on generation and/or regeneration of root of pothos on day 22 after treatment with ginsenoside Rb₁.
Fig. 11 is a photograph in place of drawing showing the effect(s) of a crude saponin fraction of ginseng (1450 fg/ml) on generation and/or regeneration of root of pothos on day 14 after treatment with the crude saponin fraction.
Fig. 12 is a photograph in place of drawing showing the effects of ginsenoside Rb₁ at concentrations of 10⁻⁴ - 10⁻⁸% by weight administered externally, topically or locally to rat open wound.
Fig. 13 is a graph showing the effects of externally or topically administered ginsenoside Rb₁ at concentrations of 10⁻⁴ - 10⁻⁸% by weight on rat open wound.
Fig. 14 is a photograph in place of drawing showing the effects of dihydroginsenoside Rb₁ at 10⁻⁴ - 10⁻⁷% by weight administered externally, topically or locally to rat open wound.
Fig. 15 is a graph showing the effects of externally or topically administered dihydroginsenoside Rb₁ at concentrations of 10⁻⁴ - 10⁻⁷% by weight on rat open wound.
Fig. 16 is a photograph of MAP2 immunoblotting in place of drawing showing a protective effect(s) of dihydroginsenoside Rb₁ on apoptosis or apoptosis-like cell dihydroginsenoside Rb₁ on apoptosis or apoptosis-like cell death of cultured nerve cells (neurons) as induced by SNP.
Fig. 17 is a graph showing the protective effect(s) of dihydroginsenoside Rb₁ on apoptosis or apoptosis-like cell death of cultured nerve cells(neurons) as induced by SNP.
Fig. 18 shows NMR chart of dihydroginsenoside Rb₁.

### Best Mode for Carrying Out the Invention

The wound healing-promoting action or the tissue regeneration and reconstruction-promoting actions of low dosages, low doses or low concentrations of ginsenosides of the present invention will be explained in detail based on concrete examples hereinbelow. For that purpose, experimental results will be explained by using ginsenoside Rb₁, one of representative ginsenosides and dihydroginsenoside Rb₁, a ginsenoside Rb₁ derivative which is one of chemically modified derivatives of natural ginsenosides, as ginsenosides of the present invention.

In order to investigate the effects of low dosages, low doses or low concentrations of ginsenoside Rb₁ on regeneration and/or reconstruction of skin tissue, the effect(s) of continuous intravenous infusion of ginsenoside Rb₁ on incised wound of skin which enabled us to observe easily regeneration phenomena of tissues or cells was examined. Male, Wistar rats (body weight about 300 g) were used. The animals were bred in a room with a 12 : 12 hour light-dark cycle, and water and feed were supplied ad libitum. Incised wound with length about 3 cm was made in the dorsal region of animals under inhalation anesthesia, and sutured with nylon thread. One hour later, ginsenoside Rb₁ (60 µg) dissolved in physiological saline was once intravenously injected. Thereafter continuous intravenous infusion of ginsenoside Rb₁ (60 µg/day) was performed for 7 days by using an Alza osmotic minipump.

An equal amount of physiological saline was administered intravenously in the control animals, for which the same open wound was made and sutured with nylon thread.

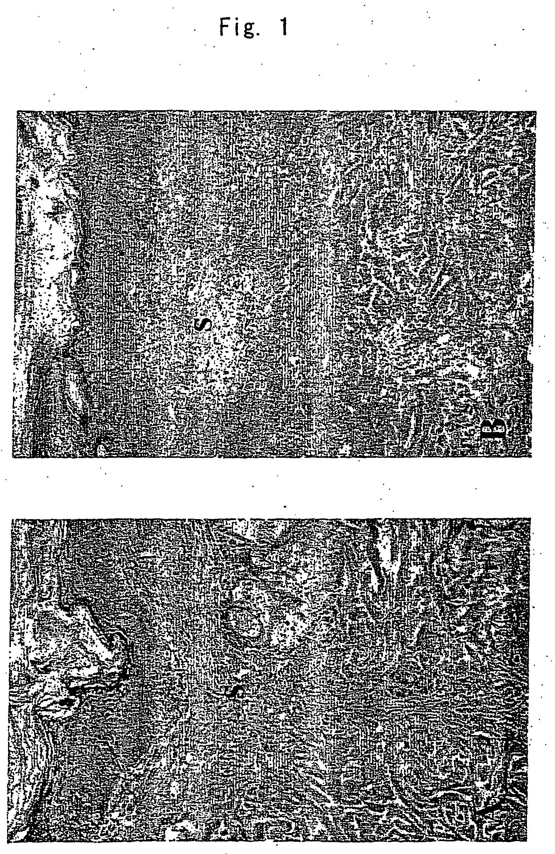

On day 2 after finishing continuous intravenous administration of ginsenoside Rb₁ or physiological saline, the animals were anesthetized with pentobarbital and perfused transcardially with 0.1 M phosphate buffer containing 4% paraformaldehyde. Thereafter, the skin tissue including the sutured region of incised wound was collected, post-fixed and embedded in paraffin. Paraffin sections with 5 µm thickness were prepared to supply for hematoxylin-eosin staining (HE). Result is shown in Fig. 1. Fig. 1 is a photograph in place of drawing. Fig. 1A shows a case of ginsenoside Rb₁ administration and Fig. 1B shows a case of physiological saline administration. In the figure, "s" indicates scar or granulation.

As shown in Fig. 1A, in the case of ginsenoside Rb₁ administration, as compared with the case of physiological saline administration in Fig. 1B, many skin appendages such as sweat glands, sebaceous glands and hair follicles were observed in close proximity to the local lesion of incised wound. This indicates that as a result of intravenous administration of ginsenoside Rb₁ in low dosage or low dose, sweat glands, sebaceous glands, hair follicles and cells thereof are rapidly regenerated and reconstructed. Further, in case of low dosages or low doses of ginsenoside Rb₁ administration, which differs from the case of physiological saline administration, epidermis, dermis and subcutaneous tissue except for the local lesion of wound, were regenerated, reconstructed or recovered to the condition close to normal. Namely, it can be said that intravenous administration of ginsenoside Rb₁ regenerates and reconstructs rapidly the skin tissue with wound, as a result, wound healing is obviously promoted.

In the case of physiological saline-administered group in Fig. 1B, scar or granulation, so called large growth of scar or granulation is observed, but regeneration of injured tissues is hardly observed. Namely, in the conventional wound healing, only scar or granulation is growing, and neither systematic regeneration nor reconstruction of injured tissue is observed. However, as observed in Fig. 1A in the present invention, it is the specific feature that administration of ginsenoside Rb₁ not only reduces scar or granulation region but also regenerate and reconstruct each injured tissue.

Consequently, judging from the intravenous administration of ginsenoside Rb₁ to cause progress of regeneration and/or reconstruction of tissue even in the deep region of skin tissue and to facilitate wound healing with good order, if ginsenosides, especially ginsenoside Rb₁, are administered intravenously before and/or after operation in aged people, patients with low nutrition, patients with diabetes, patients with immunodeficient diseases, patients with AIDS or patients with cancer, who easily suffer from insufficient suture, it is expected to exhibit superior effects and efficacy. Further, intravenous administration of ginsenosides, especially ginsenoside Rb₁, before and/or after plastic surgical operation (including so called vanity surgery) or after onset of diseases caused by skin injury, wound, traumatic injury or defect, will "cure injury rapidly and surely" through superior wound healing-promoting effect and tissue regeneration and reconstruction-promoting action. As shown in Fig. 1A, in case of ginsenoside Rb₁ administration, since the tissue regeneration and reconstruction progress in a satisfactory manner, and collagen fibers, elastic fibers, reticular fibers and extracellular matrices are sufficiently produced and excreted to the levels of nearly normal condition in the dermis and subcutaneous tissue, as a result, scar or granulation becomes smaller than the case of physiological saline administration in Fig. 1B.

We have examined whether intravenous administration of ginsenoside Rb₁ at low dosages or low doses can promote regeneration and/or reconstruction of skin tissue in diseases with defect of skin tissue (bedsore, skin ulcer, burn, frostbite, radiation injury, open wound, ultraviolet injury, electric injury, etc.). For that purpose, for example, by using male Wistar rats (body weight about 300 g), punch biopsy with diameter 6 mm was performed in the dorsal skin region of animals under inhalation anesthesia to prepare open wound, and the animals were allowed to leave as they were. About 1 hour later, ginsenoside Rb₁ (12 µg) dissolved in physiological saline was once administered intravenously, then ginsenoside Rb₁ was continuously infused intravenously for 7 days by using an Alza osmotic minipump (12 µg/day).

An equal amount of physiological saline was administered intravenously in the control animals, for which the same open wound was made to leave the animals as they were.

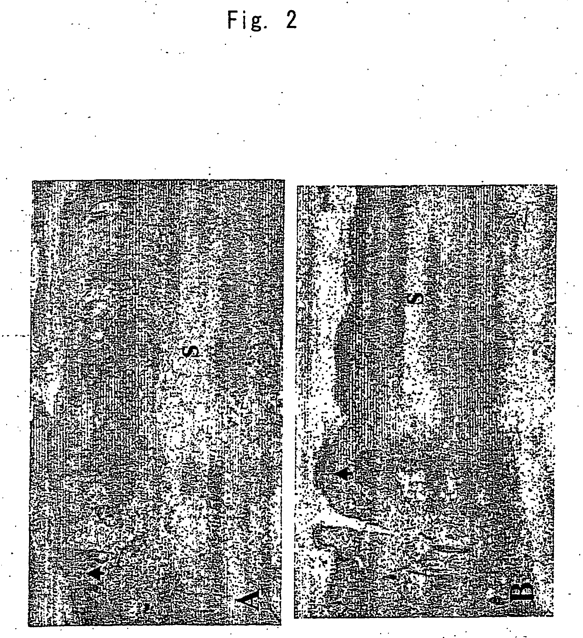

On day 2 after finishing continuous intravenous administration of ginsenoside Rb₁ or physiological saline, the animals were anesthetized with pentobarbital and fixed transcardialy with perfusion of 0.1 M phosphate buffer containing 4% paraformaldehyde. Thereafter, the skin tissue including the open wound was dissected out, post-fixed and embedded in paraffin. Paraffin sections with 5 µm thickness were cut and stained with hematoxylin-eosin (HE). Result is shown in Fig. 2. Fig. 2 is a photograph in place of drawing. Fig. 2A shows a case of ginsenoside Rb₁ administration and Fig. 2B shows a case of physiological saline administration. Left side from the arrow in Fig. 2A and B, indicates healthy region; right side from the arrow in Fig. 2A, indicates regenerated skin tissues; and right side from the arrow in Fig. 2B, indicates mainly scar or granulation. In the regenerated skin tissues in Fig. 2A, many hair follicles, hair papillae, sebaceous glands and pilomotor muscles are observed in the subepidermal connective tissues (dermis or subcutaneous tissue), and a small amount of scar or granulation was observed beneath the regenerated and reconstructed skin tissues.

As shown in Fig. 2A, in the ginsenoside Rb₁-administered case, as compared with the physiological saline-administered case in Fig. 2B, sufficient epithelization or epidermization occurred, and regeneration and reconstruction of the connective tissue of dermis with papillae and of the subcutaneous tissue progressed to the condition close to normal tissue. Further, in the ginsenoside Rb₁-administered case, which is different from the physiological saline-administered case, the skin appendages such as hair follicles, hair papillae, sebaceous glands, pilomotor muscles, sweat glands, etc. were observed abundantly in the skin tissues regenerated after open wound, and the blood vessel networks were also regenerated, reconstructed and/or recovered to a condition close to that of the normal tissues. Perhaps, as the results of regeneration and/or reconstruction of the epidermis, the connective tissue of the dermis, the dermal papillae, the subcutaneous tissues, the skin appendages and the blood vessels, the peripheral nerves, which were excised at the occasion of open wound preparation, appear to be regenerated by means of intravenous administration of ginsenoside Rb₁. As shown in Fig. 2A, in the ginsenoside Rb₁-administered case, since the tissue regeneration and reconstruction progressed in a satisfactory manner and collagen fibers, elastic fibers, reticular fibers and extracellular matrices (matrix) are sufficiently produced and excreted to the levels of nearly normal condition in the dermis and subcutaneous tissue, as a result, scar or granulation becomes smaller than the case of physiological saline administration in Fig. 2B.

Then, we (the present inventors) have examined whether intravenous administration of ginsenoside Rb₁ before preparation of open wound of skin can promote regeneration and/or reconstruction of the skin tissue. For that purpose, ginsenoside Rb₁ (12 µg) dissolved in physiological saline was intravenously administered once into male Wistar rats (body weight about 300 g), under inhalation anesthesia, subsequently, continuous intravenous infusion of ginsenoside Rb₁ (12 µg/day) was conducted for 4 days by using an Alza osmotic minipump. Thereafter, the punch biopsy with diameter 6 mm was performed in the dorsal skin region of animals under inhalation anesthesia to make open wound, and simultaneously, ginsenoside Rb₁ was continuously infused intravenously for 3 days.

An equal amount of physiological saline was administered intravenously in the control animals, for which the same open wound was prepared to leave the animals as they were.

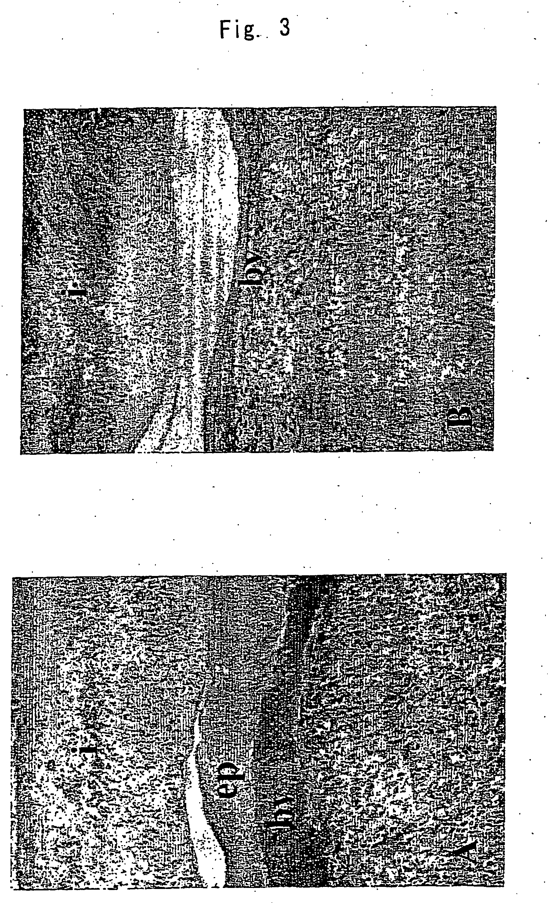

On day 2 after finishing continuous intravenous administration of ginsenoside Rb₁ or physiological saline (i.e. on day 5 after preparation of the open wound), the animals were anesthetized with pentobarbital and fixed transcardially with perfusion of 0.1 M phosphate buffer containing 4% paraformaldehyde. Thereafter, the skin tissue including the open wound was dissected out, post-fixed and embedded in paraffin according to the conventional method. Paraffin sections 5 µm thick were cut and stained with hematoxylin-eosin (HE). Result is shown in Fig. 3. Fig. 3 is a photograph in place of drawing. Fig. 3A shows a case of ginsenoside Rb₁ administration and Fig. 3B shows a case of physiological saline administration. "i" indicates incrustation or eschar, "ep" indicates stratified squamous epithelium of epidermis, and "bv" indicates blood vessel.

As shown in Fig. 3A, in the ginsenoside Rb₁-administered case, on day 5 after preparation of the open wound, the epidermis (stratified squamous epithelial tissue) was obviously regenerated and reconstructed under the eschar, and thick regenerated blood vessels or generated blood vessels filled with erythrocytes were distributed just beneath the epidermis (stratified squamous epithelium) and relatively thin blood vessels, which were dissociated from said blood vessel, were observed densely in the connective tissues of the dermis or the subcutaneous tissues. On the other hand, as shown in Fig. 3B, in the physiological saline-administered case, even on day 5 after preparation of the open wound, regeneration of the epidermis under eschar was extremely incomplete, and the regenerated blood vessel just beneath the very thin epidermis was obviously thin as compared with the case of ginsenoside Rb₁-administration. For that reason, a small number of extremely thin blood vessels was observed in the subepidermal connective tissues, which was considered to be scar or granulation in future. Consequently, it was elucidated that as a result of intravenous administration of ginsenoside Rb₁, regeneration and reconstruction of skin tissue were obviously promoted, and that regeneration, generation and reconstruction of once ruptured and excised blood vessels were promoted by intravenous administration of ginsenoside Rb₁. Further, it should not be forgotten that as shown in Fig. 3A, the thick blood vessel observed immediately beneath the epidermis (stratified squamous epithelium) on day 5 after the preparation of open wound in the case of ginsenoside Rb₁ administration almost degenerated or disappeared on day 9 after the preparation of open wound as shown in Fig. 2A, and the connective tissue of the dermis with papillae was observed in that region. Namely, in the case of ginsenoside Rb₁ administration, the regeneration or the generation of blood vessels occurs at the early stage after preparation of the open wound, and the reconstruction of blood vessels takes place in harmony with the completion of regeneration and/or reconstruction of the skin tissue. It should be said that the present invention, which proved that the one compound could achieve the complex vital phenomenon of tissue regeneration and reconstruction so clearly, is the first thing in the human history. In the healthy tissues, no clear difference was observed between the ginsenoside Rb₁-administered case and the physiological saline-administered case. This supports that continuous intravenous administration of low dosages or low doses of ginsenoside Rb₁ does not affect the healthy tissues but gives favorable effects on only lesioned tissues or damaged (injured) tissues. Namely, it can be said that ginsenosides, especially ginsenoside Rb₁, is a pharmaceutical composition(s) with less side effect.

A result of the present experiment wherein once defected skin tissues caused by open wound are regenerated and reconstructed rapidly and nearly to normal condition by the intravenous administration of ginsenoside Rb₁, demonstrates that ginsenosides, especially ginsenoside Rb₁, promote division, growth, migration and differentiation of epidermal cells, epidermal keratinocytes, cornified cells, corneocytes, keratinized cells, hornified cells, Merkel cells, Langerhans cells, stem cells, fibroblasts, mesenchymal cells, vascular endothelial cells, pilomotor muscular cells and vascular smooth muscle cells, and facilitate differentiation of epidermal cells to hair follicles, sweat gland and sebaceous gland cells. Furthermore, it has been invented that as a result of the well-organized and systematic regeneration and/or reconstruction of the above-mentioned various cells, peripheral nerves and blood vessels by the administration of ginsenoside Rb₁, open wound of the skin was rapidly recovered to the condition of nearly normal skin tissues. Namely, as a result of continuous intravenous administration of ginsenosides, especially ginsenoside Rb₁, at low doses, newly regenerated epidermal cells and fibroblasts in the defected region of skin are arranged in the mode similar to that of the normal skin tissue and the extracellular matrices (matrix), collagen fibers, elastic fibers and reticular fibers are regenerated and reconstructed nearly to the condition of normal skin tissue. As shown in the results of the present experiments (Fig. 2A and Fig. 3A), since not only the epidermal tissue but also the dermis and subcutaneous tissues are regenerated and reconstructed in the open wound (skin defected region) by intravenous administration of ginsenoside Rb₁, even if the same region is loaded again with traumatic injury after epithelization (epidermization) of the open wound, it is expected that exfoliation of the epidermal tissue is difficult to occur in the open wound region once epithelized by administration of ginsenoside Rb₁.

On the other hand, as shown in Fig. 2B and Fig. 3B, in case of physiological saline administration, even if epithelization (epidermization) occurs in appearance, since the dermis and the subcutaneous tissue are accompanied by neither regeneration nor reconstruction and scar or granulation is formed, the high possibility is left open that the epidermal tissue is easily exfoliated only by addition of mild external force. According to the inventors' experiences, even epidermal growth factor (EGF), platelet-derived growth factor (PDGF) or basic fibroblast growth factor (bFGF) was sprayed or spread on the local open wound of rats' skin, their actions to promote wound healing were inferior to the effects of continuous intravenous administration of ginsenoside Rb₁ at low dosages or low doses. The fact that such the low dosage or low dose of ginsenoside Rb₁ achieves so clearly the regeneration and/or reconstruction of skin tissue or wound healing by itself, indicates that the production of cytokines (EGF, TGF-β₁, TGF-α, FGF, VEGF, PDGF-BB, TGF-β₁, PDGF-AB, IGF, KGF, PDGF, TGF-β₂, TGF-β₃, FGF-2, U-PA, t-PA, etc.) involved in wound healing or regeneration and reconstruction of skin tissue and the functions of blood cell components or plasma components are regulated by the low dosages or low doses of ginsenosides, especially ginsenoside Rb₁. As described in the review, Singer, A. J. and Clark, R. A. F., New Engl. J. Med., 341, 738-746, 1999, low dosages, low doses or low concentrations of ginsenosides, especially ginsenoside Rb₁, achieve independently the complex vital phenomena involved in wound healing, or tissue regeneration or reconstruction.

According to the results of the present experiments using open wound of rats' skin, the wound healing-promoting effect and the skin tissue regeneration or reconstruction-promoting action of the preparations for intravenous administration comprising ginsenosides, especially ginsenoside Rb₁ are thought to be the most potent in history. Ginsenosides, especially ginsenoside Rb₁, metabolites thereof or salts thereof appear to exhibit the extremely potent wound healing-promoting action and skin tissue regeneration or reconstruction-promoting action, and this fact supports that ginsenosides, especially ginsenoside Rb₁, metabolites thereof or salts thereof can be a leading compound(s) for prevention, treatment or therapy of diseases caused by damage, injury, traumatic injury or defect of skin, or diseases causing histolopathogical changes of skin.

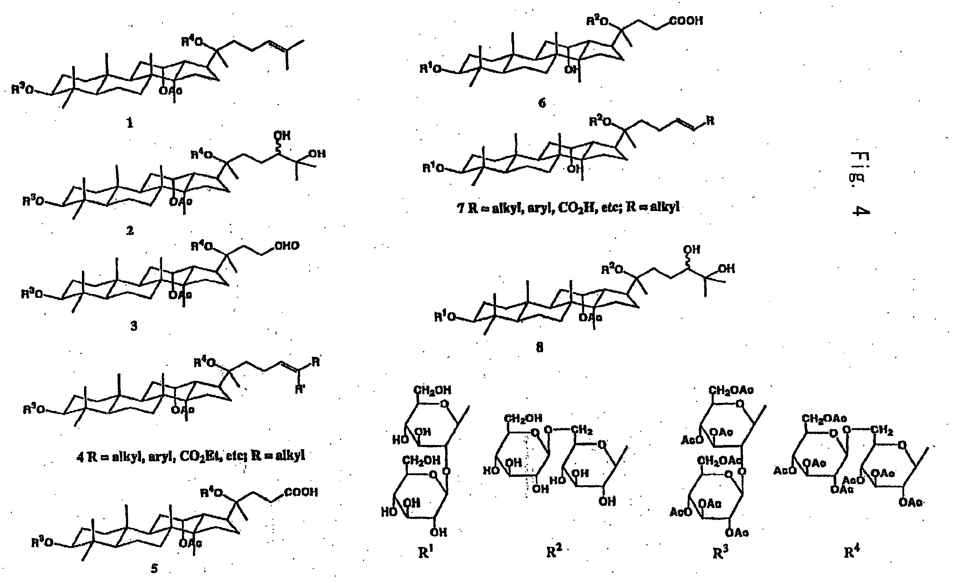

As for the therapeutic drug candidates for diseases of the skin prepared by applying ginsenoside Rb₁ as a leading compound, the most highly probable compound is dihydroginsenoside Rb₁. Said compound can be prepared, as described hereinbefore, by reducing a double bond in the side carbon chain binding to a steroid-like skeleton or structure (dammarane skeleton or structure) of ginsenoside Rb₁. Other chemical derivatives of ginsenoside Rb₁ shown in Fig. 4 can also be, as like ginsenoside Rb₁, agents for prevention, treatment or therapy of diseases caused by damage, injury, traumatic injury or defect of skin or mucosa, or diseases causing histopathological changes of skin or mucosa. Fig. 4, upper left, (1) is an example of derivatives acylating hydroxyl group(s); (2) is an example of, in addition to acetylation, converting a double bond in the side carbon chain to single bond and bonding optional functional group such as a hydroxyl(s) and a hydroxyl group(s); (3) is an example of, in addition to acetylation, cleaving a double bond in the side chain and converting the terminal thereof to aldehyde; (4) is an example of, in addition to acetylation, bonding optional functional group such as alkyl or aryl to the side chain terminal; (5) is an example of, in addition to acetylation, cleaving a double bond in the side chain and bonding with carboxyl; (6) is an example of cleaving a double bond in the side chain and bonding with carboxyl; (7) is an example substituting methyl on one side in the side chain terminal to hydrogen atom and substituting methyl in the other side by optional functional group such as alkyl or aryl; and (8) is an example converting a double bond in the side chain to a single bond and bonding optional functional group such as a hydroxyl(s) and a hydroxyl group(s). with regard to the above-described derivatives, they are described in "Disclosure of the Invention" as well. In ginseng, besides ginsenoside Rb₁, about 30 kinds of known purified saponins, i.e. ginsenosides, are included (Shoji, Junzo, "Ginseng '95, pp251-261, Kumagai, Akira, Ed. Kyoritsu Publishing Co., Ltd.), and since chemical structures of these purified saponins, i.e. ginsenosides, are similar to ginsenosides Rb₁, these can be agents for treatment, prevention or therapy of the above-described skin diseases or mucosal diseases. Of course, novel chemical derivatives prepared by applying ginsenoside Rb₁ as a leading compound are not limited within the above-described compounds. Chemical derivatives of purified saponins, i.e. ginsenosides (especially, protopanaxadiol and protopanaxatriol saponins), except for ginsenoside Rb₁ can be prepared by reducing the side chain of the dammarane skeleton or structure (steroid-like skeleton or structure) or by modifying the purified saponins in similar ways to Fig. 4. With regard to chemical derivatives of oleanolic acid such as ginsenoside Ro are described in "Disclosure of the invention". The above-described chemical derivatives of ginsenosides, especially ginsenoside Rb₁, i.e. ginsenosides derivatives and known analogues, namely compounds having chemical structures of three types of aglycone described in the review by Shoji are expected to exhibit effects and efficacy similar to those of ginsenoside Rb₁ against brain and nervous diseases and diseases accompanied with cell death such as cerebral apoplexy, neurodegenerative diseases, spinal cord injury, head injury, neurotrauma, nerve injury, etc. (Japanese Patent Appln. No. Hei 10-365560, PCT/JP99/02550, Brain cell or nerve cell-protective agents comprising ginsenoside Rb₁; Japanese Patent Appln. No. Hei 11-340850, PCT/JP99/06804, Cerebrovascular regeneration and reconstruction promoter and nerve tissue secondary degeneration inhibitor comprising ginsenoside Rb₁; and PCT/JP00/04102, Brain cell or nerve cell protecting agents comprising ginseng). Among various diseases and disease states (pathologic conditions) accompanied with cell death, for which application of the above-described ginsenosides or ginsenosides derivatives is expected, all diseases and disease states (pathologic conditions) described in the book (Today's therapy; Ed. Hinohara, Shigeaki and Abe, Masakazu, Igaku Shoin, 1995) are included. Of course, 45 specific diseases indicated by the Japanese Ministry of Health and Welfare (MHW) can be prevented, treated or cured by ginsenosides, especially ginsenoside Rb₁, or ginsenosides derivatives, especially dihydroginsenoside Rb₁.

Since the skin tissue regeneration and reconstruction-promoting effect of ginsenosides, especially ginsenoside Rb₁, is epoch-making, not only the novel agents for treatment or therapy of skin diseases or tissue regeneration promoters can be prepared by using ginsenosides, especially ginsenoside Rb₁, or metabolites thereof as a leading compound(s) but also the development of agents for treatment or therapy of skin diseases can be directed by synthesizing a compound(s), which modifies function of a target molecule, after identifying the target molecule of ginsenosides, especially ginsenoside Rb₁, or metabolites thereof.

In the present experiments, ginsenoside Rb₁ at low doses or low dosages was continuously administered intravenously after preparation of open wound, which generates skin defect; as a result, regeneration and reconstruction of the skin tissue were promoted and wound healing was markedly facilitated. This fact indicates that the continuous intravenous administration of ginsenosides, especially ginsenoside Rb₁, at low doses or low dosages also exhibits favorable effects and efficacy on the other diseases causing skin defect (e.g. bedsore, skin ulcer, burn, frostbite, radiation injury, bullous skin diseases, ultraviolet injury, electric injury, sunstroke, skin trauma, etc.). Further, intravenous or local administration of ginsenosides, especially ginsenoside Rb₁ is said to be effective for not only skin diseases but also other diseases, in which a part(s) of organs or tissues is defected, (e.g. peptic ulcer, ulcerative colitis, mucosal erosion, mucosal ulcer, mucosal erosion of the digestive tract or tube, chronic gastroenteritis, acute gastroenteritis, Crohn's disease, Behcet's disease, tympanic injury, corneal injury, corneal erosion, corneal ulcer, bone defect, injuries to the urinary bladder, urethra and penis, etc..). In the above-mentioned diseases, ginsenosides, especially ginsenoside Rb₁ can be applied as nasal drop administration, rectal administration, ear drop administration, eye drop administration or sublingual administration. Of course, low doses of ginsenosides, especially ginsenoside Rb₁, exhibit effectiveness and efficacy by promoting regeneration or reconstruction of organs or tissues causing histopathological changes. Examples of the diseases and pathological conditions causing such the histopathological changes are wound, burn, traumatic injury, trauma, morsus, skin ulcer, bedsore, decubitus, and all diseases and pathological conditions described in the book (Today's therapy; Ed. Hinohara, Shigeaki and Abe, Masakazu, Igaku Shoin, 1995).

In the present experiments using open wound of rats' skin, intravenous administration of the low dosages or low doses of ginsenoside Rb₁ promoted markedly regeneration and/or reconstruction of skin tissue. Although it needs no repetition here, the skin has constituting elements in common with other organs and tissues, such as epidermis (stratified squamous epithelium), connective tissues, bloodvessels, nerves, secretory glands, etc. Consequently, the present experimental fact that the continuous intravenous administration of low dosages or low doses of ginsenosides, especially ginsenoside Rb₁, significantly promotes regeneration and/or reconstruction of skin tissue, indicates that it also facilitates regeneration and/or reconstruction of the other all organs and tissues (e.g. liver, kidneys, digestive tract, digestive tube, pancreas, muscular tissues, respiratory organs, sensory organs, urogenital organs, endocrine organs, cornea, mucosa, mouth mucosa, nervous tissues, etc.). Namely, after partial hepatectomy, or in pathological conditions such as crush syndrome, acute tubular necrosis, acute renal failure, hepatitis, nephritis, partial excision of pancreas, peptic ulcer, ulcerative colitis, Crohn's disease, corneal injury, corneal erosion, corneal ulcer, stomatitis, aphthous stomatitis, oral mucosal injury, tympanic membrane injury, etc., in order to promote regeneration or reconstruction of said organs, intravenous administration or local administration of ginsenosides, especially ginsenoside Rb₁, appears to be effective.

It was also demonstrated in the present experiments that ginsenoside Rb₁ of the present invention promoted regeneration and/or reconstruction of blood vessels, regeneration of peripheral nerves, and regeneration of hair follicles, sweat glands and sebaceous glands in the defected skin region caused by open wound. According to these results, the low dosage, low doses and low concentrations of ginsenosides, especially ginsenoside Rb₁, can be utilized as agents for prevention, therapy or treatment of diseases with a main symptom of blood flow disorder (aortitis syndrome, peripheral arterial obliteration, thromboangitis obliterans, arteriosclerosis obliterans, Raynaud's disease, Raynaud's syndrome, angina pectoris, myocardial infarction, pulmonary embolism, cerebral infarction, ischemia reperfusion injuries of liver, kidney and heart, cerebrovascular disorders (diseases), piles, hemorrhoids, moyamoya disease, etc.), hair restorers or hair tonic, agents for prevention of progress of depilation, agents for therapy or treatment of depilation (alopecia areata, androgenic alopecia, male pattern alopecia and diffuse depilation), or as agents for prevention, therapy or treatment of peripheral nerve disorders (diseases) and neuralgia.

According to the above experimental results, it has been demonstrated that the preparation(s) for intravenous administration comprising low dosage, low doses or low concentrations of ginsenosides, especially ginsenoside Rb₁, or salts thereof was useful for treatment, prevention or therapy of diseases caused by skin injury, wound (excised wound or open wound), traumatic injury, trauma or defect, or diseases causing histopathological changes of skin through excellent wound healing-promoting action or skin tissue regeneration or reconstruction-promoting effect.

Low dosages, low doses or low concentrations of ginsenosides, especially ginsenoside Rb₁, or salts thereof of the present invention are known as the components of ginseng and are substances with extremely low adverse effect.

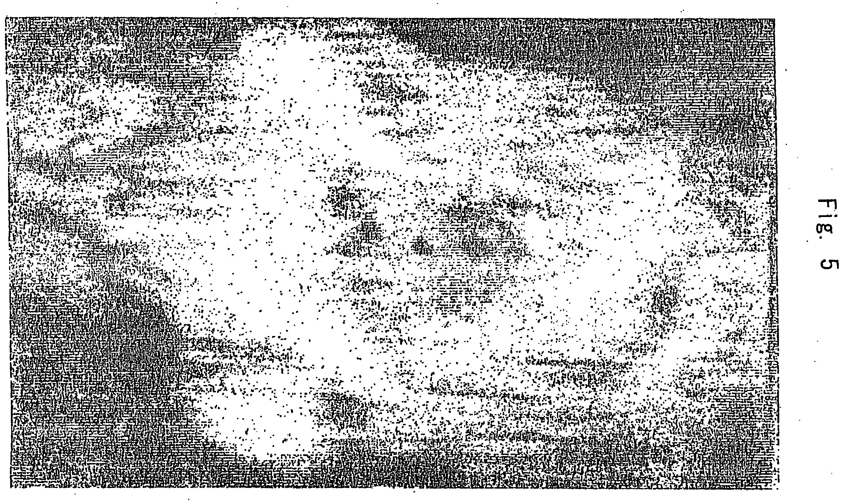

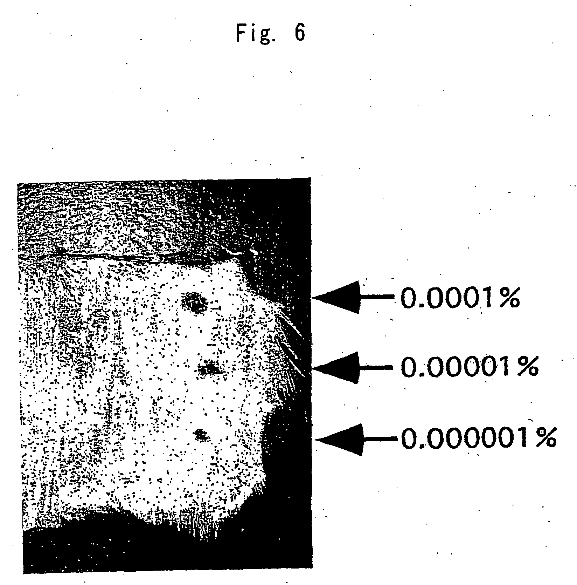

We have examined whether or not external or topical administration of ginsenoside Rb₁ to skin can exhibit effectiveness and efficacy on diseases with defect of skin tissue (bedsore, skin ulcer, burn, frostbite, radiation injury, open wound, ultraviolet injury, electric injury, etc.). For that purpose, for example, by using Wistar male rats (body weight about 300 g), the punch biopsies with diameter 6 mm were made in the dorsal region of animals after shaving under inhalation anesthesia to prepare 3 regions of open wound. Among them, 0.1 g of ophthalmic white Vaseline (Propet) containing 0.01% by weight or 0.001% by weight of ginsenoside Rb₁ was spread once for every day in two regions, and in the remaining open wound, the equal amount of ophthalmic white Vaseline (propet) was spread. Nine days after preparing open wound, the skin including wound regions was photographed. Further, we have examined the effects of external or topical cutaneous spread of 0.0001% by weight, 0.00001% by weight or 0.000001% by weight of ginsenoside Rb₁ by the same procedure. Experimental animals were euthanized by anesthetia immediately before photographing, then wound regions were dissected out after photographing or after dissecting out wound regions, photographing was performed. Thereafter, the wound region tissues were stored in the fixative. Result is shown in Fig. 5 and Fig. 6. Fig. 5 and Fig. 6 are photographs in place of drawings.

In Fig. 5, the first wound from the top is a case of external administration (external spread) of only propet after preparation of the open wound, which shows obvious red colored open wound (in the photograph, black open wound). In Fig. 5, the second wound from the top is a case of external spread of propet containing 0.001% by weight of ginsenoside Rb₁ (external cutaneous administration), and the open wound. area is slightly reduced as compared with the first wound which is externally or topically spread only with propet. The third open wound which is externally or topically spread with propet containing 0.01% by weight of ginsenoside Rb₁ shows no difference as compared with the control of the first wound. Namely, propet containing 0.01% by weight of ginsenoside Rb₁ (i.e. propet containing 100 µg/g ointment base) does not promote significantly regeneration and/or reconstruction of skin tissue even after spreading on the open wound, consequently wound healing does not progress so significantly, as a result, it may not so suppress cicatrization or scar formation.

As shown in Fig. 6, on the second and the third from the top, propet containing 0.00001% by weight (10⁻⁵% by weight) or 0.000001% by weight (10⁻⁶% by weight) of ginsenoside Rb₁ shows superior effect as compared with propet containing 0.0001% by weight of ginsenoside Rb₁. This demonstrates that in case that the agent(s) for external or topical application to skin comprising or consisting essentially of low concentrations of ginsenoside Rb₁ is externally spread or externally sprayed on open wound, almost the same effects and efficacy as those of continuous intravenous administration of low dosage or low doses of ginsenoside Rb₁ can be obtained. Further, as a result of extracutaneous or external skin administration of 0.000001% by weight of ginsenoside Rb₁, obvious hair restoration or hair growth from the regenerated open wound was observed. Namely, matters in relation to the effects, efficacy and usages of continuous intravenous administration of low dosage or low doses of ginsenoside Rb₁, as explained in detail in the present invention, can be applied mostly to local administration or external administration of ginsenoside Rb₁ onto lesions. The extracutaneous spread or external skin spread of low concentrations of ginsenosides, especially ginsenoside Rb₁, is thought to promote regeneration and/or reconstruction of cutaneous epidermal tissue, connective tissue of the dermis, dermal papilla, blood vessels, sebaceous glands, nerves, sweat glands, hair papillae, pilomotor muscles, hair follicles, etc. and facilitates wound healing. As far as we know, the effect of extracutaneous administration or topical cutaneous administration of ginsenoside Rb₁ is far superior to the effects of peptide growth factors (PDGF, EGF and bFGF). The ointment or agents for external use or topical application comprising containing low concentrations of ginsenoside Rb₁ used in the present experiments can promote regeneration or reconstruction of lesioned tissues by externally or topically administering to not only skin but also all organs and tissues with injury or histopathological changes (cornea, oral cavity, external ear, tympanic membrane, vagina, uterus, urinary tract, rectum, anus, etc.). As a result of the present experiments, it was found that the amount of admixing ginsenosides, especially ginsenoside Rb₁, is 0.1 mg or less, preferably 0.0001 mg or less per 10 g of propet. Namely, the amount of extracutaneous administration of ginsenosides, especially ginsenoside Rb₁, in human with skin diseases or cutaneous diseases is, though depending on individual difference or disease condition, far smaller than previously considered. In the present experiments, the effect of high concentrations of ginsenoside Rb₁ (0.0001% by weight or more) is dispersed depending on animals or varies from animal to animal. In rats, even though high concentrations of ginsenoside Rb₁ ranging 0.001% by weight - 0.0001% by weight were externally or topically administered to open wound, since animals frequently licked the open wound, as a result, the concentration of ginsenoside Rb₁ in the open wound region was reduced and thus preferable results appeared to be obtained sometimes. However, since actions of licking the open wound hardly occurs in human, in order to promote wound healing, external or topical administration of lower concentrations of ginsenoside Rb₁ (for example, concentrations less than 0.00002% by weight) is preferable in human.

As described hereinbefore, the fact that the extracutaneous spread or external skin spread of low concentrations of ginsenosides, especially ginsenoside Rb₁, promotes regeneration and/or reconstruction of cutaneous epidermal tissue, connective tissue of the dermis, dermal papilla, subcutaneous tissue, blood vessels, pilomotor muscles, sebaceous glands, sweat glands, hair papillae, hair follicles, etc., demonstrates quite naturally that extracutaneous spread or external skin application of ginsenoside Rb₁ also promotes regeneration and/or reconstruction of epidermal cells, epidermal keratinocytes, Merkel cells, melanocytes, Langerhans cells, cornified cells, hornified cells, keratinized cells, fibroblasts in the dermis and subcutaneous tissue, vascular endothelial cells, vascular smooth muscle cells, sebaceous gland cells, adipocytes, sweat gland cells, hair follicle cells, pilomotor muscular cells, mesenchymal cells, skin stem cells, etc. Namely, ginsenosides, especially ginsenoside Rb₁, are thought to promote regeneration and/or reconstruction of all cells and secretion thereof, which constitute skin tissues. On the other hand, various symptoms of skin accompanied by aging (shrinkage or atrophy of the skin, vulnerability to infection, easy infectivity, slackening, loosening, dermatrophia, flabbiness, itching, roughness, rhagade, crack, fissure, asteatosis, oligosteatosis, exfoliation or ablation of hornified cells, cornified cells, keratinized cells, keratinocytes and stratum corneum, chapped, ephelis, spots, blotches, lines, furrows, freckle, depilation, alopecia, poliosis, gray hair, scurf, dandruff, pigmentation, sunburn, dry skin, etc.) are thought to occur due to gradual death or dysfunction of the skin tissue-constituting cells mentioned above, which are damaged by ultraviolet ray or vital senility and are impossible to regenerate to the original healthy condition. For example, roughness of the skin, dry skin, depilation, alopecia, crack, exfoliation of kiratinocytes and stratum corneum, fissure, chapping, chapped, oligosteatosis, asteatosis, itching, etc. accompanied by aging and senility are thought to develop due to lack of regeneration after functional disability or death of cells in the sweat glands, hair follicles and sebaceous glands of skin. Further, sunburn, pigmentation, spots, blotches, ephelis, freckle, etc. can be produced due to lack of regeneration of skin cells to the original state, even if skin cells, which are irradiated by sun light or ultraviolet ray, are gone to death. Further, it can be said that wrinkles, lines, furrows flabbiness, shrinkage, atrophy, etc. of skin are generated as a result that fibroblasts or mesenchymal cells of the dermis and subcutaneous tissue fall into dysfunction or decrease in number in line with aging and that the dermis or subcutaneous tissue can not maintain sufficient collagen fibers, elastic fibers, reticular fibers or extracellular matrices (matrix). Poliosis, gray hair and vulnerability to infection can be generated due to dysfunction of melanocytes or Langerhans cells.

Since low concentrations of ginsenosides, especially ginsenoside Rb₁, can promote regeneration and/or reconstruction of all cells constituting skin tissue, if they are utilized as a cosmetic composition(s), various symptoms caused by decrease in the constituting cells of skin (cell death) and/or by dysfunction of the cells in association with aging (shrinkage or atrophy, easy infectivity, vulnerability to infection, flabbiness, looseness, rhagades, itching, crack, roughness, oligosteatosis, asteatosis, exfoliation of keratinocytes, cornified cells or hornified cells, exfoliation or ablation of the stratum corneum, chapped, ephelis, wrinkles, lines, furrows, freckles, poliosis, gray hair, scurf, dandruff, depilation, pigmentation, insufficient regeneration, sunburn, aplasia, dryness, etc. of skin), can be prevented, reduced or improved. Further, ginsenoside Rb₁ is thought to increase the expression of a cell-death suppressive gene product Bcl-x_{L} and to protect all skin cells including epidermal cells, epidermal keratinocytes, hornified cells, cornified cells, keratinized cells, sebaceous gland cells, hair follicle cells, pilomotor muscular cells, sweat gland cells, fibroblasts, stem cells, mesenchymal cells, vascular endothelial cells, vascular smooth muscle cells, adipocytes, etc. as described in the prior patent application (JP Appln. No. Hei 10-365560, PCT/JP99/02550, Brain cell or nerve cell-protective agents comprising ginsenoside Rb₁ and PCT/JP00/04102, Brain cell or nerve cell-protecting agents comprising ginseng) of the present inventors (Sakanaka and Tanaka); consequently, it may be able to prevent death and functional disorder of constituting cells of skin accompanied by aging and senility in advance. As explained hereinabove, ginsenosides, especially ginsenoside Rb₁, can protect all cells constituting the skin and even if once cells of skin are going to death or fall into dysfunction, senile symptoms of the skin accompanied by aging (shrinkage, atrophy, vulnerability to infection, easy infectivity, looseness, flabbiness, slackening, itching, crack, rhagades, fissure, roughness, oligosteatosis, asteatosis, exfoliation of keratinocytes, keratinized cells, cornified cells or hornified cells, exfoliation of the stratum corneum, chapped, spots, blotches, lines, ephelis, chloasma, wrinkles, furrows, freckles, gray hair, poliosis, dandruff, scurf, depilation, alopecia, pigmentation, sunburn, poor regeneration, insufficient regeneration, aplasia, dryness, etc. of skin) are thought to be prevented, improved or reduced through regeneration of the skin cells by ginsenosides, especially ginsenoside Rb₁. Namely, ginsenosides, especially ginsenoside Rb₁, can be said to prevent, improve or reduce senile symptoms of the skin accompanied by aging through two potent actions: cytoprotective action and action for promoting tissue and cell regeneration. Moreover, as demonstrated in the experimental results of the present invention and the prior patent application (Japanese Patent Appln. No. Hei 10-365560, PCT/JP99/02550), ginsenosides, especially ginsenoside Rb₁, can exhibit cytoprotective action and action for promoting tissue and cell regeneration, when the extracellular fluid concentrations in lesioned tissues and/or cutaneous tissues are 1 ng/ml or less, preferably 10 pg/ml or less, more preferably 100 fg/ml or less. Further, as shown in examples hereinbelow, low concentrations, low doses or low dosages of ginsenosides, especially ginsenoside Rb₁, can promote regeneration and/or reconstruction of mucosal tissues and cure morsus of oral mucosa. Consequently, when trace amounts of ginsenosides, especially ginsenoside Rb₁, are admixed into every cosmetics or drugs for health promotion [cosmetic lotion (skin lotion), milky lotion, beauty liquid, agent for massage, agent for pack, emulsion, foundation cream, hand cream, gel, lotion, emulsion, powder, hair dye, hair manicure, cold cream, eye shadow, cleansing cream, cleansing foam, night cream, beauty cream, troches, candy for cough, face powder, lipstick, bath gel, cosmetic soap, water for health promotion, isotonic water, ice for water dilution of alcohol, sherbet, ice cream, alcohol beverage, collyrium, eye wash, cleansing liquid, collutorium, shampoo, hair rinse, tooth powder, tooth paste, lip cream, makeup base, UV liquid foundation, powder foundation, etc.] and used to maintain the extracellular fluid concentrations of ginsenosides, especially ginsenoside Rb₁, in local region of skin or local region of mucosa to the low levels hereinbefore, excellent effects can be obtained on senile symptoms of the skin and mucosa accompanied by aging (shrinkage, atrophy, vulnerability to infection, easy infectivity, flabbiness, looseness, slackening, itching, fissure, crack, roughness, poor regeneration, aplasia, epithelial exfoliation or ablation, mucosal exfoliation or ablation, oligosteatosis, asteatosis, exfoliation of keratinocytes, keratinized cells, hornified cells or cornified cells, exfoliation of the stratum corneum, chapped, ephelis, rhagades, blotches, spots, lines, furrows, wrinkles, freckles, poliosis, gray hair, scurf, dandruff, depilation, alopecia, pigmentation, sunburn, dryness, etc.). For example, only defect of skin lipid secretion (namely asteatosis or oligosteatosis) accompanied by aging or senility causes roughness, chapped, dryness, crack, rhagades, itching, exfoliation of keratinocytes, keratinized cells, cornified cells or hornified cells, etc., but as a result of applying every cosmetics, in which ginsenosides, especially ginsenoside Rb₁, is admixed at low concentrations, protection or regeneration and reconstruction of the sebaceous glands can be promoted and thus the above-described senile symptoms of skin accompanied by aging are thought to be prevented, improved or reduced. Since any cosmetics containing low concentrations of ginsenosides, especially ginsenoside Rb₁, not only protect epidermal cells (cornified cells, keratinized cells, hornified cells) or epidermal keratinocytes but also promote regeneration thereof, as the results of promotion by ginsenosides, especially especially ginsenoside Rb₁, of production and secretion of intercellular lipids (i.e. lipids between keratinized cells, hornified cells or cornified cells in the stratum corneum) and natural humectant factors, dryness and roughness of the skin are suppressed to provide natural moisture in the skin. Further, as the results of admixing ginsenosides, especially ginsenoside Rb₁, a ginseng extract(s) or a crude saponin fraction(s) of ginseng into mineral water, injury of mouth mucosa or digestive tract mucosa (especially esophageal mucosa) caused by alcoholic beverage or high temperature irritation can be improved, prevented or treated. Low concentrations of ginsenosides, especially ginsenoside Rb₁, can be used as a composition(s) for chemical peeling by mixing them with one or more of reagents or administrative agents used in the total process(es) of chemical peeling (before, during and/or after chemical peeling). With regard to examples of base for the cosmetic composition(s), the composition(s) for hair restoring, nourishing and growing, and the composition(s) for chemical peeling in the present invention (ginseng, a ginseng extract(s), a crude saponin fraction(s) of ginseng, ginsenosides and ginsenosides derivatives), are there fats and oils, waxes, hydrocarbons, fatty acids, lower alcohols, higher alcohols, polyalcohols, esters, surface active agents (surfactants), water soluble polymers, etc. The above-mentioned composition(s) for extracutaneous use or external or topical application to skin of the present invention can be admixed with any one or more of other skin cell activator, antiinflammatory agent, active oxygen scavenger, beauty agent, ultraviolet absorber, antiseptics and antifungal agent, emollient agent, natural product extract and retinoic acid.

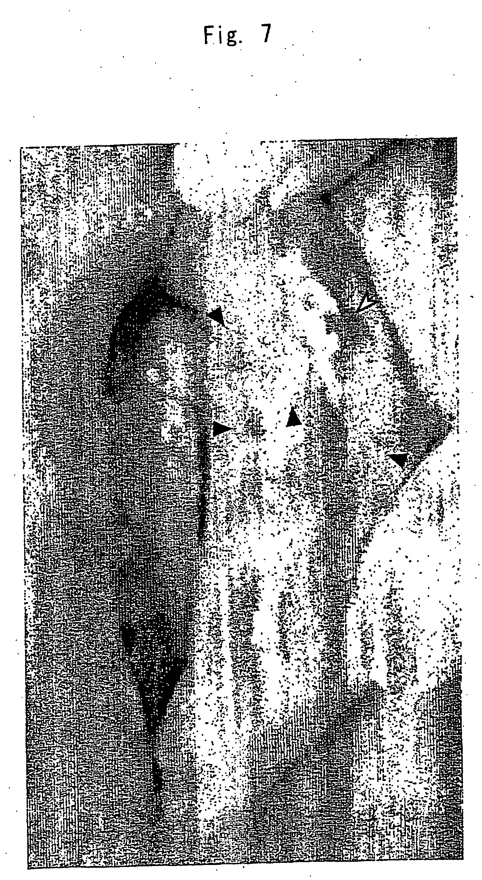

Next, one of the present inventors (Sakanaka) had confirmed by himself whether or not propet containing low concentration of ginsenoside Rb₁ was effective to morsus of mouth or oral mucosa. On May 2nd, 2000, at about 2:00 p.m. after dental treatment, Sakanaka had started to have late lunch before waking up from infiltration anesthesia of the left third branch of the trigeminal nerve and periodontal anesthesia due to heavy hunger. He had bitten himself three times his own left lip mucosa and since he felt iron taste (blood) in his oral cavity, as a result of confirmation of his morsus by a mirror, he found at least five regions of erosion or defect of mouth or oral mucosa, further hematocele or hematoma was found at one spot. Since he concluded that if it (i.e. morsus of oral mucosa) was allowed to leave as it is, aphthous stomatitis would be complicated within several days to require a week to ten days for complete cure, propet containing a low concentration (0.00001% by weight) of ginsenoside Rb₁, which had been confirmed to show effectiveness and efficacy by animal experiments of the present inventors, was applied with small amount to the five regions with erosion or defect of oral mucosa and one hematocele or hematoma region. External or topical application was performed before and after meal and before and after eating between meals. The external or topical application was performed after tooth brushing was performed as much as possible after the meal. Namely, propet containing 0.00001% by weight of ginsenoside Rb₁ was applied externally or topically onto morsus regions of the labial mucosa separately 6 - 10 times a day. A photograph of the labial mucosa at 96 hours after morsus is shown in Fig. 7. Fig. 7 is a photograph in place of drawing.

As shown in the photograph of Fig. 7, though hematocele or hematoma remained at 96 hours after morsus as indicated in the white arrow, morsus regions of labial mucosa (i.e. erosive or defected mouth mucosa) indicated by black arrows were only slightly flared with progressed complete epithelization and wound was thought to be obviously cured. Furthermore, after applying externally or topically propet containing 0.00001% by weight of ginsenoside Rb₁ to the morsus regions, pain in the wounded regions was markedly reduced. As a result of external or topical application of the low concentration of ginsenoside Rb₁ onto the mouth mucosa, since the peripheral nerves excised by morsus were rapidly regenerated in harmony with the epithelization, pain appeared to be reduced. According to the present inventors' experiences, since to cure such morsus requires at least 7 to 10 days, the labial mucosal tissue is rapidly regenerated and reconstructed by external or local administration of the low concentration of ginsenoside Rb₁, and morsus (wound) healing is promoted markedly within 96 hours. Moreover, no complication of aphthous stomatitis was observed after morsus in this case. Effectiveness against mucosal lesion can be expected by local or topical administration of an agent(s) for external use containing ginsenosides, especially ginsenosides Rb₁, with 1 - 10 times a day to the lesion.

Generally, steroid agent such as dexaltin ointment is conventionally used for treatment of aphthous stomatitis in the clinical field, but as is well known, though steroid agent can ease pain of aphthous stomatitis, it shows side effect such that wound healing or cure of defected region of tissue is delayed. Furthermore, external or topical application of steroid agent having immunosuppressive action against oral mucosal lesion, in which various microorganisms are found, is not always preferable by considering complication of infection.

On the other hand, since external mucosal administration of low concentrations of ginsenoside Rb₁ promotes wound healing and epithelization and also promotes regeneration of peripheral nerves in mucosal lesion, it is thought to be an excellent therapeutic method as compared with extramucosal or topical mucosal administration of steroid agent. Moreover, since the low concentration(s) of ginsenoside Rb₁ does not exhibit immunosuppressive action, it can be said as an extremely safe pharmaceutical composition. It is expected to utilize the low concentration(s) of ginsenoside Rb₁ as the first choice drug for aphthous stomatitis.

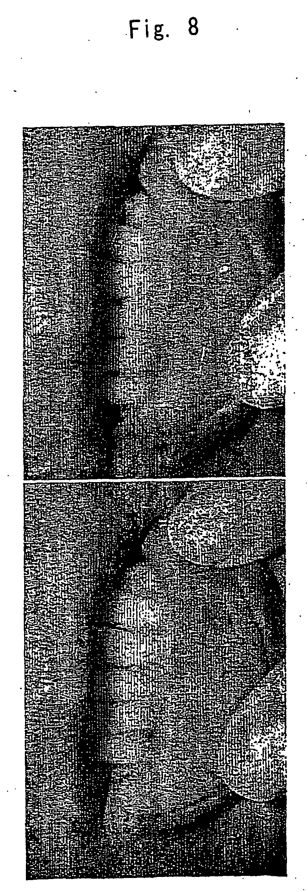

Next, one of inventors of the present invention (Sakanaka) had bitten again left lower labial mucosa on May 26, 2000 during lunch, and propet containing 0.00001% by weight of ginsenoside Rb₁ was applied topically or externally onto the morsus region. A photograph just after morsus is shown in left of Fig. 8, and a photograph at 72 hours after morsus is shown in right of Fig. 8. Fig. 8 shows photographs in place of drawing.

As shown in Fig. 8, if low concentration of ginsenoside Rb₁ was externally or topically applied on the mucosa, it was found that morsus was rapidly cured without complication of aphthous stomatitis. Consequently, low concentrations of ginsenoside Rb₁ can promote regeneration and/or reconstruction of not only the skin tissue but also the mucosal tissues such as human mouth (oral) mucosa and can facilitate wound healing.rapidly. Based on such the fact, low concentrations of ginsenosides, especially ginsenoside Rb₁, can be said to exhibit effectivess and efficacy for all diseases and pathological conditions causing histopathological changes of mucosa or intraoral tissues including mouth mucosa through tissue regeneration and reconstruction-promoting action. Examples of these diseases are caries, pulpitis, periodontal disease, marginal periodontitis, stomatitis, glossitis, recurrent aphtha, intraoral aphtha, halitosis, mouth odor, oral dysesthesia, odontogenic infection, oral mucosal morsus, lingual morsus, oral mucosal burn, lingual burn, oral mucosa injury, gingivitis, alveolar pyorrhea, catarrhal stomatitis, gangrenous stomatitis,. Vincent stomatitis, aphthous stomatitis, acute herpetic gingivostomatitis, herpangina, herpes zoster, oral mucosa erosion, oral mucosal ulcer, decubitus ulcer, radiation stomatitis, pemphigus, oral candidiasis, lichen planus, Riga-Fede disease, bald tongue, red flat tongue, corneal erosion, corneal ulcer, dry eye, Sjögren's syndrome, etc.

The fact that external or topical application of the low concentrations of ginsenoside Rb₁ was effective for morsus of mouth mucosa supports that ginsenosides, especially ginsenoside Rb₁, promote regeneration or reconstruction of mouth or oral mucosa including epidermis of mucosa, lamina propria, salivary glands, mucous glands, mixed glands, connective tissue, muscular tissue, blood vessels, peripheral nerves, epithelial cells, glandular cells, myoepithelial cells, fibroblasts, stem cells, mesenchymal cells, vascular endothelial cells, smooth muscle cells, muscle cells, extracellular matrices (matrix), collagen fibers, elastic fibers or reticular fibers.

An agent(s) for external use on mucosa or topical application to mucosa comprising low concentrations of ginsenosides, especially ginsenoside Rb₁ exhibits marked effectivess for diseases and pathological states causing histopathological changes of mucosa by applying on not only mouth mucosa but also digestive tract mucosa, nasal mucosa, eye mucosa (conjunctiva and cornea), vaginal mucosa, uterine mucosa, urinary mucosa, vesical mucosa, trachea and bronchial mucosa. In particular, external or topical administration of ginsenosides, especially ginsenoside Rb₁ is useful for wound, burn, inflammation, erosion, ulcer, defect, pollinosis, or spring conjunctivitis of mucosa. Further, an agent(s) for external use on mucosa or topical application to mucosa comprising low concentrations of ginsenosides, especially ginsenoside Rb₁, can be used as a drug(s) for health promotion for preventing, improving or treating senile symptoms of mucosa (atrophy, shrinkage, epithelial exfoliation or ablation, mucosal exfoliation or ablation incomplete or poor regeneration, crack, rhagades, chapped, dryness, etc.). In addition, the effects, efficacy and usages of low concentrations of ginsenosides, especially ginsenoside Rb₁, described hereinbefore can be applied to a crude saponin fraction(s) of ginseng, a ginseng extract(s) or ginseng. Of course, the effects, efficacy and usages of ginsenoside Rb₁ as described above are in common with those of ginsenosides derivatives, especially dihydroginsenoside Rb₁, which will be explained later.

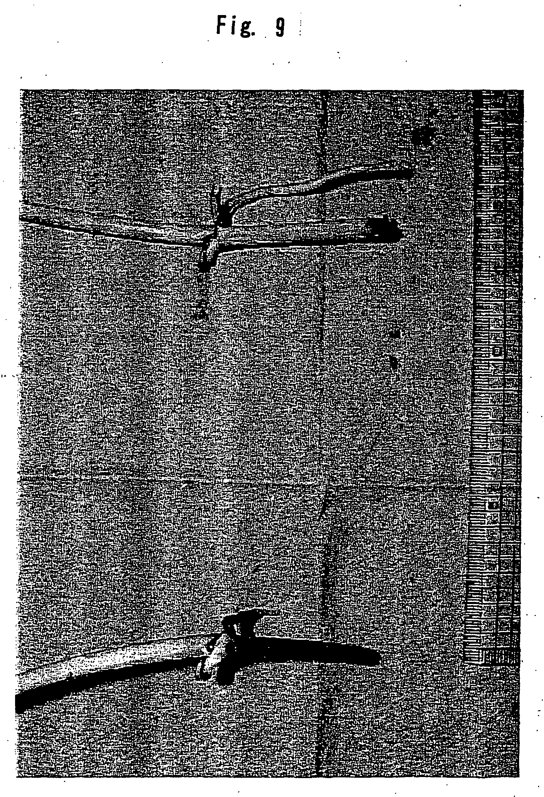

Next, we have examined whether ginsenosides, especially ginsenoside Rb₁, can promote generation, regeneration or reconstruction of not only skin tissue or mouth mucosal tissue but also plant tissues. For that purpose, one of foliage plants, pothos is selected. Six cuttings resembled with each other from the parent plant of pothos in the room of one of the inventors (Tanaka) were collected. Among them, 3 cuttings were cultured by hydroponics with the use of only water and the remaining three were cultured in water containing ginsenoside Rb₁ at a concentration of 100 fg/ml. A photograph of cuttings on day 13 culture is shown in Fig. 9. Fig. 9 is a photograph in place of drawing.

Left photograph in Fig. 9 is the cutting (stem and branch of pothos) cultured with only water and right photograph in Fig. 9 is the cutting cultured with water containing ginsenoside Rb₁ at a concentration of 100 fg/ml. In case that the cutting of pothos is cultured with water containing the low concentration of ginsenoside Rb₁ (100 fg/ml) in hydroponics, growth of root is promoted.

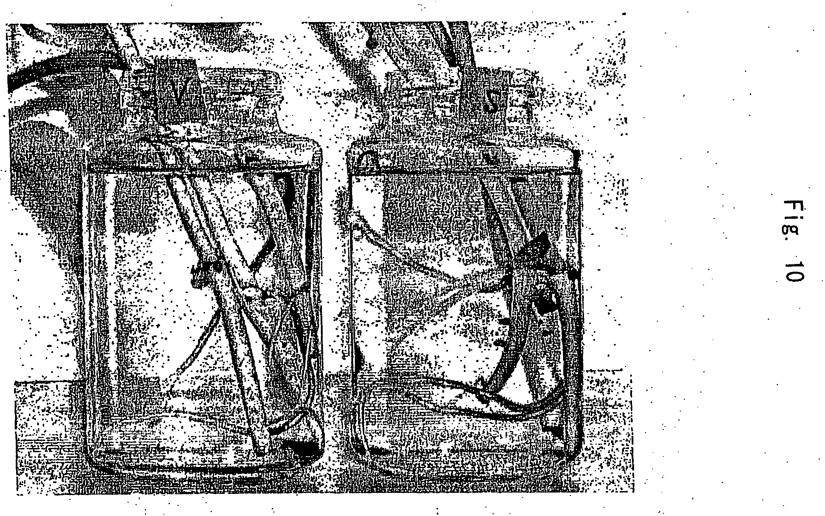

Then, we have further continued cultivation of the above cuttings with hydroponics and on-day 22, again photographs were taken. Result is shown in Fig. 10. Fig. 10 is a photograph in place of drawing. Left photograph in Fig. 10 is the cuttings of pothos cultured with only water for 22 days and right photograph in Fig. 10 is the cuttings cultured with water containing the low concentration of ginsenoside Rb₁ (100 fg/ml). Waters with or without containing ginsenoside Rb₁ for hydroponics were exchanged once in a week. We have further made observation of rooting region on day 27.

As shown in Fig. 10, when the cuttings were cultured with water containing the low concentration of ginsenoside Rb₁ (100 fg/ml) for 22 days, as compared with pothos cultured with only water, many roots were generated or regenerated to grow to contact glass vessel for hydroponics. Further on day 27, secondary rooting arising from the primary rooting regions was clearly observed on all three cuttings of pothos cultured with water containing the low concentration of ginsenoside Rb₁ (100 fg/ml). However, no secondary rooting was observed on three cuttings of pothos cultured only with water. Consequently, as the results of the present experiments, it was demonstrated that.low concentrations, low doses and/or low dosages of ginsenosides, especially ginsenoside Rb₁, promoted not only skin tissue and human mouth mucosal tissue but also rooting, budding, growth, differentiation, generation, regeneration or reconstruction of plant tissue.

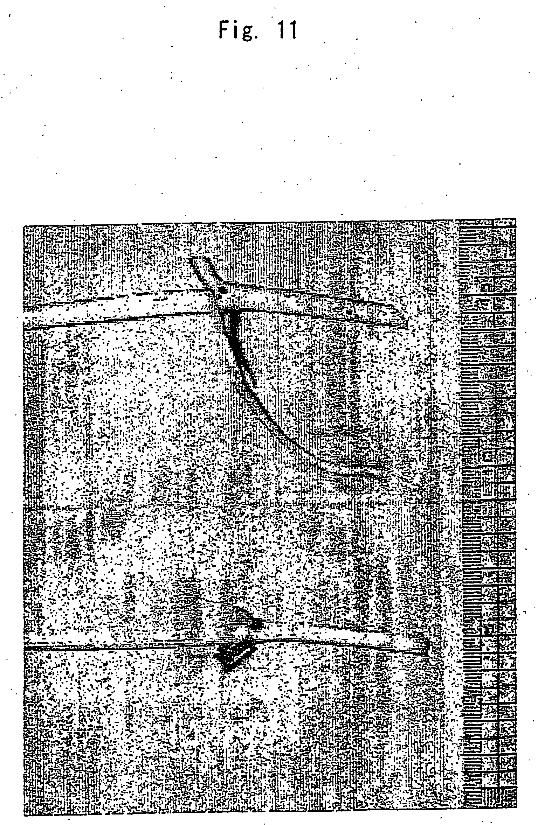

Next, we have examined whether a crude saponin fraction of ginseng, like ginsenoside Rb₁ at low concentrations, can promote, generation, regeneration or reconstruction of the cutting of pothos. For that purpose, two cuttings resembled with each other from the parent plant of pothos were collected. Among them, the one cutting was cultured with only water and the remaining one was cultured in water containing the crude saponin fraction of ginseng at a concentration of 1450 fg/ml. A photograph of cuttings on day 14 culture is shown in Fig. 11. Fig. 11 is a photograph in place of drawing. Left photograph in Fig. 11 is the cutting cultured with only water and right photograph in Fig. 11 is the cutting cultured with water containing the crude saponin fraction of ginseng (1450 fg/ml). In case that the cutting of pothos was cultured with water containing the low concentration of crude saponin fraction of ginseng (1450 fg/ml) in hydroponics, generation, regeneration or growth of root (i.e. rooting) was promoted. Namely, the crude saponin fraction(s), in the same manner as in ginsenoside Rb₁, promotes generation, regeneration or reconstruction of plant tissue. Quite naturally, a ginseng extract(s) and ginseng containing the crude saponin fraction(s) are also to have the same action. Namely, it can be said that ginseng, a ginseng extract(s), a crude saponin fraction(s) of ginseng and ginsenosides, especially ginsenoside Rb₁, can promote regeneration, generation and reconstruction of all vital, viable or living tissues (animal and plant tissues).

As demonstrated in the experiments hereinbefore, when plant tissue such as cutting of pothos is cultured in aqueous solution containing 100 fg/ml of ginsenoside Rb₁ or 1450 fg/ml of crude saponin fraction, generation of root is obviously promoted as compared with the control cutting. Consequently, as described hereinbefore, a ginseng extract(s) or ginseng containing ginsenosides, especially ginsenoside Rb₁, or a crude saponin fraction(s) of ginseng can promote rooting, budding, growth, differentiation, generation, regeneration or reconstruction of not only animal tissues but also plant tissues.

Furthermore, it was found that the extracellular fluid concentrations of ginsenoside Rb₁ or a crude saponin fraction which could promote rooting, budding, growth, differentiation, generation, regeneration or reconstruction of plant tissues were, as same manner in case of regeneration and reconstruction of animal tissues (skin tissue and mouth mucosal tissue), extremely low. Consequently, ginseng, a ginseng extract(s), a crude saponin fraction(s) of ginseng and ginsenosides, especially ginsenoside Rb₁, can be applied for cultivation, growth or preservation of plant, preservation, cultivation or growth of fresh flower, aqueous cultivation, hydroponics, cultivation or growth of farm products, cultivation or growth of vegetables, cultivation and growth of fruits, cultivation and growth of tobacco, cultivation of mushrooms, cultivation of medicinal plant or cultivation and growth of tea-leaves. A rooting, budding, growth or differentiation promoter or fertilizer additives comprising the above described ginseng, a ginseng extract(s), a crude saponin fraction(s) of ginseng and/or ginsenosides, especially ginsenoside Rb₁, can be admixed or added to any fertilizers, preferably at low concentrations, or can be used as rooting, budding, differentiation, regeneration, reconstruction, generation or growth promoter for plant tissues independently. Of course, ginsenosides derivatives, especially dihydroginsenoside Rb₁, hereinbefore described promote, in the same manner as ginsenoside Rb₁, rooting, budding, generation, growth, regeneration, differentiation or reconstruction of plant tissues and are utilized for cultivation, growth and preservation of farm products, plants or vegetables as described hereinbefore.

The fact that ginsenosides, especially ginsenoside Rb₁, and a crude saponin fraction(s) of ginseng can promote rooting, budding, differentiation, growth, generation, regeneration or reconstruction of not only animal tissues (skin tissue and mouth mucosal tissue) but also plant tissues, indicates that ginseng, a ginseng extract(s), a crude saponin fraction(s) of ginseng and ginsenosides, especially ginsenoside Rb₁, promote rooting, budding, differentiation, growth, generation, regeneration or reconstruction of all vital, viable or living tissues. Consequently, ginseng, a ginseng extract(s), a crude saponin fraction(s) of ginseng and ginsenosides, especially ginsenoside Rb₁, can be utilized as additives for feeds for livestock, cultivated fish and shellfish and pet animals. For example, in the cultivation of fish and shellfish, crustacean, eel, pearl shell, pearl oyster, conger, etc., the addition of low concentrations of ginseng, ginseng extract(s), crude saponin fraction(s) of ginseng and/or ginsenosides, especially ginsenoside Rb₁, together with conventional feeds, to sea water or fresh water is thought to promote growth of these aquatic or marine resources. Of course, ginseng, a ginseng extract(s), a crude saponin fraction(s) of ginseng and ginsenosides, especially ginsenoside Rb₁, can protect marine resources such as fish and shellfish, crustacean, eel, conger, etc. from trauma, wound, pathological microorganisms, biohazards, endocrine disrupters, environmental pollution, toxins, etc. through their cytoprotective actions. Namely, feed additives of the present invention will be essential for securing human from forthcoming food crisis. The fact that a crude saponin fraction(s) promotes regeneration, generation or reconstruction of plant tissues supports that a ginseng extract(s) or ginseng containing low concentrations of the crude saponin(s) or crude saponin fraction(s) can be utilized as a cosmetic composition(s), health drug composition(s), veterinary drug composition(s) or pharmaceutical composition(s) by promoting regeneration or reconstruction of skin tissue or mucosal tissues. Of course, ginsenosides derivatives, especially dihydroginsenoside Rb₁, hereinafter described can be utilized, in the same manner as ginsenoside Rb₁, as a growth promoter(s) of aquatic or marine resources described hereinbefore.

In the before-mentioned cases of mouth mucosal morsus, the inventor (Sakanaka) showed that external or topical application of propet containing 0.00001% by weight (10⁻⁵% by weight) of ginsenoside Rb₁, 6 - 10 times a day, regenerated and reconstructed rapidly the mouth mucosal tissue and cured wound as well as preventing aphthous stomatitis in advance. However, quite naturally, even if propet containing 0.00001% by weight (10⁻⁵% by weight) of ginsenoside Rb₁, is applied externally or topically onto the mouth mucosal morsus region, it can be estimated that the extracellular fluid concentration of ginsenoside Rb₁ in proximity to morsus region is rapidly decreased by an action of saliva. It can be thought that propet containing 0.00001% by weight of ginsenoside Rb₁, may promote sufficiently regeneration and/or reconstruction of oral mucosal tissue with morsus even though considerably diluted. Namely, ginsenoside Rb₁ can be estimated to exhibit effectivess and efficacy at far lower concentrations than 0.00001% by weight. Consequently, when an agent(s) for external use or topical application comprising ginsenoside Rb₁ is applied onto skin wound lesion, since the concentration of ginsenoside Rb₁ in the said agent for external use or topical application is not thought to be significantly diluted, it may be preferable to use lower concentrations of ginsenoside Rb₁ than 0.00001% by weight. Consequently, the present inventors examined the effects of external or topical application of propet containing the lower concentrations of ginsenoside Rb₁ onto open wound of rat skin hereinbefore described.

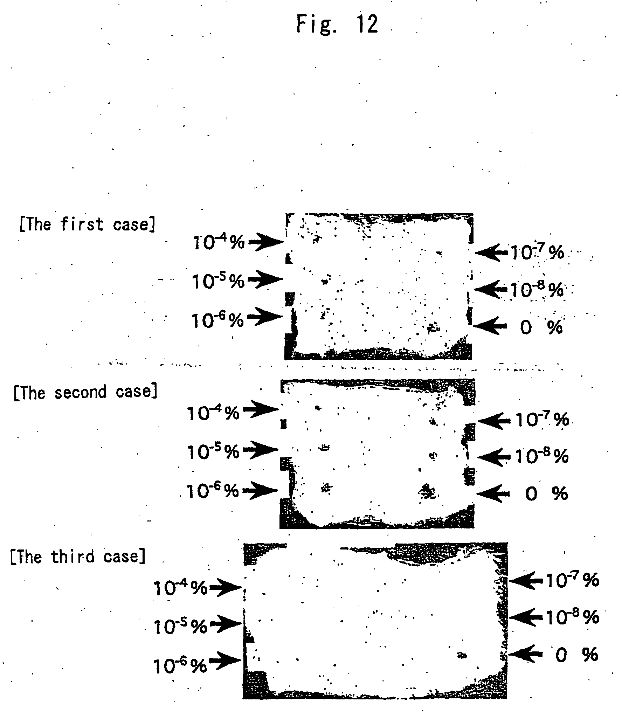

The punch biopsy with diameter 6 mm was performed in the dorsal 6 regions of each animal under inhalation anesthesia to prepare open wound. Thereafter, 0.1g of propet (ophthalmic white vaseline) containing ginsenoside Rb₁ at the concentrations of 0.0001% by weight (10⁻⁴% by weight), 0.00001% by weight (10⁻⁵% by weight), 0.000001% by weight (10⁻⁶% by weight), 0.0000001% by weight (10⁻⁷% by weight) and 0.00000001% by weight (10⁻⁸% by weight), respectively, was applied externally, once a day, for 9 days on each open wound. The equal amount of propet alone was applied externally or topically in the control animals. Then, immediately after euthanasia by anesthetization, the skin containing the open wound was dissected out and photographed. The collected skin tissue was preserved in a fixative. Results are shown in Fig. 12. Fig. 12 is a photograph in place of drawing.

The upper column of Fig. 12 shows the first case; the middle column shows the second case; and the lower column shows the third case. In each case, there are three wounds on the left side and three wounds on the right side, totally 6 marks of open wounds. From the upper left side to the lower left side, are shown the case of 10⁻⁴% by weight,. the case of 10⁻⁵% by weight, and the case of 10⁻⁶% by weight, and from the upper right side to the lower right side, are shown the case of 10⁻⁷% by weight, the case of 10⁻⁸% by weight, and the case of 0% by weight (control or carrier alone).

As shown in Fig. 12, even when propet containing ginsenoside Rb₁ at concentrations from 10⁻⁶% by weight to 10⁻⁸% by weight (i.e. ginsenoside Rb₁ at concentrations from 10 ng/g to 100 pg/g) was externally or topically applied to the open wounds, wound healing was obviously promoted as compared with the open wounds externally or topically administered with only propet. In case of external administration of the low concentrations of ginsenoside Rb₁, growing hair was obviously observed in the regions of wound healing. Consequently, in case that ginsenosides, especially ginsenoside Rb₁, are used as an agent(s) for external use on skin or for topical application to skin, the concentration in the agent(s) for external use or topical application is thought to set preferably around 10⁻⁸% by weight or less. Consequently, in case that ginsenosides, especially ginsenoside Rb₁, a crude saponin fraction(s) of ginseng, a ginseng extract(s) or ginseng are used as a composition(s) for hair growth, hair nourishment or rearing, a composition(s) for chemical peeling or as a composition(s) for cosmetics, the concentration in cosmetics should be set less than 0.001% by weight, preferably at 0.00001% by weight (10⁻⁵% by weight) or less, more preferably at 0.00000001% by weight (10⁻⁸% by weight) or less.

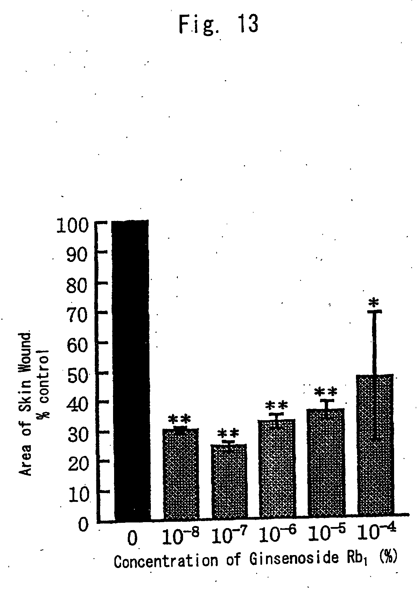

In the experimental cases hereinbefore explained, an area of open wound (flair region) applied externally only with propet is set as a denominator and an area of open wound administered externally with ginsenoside Rb₁ at concentrations from 10⁻⁴% by weight to 10⁻⁸% by weight is set as a numerator, and ratio thereof is calculated. Result is shown in Fig. 13. In Fig. 13, n = 3 and single asterisk (*) indicates significant difference, P < 0.05, and two asterisks (**) indicate significant difference, P < 0.01. Statistical test was performed according to Scheffe's post hoc test.

As shown in Fig. 13, external or topical administration of the low concentrations of ginsenoside Rb₁ to the open wound promoted significantly wound healing. Especially, the fact that external or topical administration of ginsenoside Rb₁ at a concentration of 0.0000001% by weight (10⁻⁷% by weight) or less, i.e. 1 ng/g or less or 1 ng/ml or less of ginsenoside Rb₁, reduced significantly the open wound, strongly supports that when the extracellular fluid concentrations of ginsenoside Rb₁ in lesioned tissues are 1 ng/ml or less, generation, regeneration or reconstruction of vital tissues, living tissues or viable tissues is promoted.

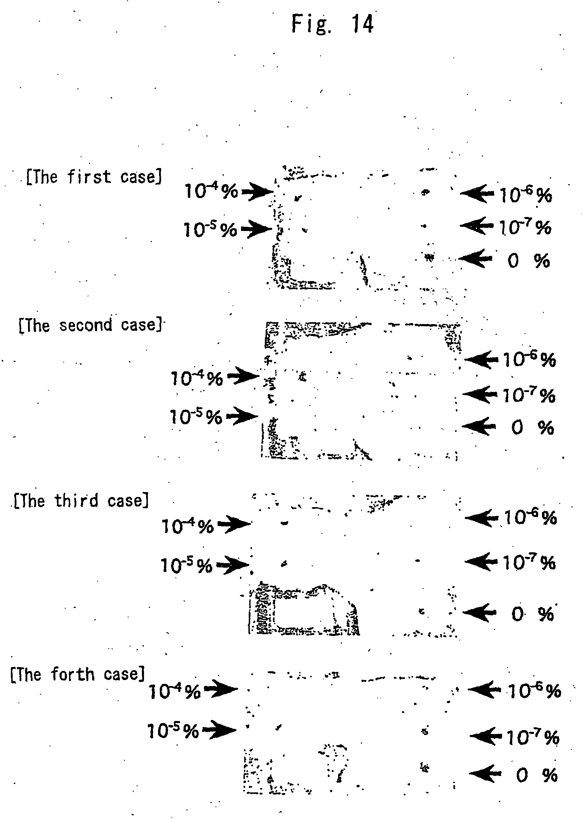

Next, we have examined whether ginsenosides' derivatives, like ginsenoside Rb₁, promote tissue regeneration and/or reconstruction at low concentrations, low doses or low dosages. For that purpose, we have selected dihydroginsenoside Rb₁ as one of ginsenosides' derivatives and examined the open wound-healing effect of the said compound. Details of dihydroginsenoside Rb₁ is described in the specification of PCT/JP00/04102 (Brain cell or nerve cell protecting agents comprising ginseng). The punch biopsy with diameter 6 mm was performed at 5 places in the dorsal region of rats (n = 4) under inhalation anesthesia to prepare open wound. Thereafter, 0.1g of propet containing dihydroginsenoside Rb₁ at concentrations of 0.0001% by weight (10⁻⁴% by weight) , 0.00001% by weight (10⁻⁵% by weight) , 0.000001% by weight (10⁻⁶% by weight) and 0.0000001% by weight (10⁻⁷% by weight), respectively, was applied topically or externally, once a day, for 9 days onto each open wound. The equal amount of propet alone was applied externally or topically in the control animals. Then, immediately after euthanasia by anesthetization, the skin containing the open wound was dissected out and photographed. The collected skin tissue was preserved in a fixative. Results are shown in Fig. 14. Fig. 14 is a photograph in place of drawing. In Fig. 14, four cases are shown, and from the upper column, the first case, the second case, the third case and the fourth case are shown. In each case, there are two wounds on the left side and three wounds on the right side, totally 5 marks of open wounds, and from the upper left side, are shown the case of 10⁻⁴% by weight and the case of 10⁻⁵% by weight; and from the upper right side, are shown the case of 10⁻⁶% by weight, the case of 10⁻⁷% by weight and the case of 0% by weight (control or carrier alone).

As shown in Fig. 14, even when propet containing dihydroginsenoside Rb₁ at contrations from 0.00001% by weight (10⁻⁵% by weight) to 0.0000001% by weight (10⁻⁷% by weight) (i.e. concentrations of dihydroginsenoside Rb₁ from 100 ng/g to 1 ng/g) was externally or topically applied to the open wounds, wound healing was obviously promoted as compared with the open wounds externally or topically administered with propet alone. In case of external or topical administration of the low concentrations of dihydroginsenoside Rb₁, growing hair was obviously observed in the regions of wound healing. Consequently, in case that ginsenosides' derivatives, especially dihydroginsenoside Rb₁, are used as an agent(s) for external use on skin or topical application to skin, the concentration in the agent(s) for external use or topical application is thought to set preferably around 0.0000001% by weight. (10⁻⁷ % by weight) or less. Consequently, in case that ginsenosides derivatives, especially dihydroginsenoside Rb₁, are also used as a composition(s) for cosmetics, the concentration in cosmetics or health-promoting drugs should be set at levels less than 0.001% by weight, preferably at 0.00001% by weight (10⁻⁵% by weight) or less, more preferably at 0.0000001% by weight (10⁻⁷% by weight) or less.

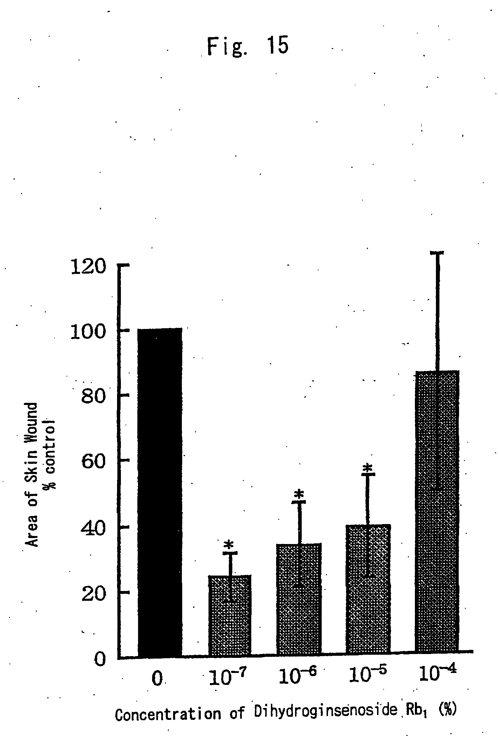

In the experimental cases hereinbefore explained, an area of open wound applied externally or topically with propet alone (ophthalmic white vaseline) is set as a denominator and an area of open wound externally or topically administered with dihydroginsenoside Rb₁ at concentrations from 0.0001% by weight (10⁻⁴% by weight) to 0.0000001% by weight (10⁻⁷% by weight) is set as a numerator, and ratio thereof is calculated. Result is shown in Fig. 15. In Fig. 15, n = 4 and asterisk(*) indicates significant difference, P < 0.05. Statistical test was performed according to Fisher's PLSD.

As shown in Fig. 15, external or topical administration of dihydroginsenoside Rb₁ at concentrations of 0.00001% by weight (10⁻⁵% by weight) or less to the open wound promoted regeneration and/or reconstruction of skin tissue and significantly facilitated wound healing. Especially, the fact that external or topical administration of dihydroginsenoside Rb₁ at concentrations of 0.00001% by weight (10⁻⁵% by weight) or less, i.e. 100 ng/g or less or 100 ng/ml or less of dihydroginsenoside Rb₁, reduced significantly the open wound, strongly supports that when the extracellular fluid concentrations of dihydroginsenoside Rb₁ in lesioned tissues are 100 ng/ml or less, generation, regeneration or reconstruction of vital tissues, living tissues or viable tissues is promoted.

We have performed experiments using cultured nerve cells (neurons) in order to confirm that dihydroginsenoside Rb₁ had the same effects, efficacy and usages as those of ginsenoside Rb₁.

We (Sakanaka and Tanaka) had reported that when cultured nerve cells were exposed for a short time to sodium nitroprusside, apoptosis or apoptosis-like cell death of neurons is induced (Toku, K. et al., J. Neurosci. Res., 53, 415-425, 1998). Using this culturing experimental system, we have already found that ginsenoside Rb₁ at extracellular fluid concentrations of 1 ng/ml or less inhibited apoptosis or apoptosis-like cell death of neurons (Japanese Patent Appln. No. Hei 10-365560, Brain cell or nerve cell-protective agents comprising ginsenoside Rb₁). We have examined the neuroprotective action of dihydroginsenoside Rb₁ using the same experimental system.

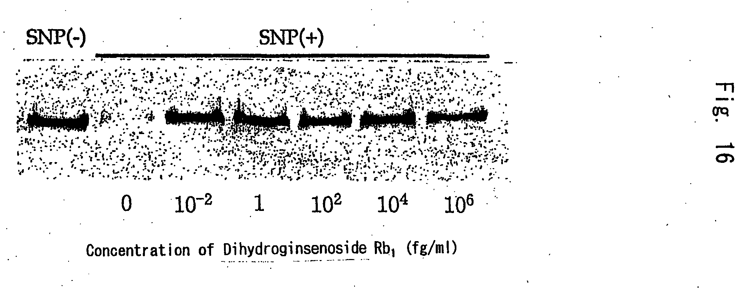

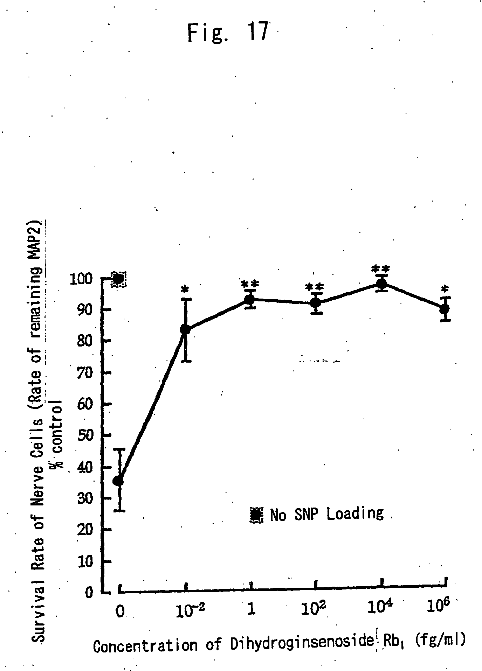

Nerve cells were isolated from the cerebral cortices of fetal rats at gestation day 17 by using trypsin EDTA and seeded onto 24 well plate coated with poly-L-lysine. After incubating the cells in Dulbecco's modified Eagle's medium (DMEM) containing 10% fetal calf serum for 16 hours, the culture medium was replaced with a serum-free medium for neuronal culture containing insulin, transferrin, etc. and the neurons were cultured for 3 or 4 days. On day 3 or 4 of culture, sodium nitroprusside (SNP) at the concentration of 300 µM was added to the neuronal culture and incubated for 10 minutes. Thereafter, the culture medium was replaced with Eagle's minimum essential medium (EMEM) containing dihydroginsenoside Rb₁ (0 - 1 ng/ml) and bovine serum albumin. Sixteen hours after SNP loading, the nerve cells (neurons) were lysed with Laemmli's sample buffer for electrophoresis, and polyacrylamide electrophoresis was performed. After transfer of electrophoresed proteins to nitrocellulose membrane, immunoblotting was performed by using antibody against neuron-specific protein MAP 2. In order to quantify the survival rate of nerve cells or neurons, immunostained MAP2 band was analyzed with densitometry. Results are shown in Fig. 16 and Fig. 17. Fig. 16 is a photograph in place of drawing. In Fig. 17, n = 5 and single asterisk (*) indicates significant difference, P < 0.001, and two asterisks (**) indicate significant difference, P < 0.0001. Statistical test was performed according to Scheffe's post hoc test.

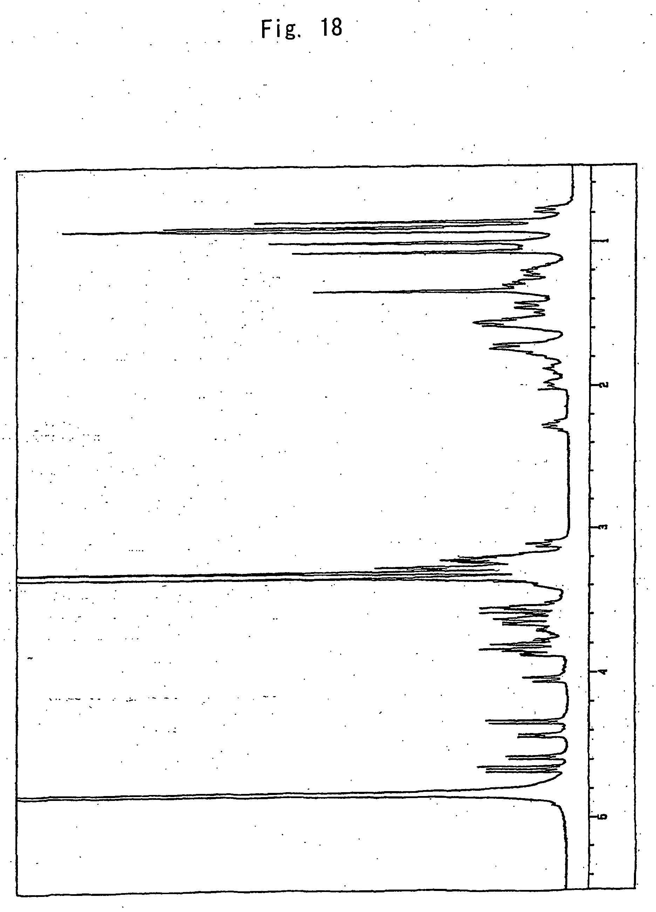

For reference, NMR chart of dihydroginsenoside Rb₁ is shown in Fig. 18 (400 MHz, CD₃OD).

Fig. 16 is a photograph showing result of immunoblotting of microtuble-associated protein 2 (MAP2) in place of drawing. The first lane on the left side indicates the control cultured neurons, showing a clear MAP2 band (i.e. a band of neuronal marker). When SNP treatment was performed, a large number of neurons entered apoptosis or apoptosis-like cell death and the band of MAP2 was clearly weakened as observed in the second lane from left. When dihydroginsenoside Rb₁ is added to the culture medium at the concentrations from 0.01 fg/ml (lane 3) to 1 ng/ml (lane 7), apoptosis or apoptosis-like cell death of neurons caused by SNP is obviously inhibited, as a result, intense bands of MAP2, which is a marker for neuronal survival, were observed.

The above-mentioned immunoblotting experiments of MAP were repeated 5 times and the results were analyzed by densitometry (Fig. 17). As shown in Fig. 17, dihydroginsenoside Rb₁ at the concentrations from 0.01 fg/ml to 1 ng/ml obviously inhibited apoptosis or apoptosis-like cell death of neurons. Namely, dihydroginsenoside Rb₁ exhibits favorable effects on cells, especially nerve cells, in the slightly wider concentration range than that of ginsenoside Rb₁. Consequently, ginsenosides derivatives, especially dihydroginsenoside Rb₁, can exhibit excellent cytoprotective action by inhibiting apoptosis of cells or apoptosis-like cell death, when the extracellular fluid concentrations in lesioned tissues are 100 ng/ml or less, preferably 10 pg/ml or less, more preferably 0.0001 fg/ml - 100 fg/ml. Statistical analysis is performed by Scheffe's post hoc test. *: P < 0.001. **: P < 0.0001.

Judging from the above experimental results that the agent for external use on skin or topical application to skin comprising 0.00001% by weight (10⁻⁵% by weight) of dihydroginsenoside Rb₁ shows excellent open wound-healing effect, ginsenosides derivatives, especially dihydroginsenoside Rb₁, exhibit regenerative and reconstructive action on vital tissues, living tissues or viable tissues, when the extracellular fluid concentrations in the lesioned tissues are 100 ng/ml or less, preferably 10 pg/ml or less, more preferably 0.0001 fg/ml - 100 fg/ml.

On the basis of the experimental results hereinabove, it was elucidated that ginsenoside derivatives, especially dihydroginsenoside Rb₁, as the same manner in ginsenoside Rb₁, exhibited superior skin tissue regeneration and reconstruction-promoting action, wound healing-promoting action and cytoprotective action. Furthermore, it was invented in PCT/JP00/04102 (Brain cell or nerve cell-protecting agents comprising ginseng) that continuous intravenous administration of dihydroginsenoside Rb₁ at low dosages or low doses, as the same manner in ginsenoside Rb₁, exhibited superior therapeutic effect on cerebral infarction. Consequently, it can be said that ginsenosides derivatives, especially dihydroginsenoside Rb₁, have all effects, efficacy and usages of ginsenoside Rb₁ as described in the present invention and prior patent application (PCT/JP99/02550, PCT/JP99/06804 and PCT/JP00/04102). Namely, since ginsenosides' derivatives, especially dihydroginsenoside Rb₁, have actions for promoting generation, regeneration, growth, rooting, budding, differentiation or reconstruction of the vital tissues, living tissues or viable tissues, they are useful as: (1) a pharmaceutical composition(s) or veterinary drug composition(s) for prevention, therapy and/or treatment of all diseases (including pathological conditions) causing histopathological changes; (2) a cosmetic composition(s) or health drug composition(s) for preventing, improving, reducing and treating senile symptoms of skin or mucosa; (3) a composition(s) for growth regulation or additives for fertilizers for cultivation, growth and preservation of farm products, vegetables, plants and fresh flowers; (4) a composition(s) for growth regulation or feed additives for protecting and rearing marine products, marine resources, livestock and fish and shellfish for cultivation; and (5) a pharmaceutical composition(s) for prevention, treatment or therapy of all diseases causing cell death. Ginsenosides derivatives, especially dihydroginsenoside Rb₁, can be used as a composition(s) for external use on mucosa or topical application to mucosa, a cosmetic composition(s), a composition(s) for hair growth, hair nourishment or hair rearing, a composition(s) for chemical peeling, a pharmaceutical composition(s), a health drug composition(s), a composition(s) for growth regulation, feed additives, fertilizer additives, a promoter(s) for rooting, budding, growth and/or differentiation of plants in the same manner as of ginsenoside Rb₁. Proviso that it is necessary to select proper dosages or doses by keeping in mind that ginsenoside derivatives, especially. dihydroginsenoside Rb₁, have possibility to exhibit favorable effects on cells and tissues in a broader concentration range than that of ginsenoside Rb₁.

Further, the present experimental results exhibiting the superior action of dihydroginsenoside Rb₁ to promote regeneration and/or reconstruction of vital tissues, living tissues or viable tissues prove that superior remedies for skin and mucosal diseases, agents for promoting tissue regeneration, generation, rooting, budding, etc. can be newly prepared by utilizing ginsenosides, especially ginsenoside Rb₁, as a leading compound(s). The idea that a pharmaceutical composition(s) for promoting regeneration and/or reconstruction of tissue is prepared by utilizing ginsenosides, especially ginsenoside Rb₁, the synthetic method of which has never been established at present, as a leading compound(s), was not known as far as we know.

In the present invention, it was found that ginsenosides, especially ginsenoside Rb₁, promoted regeneration and/or reconstruction of vital tissues, living tissues or viable tissues. In addition to that, in the prior patent application by the present inventor (Sakanaka), it was invented that low concentrations of ginsenoside Rb₁ exhibited cytoprotective action through Bcl-x_{L} expression-enhancing action (JP Appln. No. Hei 10-365560, PCT/JP99/02550). Further, it was found that ginsenoside Rb₁ promoted cerebrovascular regeneration and/or reconstruction in the prior patent application by the present inventors (Sakanaka and Tanaka) (Japanese Patent Appln. No. Hei 11-340850, PCT/JP99/06804). On the other hand, the present inventors (Sakanaka and Tanaka) have invented that prosaposin-related peptides exhibited cytoprotective action in the same low concentration ranges as in ginsenoside Rb₁ through Bcl-x_{L} expression-enhancing action, namely when the extracellular fluid concentrations in lesioned tissues were 1 ng/ml (5 nM) or less, preferably 10 pg/ml (5 pM) or less, more preferably 100 fg/ml (50 fM) or less (JP Appln. No. Hei 11-185155). Prosaposin-related. peptides mean, as described in JP Appln. No. Hei 11-185155, peptides derived from prosaposin or cytokines having specific common amino acid sequence (consensus sequence). Consequently, ginsenoside Rb₁ and prosaposin-related peptides are, though having different chemical structures, similar in their effects, efficacy and usages. In fact, the inventors (Sakanaka and Tanaka) reported that when ginsenoside Rb₁ or prosaposin-related peptide was infused into the cerebroventricles of rats with permanent obstruction of the cortical branch of the middle cerebral artery (i.e. cerebral infarcted rat) after cerebrovascular obstruction, cerebral infarction-reducting effect and place navigation disability-improving effect were observed (Igase, K. et al., J. Cereb. Blood Flow Metab., 19, 298-306, 1999; Zhang, B. et al., J. Stroke Cerebrovasc. Dis., 7, 1-9, 1998). Considering from these facts, it is easy to estimate that prosaposin-related peptides, like ginsenoside Rb₁, can promote regeneration and/or reconstruction of cerebral blood vessels. To be more specific, it is likely that prosapsin-related peptides, in the same low concentration range as that of ginsenoside Rb₁, can also promote regeneration and/or reconstruction of endothelial cells, smooth muscle cells, fibroblasts, tunica intima, tunica media, tunica externa, connective tissue, collagen fibers, elastic fibers, reticular fibers or extracellular matrix (matrices) in the cerebral blood vessels. As such, based on the fact that the effects, efficacy and usages of ginsenoside Rb₁ and prosaposin-related peptides are similar in each other, prosaposin-related peptides are likely to promote, as same manner in ginsenoside Rb₁, regeneration and/or reconstruction of all vital tissues, living tissues or viable tissues.

In the prior described JP Appln. No. Hei 11-185155, it has been invented that cytokines including erythropoietin, in the same low molar concentration ranges as in prosaposin-related peptides and ginsenoside Rb₁, promoted Bcl-x_{L} expression and exhibited cytoprotective action. Furthermore, the present inventor (Sakanaka) has found that intracerebroventricular administration of erythropoietin to rats with permanent occlusion of the cortical branch of the middle cerebral artery (i.e. cerebral infarcted rat), in the same manner as of prosaposin-related peptides and ginsenoside Rb₁, also exhibited cerebral infarction-reducing effect and place navigation disability-improving effect (Sadamoto, Y. et al., Biochem. Biophys. Res. Commun., 253, 26-32, 1998). Consequently, it is thought that erythropoietin also promotes regeneration and/or reconstruction of vital tissues, living tissues or viable tissues including cerebral blood vessels. Since we (Sakanaka and Tanaka) have found that intracerebroventricular administration of interleukin 3 (IL-3), one of colony stimulating factors, as like erythropoietin, protected nerve cells and promoted expression of Bcl-x_{L} (Wen, T.-C. et al., J. Exp. Med., 188, 635-649, 1998), similarly IL-3 can be estimated to promote regeneration and/or reconstruction of tissues at -the low molar concentrations. As described hereinabove, non-peptide factors showing neuroprotective action (e.g. ginsenoside Rb₁, isocarbacyclines, prostaglandins and. isocarbacycline derivatives) or cytokines and growth factors (e.g. erythropoietin, interleukin 3 and prosaposin-related peptides) can be estimated to promote regeneration and/or reconstruction of tissues at the low molar concentrations. In other words, compounds exhibiting brain cell or nerve cell-protective action in animal models with cerebral ischemia by intracerebroventricular infusion (e.g. ginsenosides, isocarbacyclines, trophic factors, prostaglandins, steroids, growth factors, proliferation factors, prosaposin-related peptides, cytokines, chemokines, peptide factors, non-peptide compounds or metabolites thereof) are, as described hereinbefore, thought to exhibit tissue regeneration and reconstruction-promoting action at the low molar concentrations. With regard to concrete compounds or physiologically active compounds exhibiting brain cell or nerve cell-protective action by intracerebroventricular administration, basic fibroblast growth factor (bFGF), ciliary neurotrophic factor (CNTF), brain-derived neurotrophic factor (BDNF), interleukin 6, one of isocarbacyclines TEI-7165, estradiol, glutamate antagonists, epidermal growth factor (EGF), platelet derived growth factor (PDGF), glutamate receptor antagonists, 15R-isocarbacycline derivatives, prostaglandins, 15-deoxy-isocarbacycline derivatives, etc. can be mentioned, but the present invention is not limited within these examples. Details of isocarbacyclines, prostaglandins and 15-deoxy-isocarbacycline derivatives hereinbefore are described clearly in JP-A-11-222436, ibid. 11-228418, ibid. 11-228417, ibid. 59-210044, ibid. 4-26654, ibid. 61-197518, ibid. 2-167227, ibid. 8-20540, ibid. 8-20541 and ibid. 8-40909. A report concerning the protective action of TEI-7165 on ischemic brain is opened to public by one of the present inventors (Sakanaka) (Matsuda, S. et al., Brain Res., 769, 321-328, 1997). Namely, all compounds hereinabove can be utilized for prevention, therapy or treatment of organic diseases causing histopathological changes through tissue regeneration and reconstruction-promoting action, when the extracellular fluid concentrations in lesioned tissue are 1 ng/ml or less or 0.5 nM or less, preferably 10 pg/ml or less or 5 pM or less, more preferably 0.01 - 100 fg/ml or 0.005 - 50 fM or 1 - 10000 fg/ml or 0.5 - 5000 fM.

Of course, all compounds hereinbefore have the same effects, efficacy and usages as those of ginsenosides (including ginsenoside Rb₁) and dihydroginsenoside Rb₁. Namely, the compounds hereinbefore or metabolites thereof or salts thereof are, in the same manner as of ginsenoside Rb₁, useful as a composition(s), a pharmaceutical composition(s), a health drug composition(s), a cosmetic composition(s), fertilizer additives, feed additives or a veterinary drug composition(s) for prevention, treatment, improvement or therapy of diseases, pathological states, symptoms or conditions of vital tissues causing or delaying regeneration, generation, rooting, budding, growth, differentiation or reconstruction. Diseases, pathological states, symptoms or conditions expecting application of such compounds hereinbefore can be mentioned, for example, as all diseases and pathological states causing cell death as described in PCT/JP00/04102, senile symptoms of skin and mucosa as described in the present specification, conditions of rooting, budding and growth of plant such as cutting of pothos under hydroponics, or condition of growth of fish and shellfish under cultivation. Especially, when the compound(s) hereinbefore (phharmaceutical composition(s)) is used as an agent(s) for external use on skin or topical application to skin for prevention, therapy or treatment of skin diseases such as wound, burn, bedsore, decubitus, skin ulcer, etc., since the extracellular fluid concentrations of the said compound(s) in the lesioned tissue can be relatively easily controlled, excellent effect can be obtained by applying or spraying externally or topically the compound(s) of low concentration (0.001% by weight or less or under, preferably 0.00001% by weight or less, more preferably 0.0000001% by weight or less, more further preferably 0.000000001% by weight) admixed with an agent(s) for external use on skin to the lesioned tissue.

The present invention will be explained further in the following.

As explained hereinabove, the present invention is fundamentally to find out that ginsenosides, metabolites thereof or salts thereof, preferably when used at low concentrations and low dosages, have superior action for prevention, treatment or therapy of organic diseases causing histopathological changes of vital tissue, living tissue or viable tissue. Ginsenosides, metabolites thereof or salts thereof of the present invention, preferably in use of low concentrations and low dosages thereof, can not only cure organic diseases causing histopathological changes of vital tissue, living tissue or viable tissue but also regenerate or reconstruct vital tissue, living tissue or viable tissue having histopathological changes; for example in case of skin injury as exemplified concretely hereinbefore, ginsenosides, metabolites thereof or salts thereof of the present invention, preferably in use of low concentration and low dosage thereof, can not only protect simply the tissue inside the body for recovery by intercepting injured region from the outside of the intact vital tissue but also regenerate each tissue in the injured region and reconstruct each regenerated tissue to the original condition as well as regenerating physiological state before injury. Further, it is the specific feature of the present invention to find out that use of ginsenosides, metabolites thereof or salts thereof of the present invention, preferably in low concentration and low dosage promotes the cure of organic diseases causing histopathological changes of vital tissue, living tissue, or viable tissue.

Another feature of the present invention is to find out a novel action(s) completely different from conventionally known actions of ginsenosides by using ginsenosides, metabolites thereof or salts thereof at low concentrations, low doses or low dosages, preferably at extremely low concentrations, low doses or low dosages.

Further another feature of the present invention is to administer parenterally such as intravenously, extramucosally or extracutaneously, ginsenosides, metabolites thereof or salts thereof at low concentrations, low doses or low dosages, preferably at extremely low concentrations, low doses or low dosages.

Consequently, an object of the present invention is to provide a novel pharmaceutical composition(s) or a novel veterinary drug composition(s) for prevention, treatment or therapy of organic diseases causing histopathological changes of the vital tissues, living tissues or viable tissues comprising ginsenosides such as ginsenoside Rb₁, metabolites thereof or salts thereof, preferably at low concentrations, low doses and/or low dosage.

More particularly, an object of the present invention is to provide the novel pharmaceutical composition(s) or the novel veterinary drug composition(s), which can treat or cure diseases having histopathological changes caused by injury or defect etc.of the vital, living or viable tissues through regeneration and/or reconstruction of said pathologically and histologically changed tissues, or which can prevent the vital, living or viable tissues likely to cause such changes and promote cure of these diseases.

Further, an object of the present invention is to provide a method(s) for prevention, treatment or therapy of diseases comprising administering an effective amount(s) of the pharmaceutical composition(s) or veterinary drug composition(s) of the present invention described hereinbefore to patients with organic diseases causing histopathological changes of the vital, living or viable tissues.

More particularly, an object of the present invention is to provide the novel method(s) for prevention, the novel method(s) for treatment or the novel method(s) for therapy of diseases comprising administering an effective amount(s) of the pharmaceutical composition(s) or veterinary drug composition(s) of the present invention described hereinbefore to patients with organic diseases having histopathological changes caused by injury or deficit etc.of the vital,living or viable tissues. This novel method(s) is useful for treating or curing diseases with histopathological changes caused by injury or defect etc. of the vital, living or viable tissues as a result of regenerating and/or reconstructing said pathologically and histologically changed tissues, for preventing diseases of the vital,living or viable tissues likely to cause such changes and for promoting cure of these diseases.

Further object of the present invention is to provide use of ginsenosides such as ginsenoside Rb₁, metabolites thereof or salts thereof for producing a pharmaceutical composition(s) or a veterinary drug composition(s) of the present invention.

Organic diseases in the present invention can be any condition wherein the normal condition of vital, living or viable tissue is destroyed. Examples of diseases of skin tissue are wound of skin tissue, burn, radiation injury, pernio, chilblain, frostbite, ultraviolet injury, electric injury, traumatic injury, skin ulcer, bedsore, decubitus, various postsurgical wounds, trauma, contact dermatitis, bullous dermatitis, atopic dermatitis, stagnation dermatitis, xeroderma, diabetic skin ulcer, autosensitization dermatitis, erythroderma, exfoliative dermatitis, epidermal hydroa, epidermolysis bullosa, photosensitivity, progressive pigmented purpuric dermatosis (Schamberg disease), strophulus, pollinosis, insect bite, prurigo, erythema multiforme, erythema annulare, erythema nodosum, pemphigus, bullous pemphigoid, herpetic dermatitis, dermatitis herpetiformis, palmoplantar pustulosis, psoriasis, lichen planus, ichthyosis, lichen pilaris, xanthomatosis, cutaneous amyloidosis, herpes simplex, viral wart, molluscum pendulum, mollusum contagiosum, pyoderma, skin tuberculosis, atypical mycobacteriosis of the skin, trichophytia, tinea, oral or cutaneous candidiasis, scabies, pediculosis, syphilis, keloid, hypertrophic scar, hemangioma, angioma, lymphoma, nevus, vitiligovulgaris, ephelides, moth patches, chloasma, melanosis, pompholyx, miliaria, acne vulgaris, rosacea, rosacea-like dermatitis, oral mucosa injury, stomatitis, perioral dermatitis, senile symptoms of skin (e.g. atrophy, dermatrophia, vulnerability to infection, slackening, flabbiness, dandruff, scurf, alopecia, depilation, gray hair, poliosis, itching, roughness, dry skin, oligosteatosis, asteatosis, exfoliation of hornified cells, cornified cells, keratinocytes, keratinized cells or keratic cells, exfoliation of the stratum corneum, chapped, rhagade, crack, ephelis, spots, blotches, wrinkles, lines, furrows, freckle, aplasia, poor regeneration, pigmentation, dryness, etc.), depilation, alopecia, perionychia, ingrown nail, etc.

The skin tissues or the skin cells showing promotion of regeneration or reconstruction by the composition(s) of the present invention include epidermal cells, dermis, papillae of the dermis, dermal papillae, subcutaneous tissue, connective tissue, hair papillae, hair follicles, pilomotor muscles, sebaceous glands, sweat glands, peripheral nerves, blood vessels, epidermal keratinocytes, cornified cells, keratinized cells, keratic cells, hornified cells, Langerhans cells, Merkel cells, melanocytes, stem cells, mesenchymal cells, hair follicular cells, pilomotor muscular cells, sweat gland cells, sebaceous gland cells, fibroblasts, etc.

Examples of mucosal tissue diseases are all diseases and pathological state(s) causing histopathological changes of mucosal tissues such as diseases caused by injury, morsus, wound, burn, traumatic injury, trauma or defect of mucosal tissues including the oral mucosa. For example, caries, pulpitis, marginal periodontitis, periodontal diseases, stomatitis, glossitis, recurrent aphtha, intraoral aphtha, halitosis, mouth odor, oral dysethesia, oral abnormal sensation, odontogenic infection, oral mucosa morsus, lingual morsus, oral mucosa burn, lingual burn, lingual injury, oral mucosa injury, gingivitis, alveolar pyorrhea, catarrhal stomatitis, gangrenous stomatitis, Vincent stomatitis, aphthous stomatitis, acute herpetic gingival stomatitis, herpangina, herpes zoster, oral mucosal erosion, oral mucosal ulcer, decubitus ulcer, radiation stomatitis, pemphigus, oral candidiasis, lichen planus, Riga-Fede disease, bald tongue, red plain tongue, mucosal ulcer, mucosal erosion, cataract, conjunctival erosion, corneal erosion, enteric mucosal erosion, Sjögren's syndrome, senile symptoms of mucosa, especially oral mucosa (e.g. atrophy, shrinkage, mucosal exfoliation or ablation, chapped, crack, epithelial exfoliation or ablation, aplasia, poor regeneration and driness) can be mentioned. In broad meanings, burn, decubitus, skin ulcer, frostbite, chilblain, pernio, electric injury, etc. can be included in wound. Proviso that, drug preparations or pharmaceutical compositions suitable for therapy or treatment of these diseases are different in each other. For example, in decubitus or corneal injury caused by contact lens, since epithelial cells of skin or cornea gradually enter apoptosis-like cell death or apoptosis, so called cytoprotective agent or anti-apoptotic agent is expected to exhibit effectiveness and efficacy for these diseases. However, if cells constituting vital, living or viable tissues are immediately exfoliated due to wound (e.g. incisional wound, open wound, burn, morsus, etc.), principally tissue regeneration and reconstruction promoter should be used rather than cytoprotective agent. In case that apoptosis-like cell death or apoptosis occurs in wound penumbra, cytoprotective agent or anti-apoptotic agent may be useful for prevention of expansion and deterioration of wound lesion.

Examples of organic diseases in the present invention are not limited within the above described diseases of skin tissue and mucosal tissues, and organic diseases causing histopathological changes of other all tissues, for example central nervous tissue, liver, kidneys, spleen, hematopoietic tissue, digestive tract, lung, pancreas, cornea, endocrine glands, exocrine glands, salivary glands, gonads, urinary bladder, etc. are included. Examples of these diseases are, for example, injury or disorder after hepatitis, diseases of hepatic tissue after hepatectomy and hepatic ischemia and reperfusion, central nervous tissue diseases after nerve injury or neurotrauma (head injury and spinal cord injury), diseases after amputation of finger, nephritis, diseases of kidney tissue after acute tubular necrosis, diseases of spleen, diseases of pancreas, organ diseases after pneumonectomy, regeneration and implantation of bone marrow graft, cataract, corneal injury, corneal erosion, corneal ulcer, injury of nail, peptic ulcer, surgical operations (thoracic or abdominal surgical operation, orthopedic surgery, plastic surgery, vanity surgery, gynecotocological surgery, urological surgery, ophthalmological surgery, head and neck surgery, dental oral surgery, otorhinolaryngological surgery, veterinary surgery, neurological surgery, etc.).

Further, in the open wound (defect) of skin, although peripheral nerves and blood vessels distributed in the defected skin region are destroyed and incised, these peripheral nerves and blood vessels can obviously be regenerated, reconstructed, recovered and restored rapidly by intravenous administration or extracutaneous administration of ginsenoside Rb₁ to the original healthy condition. Consequently, ginsenosides, especially ginsenoside Rb₁, can be utilized as a pharmaceutical composition(s) for promoting regeneration and/or reconstruction of nervous tissues and vascular tissues. Namely, in any one of pathological states of diabetic neuropathy, intervertebral disk hernia, spinal canal stenosis, spondylolysis, spondylolisthesis, spondylopathy, cervical spondylotic myelopathy, myelopathic radiculopathy, ossification of the posterior longitudinal ligament, spinal cord injury, peripheral neuropathy, compression neuropathy, head injury, neurotrauma, nerve injury, neuralgia, neurodegenerative diseases, peripheral nerve paralysis and cerebral apoplexy, intravenous administration, local or topical administration, nasal administration, rectal administration, etc. can exhibit effectiveness and efficacy by promoting regeneration and/or reconstruction of once injured nervous tissues. On the other hand, ginsenosides, especially ginsenoside Rb₁, can exhibit effectiveness and efficacy, through regeneration and/or reconstruction of blood vessels, for diseases with a main symptom(s) of blood flow failure or disorder (aortitis syndrome, collagen diseases, peripheral embolism, thromboangitis obliterans, arteriosclerosis obliterans, thrombophlebitis, diabetic skin ulcer, diabetic retinopathy, diabetic nephropathy, retinal embolism, Raynaud's disease, Raynaud's syndrome, myocardial infarct, decubitus, bedsore, hemorrhoids, pile, periproctitis, osteonecrosis, epiphysiopathy, peripheral circulation failure, angina pectoris, ischemia reperfusion injuries of liver, kidney and heart, cerebrovascular diseases, bone atrophy, malunited fracture of bone, delayed cure fracture of bone, etc.). Effects, efficacy and usages of ginsenosides, especially ginsenoside Rb₁, described hereinabove can be applied to a crude saponin fraction(s) of ginseng, a ginseng extract(s) or ginseng.

Organic diseases in the present invention include, in addition to the above-described diseases and traumatic injuries, diseases and pathological states causing histopathological changes of all organs and tissues. More concretely, all diseases and pathological states described in the books ("Today's therapy", Ed. Hinohara, Shigeaki and Abe, Masakazu, Igaku Shoin Publ., 1995; "Today's therapy", Ed. Tagasu, Yukio and Ogata, Etsuro, Igaku Shoin Publ., 2000; or "Today's therapy in infants", Ed. Yata, Junichi, et al. Igaku Shoin Publ., 2000) are included. Of course, even if organic diseases with unknown origin newly appear in future, and if such diseases are causing histopathological changes of vital, living or viable tissues, these diseases are included within the scope of organic diseases in the present invention.

Embodiments of the pharmaceutical composition(s) and veterinary drug composition(s) of the present invention will be explained further in detail as follows. The present invention relates to quite naturally the veterinary drug composition(s), but in the description hereinbelow, only term for pharmaceutical composition is used and a term for veterinary drug composition is not mentioned in sometimes.

The pharmaceutical composition(s) or veterinary drug composition(s) of the present invention is a composition(s) for prevention, treatment or therapy of organic diseases causing histopathological changes of skin tissue with action for promoting regeneration and/or reconstruction or action for promoting wound healing of epidermis, dermis, papilla of the dermis, subcutaneous tissue, connective tissues, hair papillae, hair follicles, sebaceous glands, pilomotor muscles, sweat glands, peripheral nerves and blood vessels, and an object of the present invention is to provide a method for prevention, treatment or therapy comprising using the said composition(s), and use of ginsenosides, metabolites thereof or salts thereof for production of the same.

The pharmaceutical composition(s) or veterinary drug composition(s) of the present invention is a composition(s) for promoting regeneration and/or reconstruction of skin tissue, and an object of the present invention is to provide a method for prevention, treatment or therapy comprising using the said composition(s), and use of ginsenosides, metabolites thereof or salts thereof for production of the same.

The pharmaceutical composition(s) or veterinary drug composition(s) of the present invention is a composition(s) for prevention, treatment or therapy of diseases caused by injury, morsus, wound, burn, traumatic injury, trauma or defect of mucosal tissues and all diseases causing histopathological changes of mucosal tissues such as oral mucosa with action for promoting regeneration and/or reconstruction of injured region and action for promoting cure, and an object of the present invention is to provide a method for prevention, treatment or therapy comprising using the said composition(s), and use of ginsenosides, metabolites thereof or salts thereof for production of the same.

The pharmaceutical composition(s) or veterinary drug composition(s) of the present invention is a composition(s) for promoting regeneration and/or reconstruction of cells or tissues of vital, living or viable tissues having histopathological changes, and an object of the present invention is to provide a method for prevention, treatment or therapy comprising using the said composition(s), and use of ginsenosides, metabolites thereof or salts thereof for production of the same.

The pharmaceutical composition(s) or veterinary drug composition(s) of the present invention is a composition(s) for promoting cure of organic diseases by regeneration and/or reconstruction of cells or tissues of vital, living or viable tissues having histopathological changes, and an object of the present invention is to provide a method for prevention, treatment or therapy comprising using the said composition(s) and use of ginsenosides, metabolites thereof or salts thereof for production of the same.

The pharmaceutical composition(s) or veterinary drug composition(s) of the present invention is a pharmaceutical composition(s), veterinary drug composition(s), a composition(s) for external use of skin or topical application to skin or a composition(s) for external use of mucosa or topical application to mucosa for preventing, improving or treating senile symptoms of skin tissue or mucosal tissues, and an object of the present invention is to provide a method for prevention, treatment or therapy comprising using the said composition(s), and use of ginsenosides, metabolites thereof or salts thereof for production of the same.

The pharmaceutical composition(s) or veterinary drug composition(s) of the present invention is a composition(s) for prevention, treatment or therapy of diseases of mucosal tissues, especially mouth mucosal tissue, by promoting regeneration and/or reconstruction of epithelium, lamina propria, salivary glands, mucous glands, mixed glands, connective tissues, muscular tissues, blood vessels and peripheral nerves, or by promoting wound healing, and an object of the present invention is to provide a method for prevention, treatment or therapy comprising using the said composition(s), and use of ginsenosides, metabolites thereof or salts thereof for production of the same.

Further, the pharmaceutical composition(s) or veterinary drug composition(s) of the present invention is a composition(s) for promoting regeneration and/or reconstruction of epithelial cells, gland cells, myoepithelial cells, fibroblasts, stem cells, mesenchymal cells, vascular endothelial cells, smooth muscle cells, extracellular matrix (matrices), collagen fibers, elastic fibers and reticular fibers, and an object of the present invention is to provide a method for prevention, treatment or therapy comprising using the said composition(s), and use of ginsenosides, metabolites thereof or salts thereof for production of the same.

Examples of effective components in the pharmaceutical composition(s) or the veterinary drug composition(s) of the present invention, ginsenosides, metabolites thereof or salts thereof, are ginsenoside hereinbefore described, natural product containing ginsenoside or extract thereof, and ginsenoside derivatives as produced by chemical modification.

Preferable example of ginsenoside is natural ginsenoside Rb₁.

Ginsenoside Rb₁ is represented by the chemical formula hereinbefore, and ginsenoside Rb₁ can be isolated and purified according to the method by Shibata, et al. (Shibata, S. et al., Economic and medicinal plant research, World Scientific, Philadelphia, pp 217-284, 1985). The purity of product isolated by such methods is confirmed to be more than 98% as determined by thin layered chromatography and NMR spectrum (Kawashima, Y. and Samukawa, K., J. Med. Pharmacol. Soc. Wakan-Yaku, 3, 235-236, 1986).

Other ginsenosides can be obtained by known methods.

Ginsenosides of the present invention can be a natural substance or extract thereof. Examples of the natural substance or extract thereof are any natural plant(s) containing ginsenosides, for example Panax ginseng, American ginseng, Sanshichi ginseng, Chikusetsu ginseng, Himalayan ginseng, Denshichi ginseng. These natural substances can be used directly, or they can be used in the form of extract, extracted preparation, liquid preparation, tablet, fraction or purified product prepared by extraction, concentration, purification or formulation. For example, crude saponin fraction of ginseng can be obtained by a conventional method that red ginseng is extracted with methanol, suspending the extract in water, washing with ether, and shaking with water saturated butanol. Yield is about 8%. In addition, yield of ginseng extract is about 35 - 45%. Ginsenoside Rb₁ and the crude saponin fraction used in examples hereinbelow are same as used in the prior paper by the inventor (Sakanaka) (lyophilized crystalline product) (Wen, T.-C. et al., Acta Neuropathol., 91, 15-22, 1996). With regard to natural product used for extraction of crude saponin fraction, not only ginseng (Panax ginseng) but also any of Chikusetsu ginseng, Tochiba ginseng, Himalayan ginseng, Sanshichi ginseng, American ginseng and Denshichi ginseng can be used.

Ginsenosides, which are effective components of the composition(s) of the present invention, can be ginsenoside(s) derivatives prepared by the method of chemical modification of natural ginsenoside hereinbefore. Examples of such ginsenoside(s) derivatives are dihydroginsenoside Rb₁ hereinbelow described and others. Dihydroginsenoside Rb₁ can be produced by reduction of natural ginsenoside Rb₁.

Ginsenosides of the present invention can be used in free form, but can be used in the form of suitable salt. Solvate(s) such as hydrate(s) thereof can also be used.

Further, a part(s) of chemical structure of these ginsenosides is modified to prepare prodrug and any route of administration and any method of administration can also be selected. For example, a prodrug is prepared by esterification of a hydroxyl(s) or a hydroxyl group(s) of ginsenoside Rb₁, administered and hydrolyzed by endogenous esterase to work ginsenoside Rb₁ in the vital, living or viable tissues.

The pharmaceutical composition(s) or veterinary drug composition(s) of the present invention is preferably comprising low concentrations of ginsenosides, such as ginsenoside Rb₁, metabolites thereof or salts thereof.

According to results of the present experiments, the amount(s) of intravenous administration for use of ginsenoside Rb₁ as skin tissue regeneration and reconstruction promoter is similar to that of intravenous administration for using said compound as brain cell or nerve cell protective agent (Japanese Patent Appln. No. Hei 10-365560, PCT/JP99/02550, Brain cell or nerve cell-protective agents comprising ginsenoside Rb₁). Judging from this fact, the concentration(s) of ginsenosides, especially ginsenoside Rb₁, which acts as skin tissue or mucosal tissue regeneration and reconstruction promoter, is preferably low as described in Japanese Patent Appln. No. Hei 10-365560, PCT/JP99/02550 (Brain cell or nerve cell-protective agents comprising ginsenoside Rb₁), and more concretely, the extracellular fluid concentrations of ginsenosides, especially ginsenoside Rb₁, in lesioned regions are 1 ng/ml or less, preferably 10 pg/ml or less, more preferably 100 fg/ml or less. In case that ginsenosides, especially ginsenoside Rb₁, of the present invention is used as a preparation(s) for intravenous administration or a preparation(s) for external use on skin or topical application to skin, the preparation(s) comprising ginsenosides, especially ginsenoside Rb₁, is preferably adjusted so that the extracellular fluid concentrations of said compounds in lesioned tissues of patients are in the range of the above levels. Sufficient effect can be achieved by the pharmaceutical composition(s) and preparation(s) of the present invention even when the extracellular fluid concentrations in lesioned tissues are about 1 - 100 fg/ml or less (e.g. 0.01 fg/ml). Namely, ginsenosides, especially ginsenoside Rb₁, are thought to exhibit the superior effectiveness and efficacy, when the extracellular fluid concentrations in lesioned tissues are 0.01 - 100 fg/ml or 1 - 10000 fg/ml. Further, when ginsenosides, especially ginsenoside Rb₁, are applied in order to achieve prevention, treatment and/or improvement of senile symptoms of skin and mucosa by using them as the composition(s) of cosmetics or health-promoting drugs, the amounts of ginsenosides, especially ginsenoside Rb₁, admixed into cosmetics or health-promoting drugs should be adjusted so that the extracellular fluid concentrations of ginsenosides, especially ginsenoside Rb₁, in the local region of skin or mucosa are kept low as described hereinbefore.

As described hereinbelow, although ginsenosides can be admixed to cosmetics together with other cosmetic composition(s) described in U.S. Patent 5,663,160 or WO 99/07338, the composition(s) of the present invention has specific feature to use at lower concentrations than the concentrations as described in U.S. Patent 5,663,160 or WO 99/07338. Ginsenosides, especially ginsenoside Rb₁, can be admixed in every cosmetics or health-promoting drugs together with any other cosmetic composition(s) or health-promoting drug composition(s), and the concentrations of the other cosmetic composition(s) or health-promoting drug composition(s) used with ginsenosides, especially ginsenoside Rb₁, should preferably be lower than the concentration(s) as described in the prior references and patent specifications.

As obvious from examples described hereinbefore, when ginsenoside Rb₁ was intravenously administered to rats (body weight about 300 g) with incised wound, open wound or defect of skin, in a daily dose of 12 - 60 µg, superior therapeutic effect, which has never known in the history, could be obtained through skin tissue regeneration and reconstruction-promoting action and wound healing-promoting action. Further, as the results of applying the agent for external use on skin comprising or containing ginsenoside Rb₁ at a concentration of 0.0001% by weight, 0.00001% by weight, 0.000001% by weight, 0.0000001% by weight or 0.00000001% by weight, to open wound of rats, wound healing and skin tissue regeneration and reconstruction were obviously promoted and area of open wound region was reduced to about 1/2 - 1/4 of the control or vehicle (carrier) group. The agent for external use on mucosa comprising or containing 0.00001% by weight of ginsenoside Rb₁ also exhibited excellent effect on morsus of human mouth mucosa. However, since therapeutic effect on open wound in rats of 0.01% by weight of ginsenoside Rb₁ was trivial, ginsenosides, especially ginsenoside Rb₁, should preferably be admixed at concentrations of 0.001% by weight or less or at concentrations lower than 0.001% by weight in the agent(s) for external use on skin or topical application to skin or the agent(s) for external use on mucosa or topical application to mucosa.

These experimental results indicate that ginsenosides of the present invention are effective when used at low concentrations.

Based on the experimental results hereinbefore, an intravenous optimum dose of drug (ginsenosides, especially ginsenoside Rb₁) in patient or vertebrate (estimated body weight 60 kg) suffering from disease caused by injury, wound, traumatic injury or defect of skin tissue or mucosal tissue or from disease causing histopathological changes of skin or mucosa is calculated to be from 2.4 mg to 12 mg a day. Consequently, in case of using the pharmaceutical composition(s) of the present invention for prevention, therapy or treatment of human skin diseases or mucosal diseases, a systemic dose per day is, though depending on individual difference or diseases of patients, 0.01 mg or more, preferably 0.1 mg or more, more preferably 10 mg or more. However, since, generally, necessary amount of drug for administration per kg body weight is decreased depending on increase in body weight of animals, there is possibility that ginsenosides, especially ginsenoside Rb₁, exhibit sufficient effectiveness and efficacy in an amount of 1/10 or less of that dose in human. Although the pharmaceutical composition(s) of the present invention has less side effect and the pharmaceutical composition(s) can be set to considerably large amount as an upper limit of administration for prevention, treatment or therapy of the skin diseases hereinbefore described, it is 1 g/day or less, preferably 0.1 g/day or less. Amount of ginsenosides, especially ginsenoside Rb₁, in 10 g of an agent for external use on skin or topical application to skin or an agent for external use on mucosa or topical application to mucosa can be 0.1 mg or less, preferably 0.001 mg or less, more preferably 0.0001 mg or less. Namely, amount of ginsenosides, especially ginsenoside Rb₁, for external or topical administration to skin or mucosa per day for human patients with skin diseases or mucosal diseases is thought to be, though depending on individual difference or disease state of patients, usually 0.1 mg or less, preferably 0.001 mg or less, more preferably 0.0001 mg or less.

In case that natural product or extract(s) thereof as ginsenosides of the present invention is used, generally, since the optimum extracellular fluid concentrations of crude saponin fraction of ginseng in lesioned tissue is thought to be 14.5-fold of the optimum extracellular fluid concentrations of ginsenoside Rb₁, the amount of 14.5-fold may be used.

The pharmaceutical composition(s) or veterinary drug composition(s) of the present invention is preferably in the form of parenteral administration such as intravenous administration, mucosal administration or extracutaneous administration. More particularly, the pharmaceutical composition(s) or veterinary drug composition(s) of the present invention is preferably used as a preparation(s) for parenteral administration, a preparation(s) for external or topical application to mucosa, a preparation(s) for external or topical spray on mucosa, a preparation(s) for external or topical application to skin or as a preparation(s) for external or topical spray onto skin comprising containing ginsenosides, especially ginsenoside Rb₁, metabolites thereof or salts thereof at low concentrations.

Consequently, the present invention provides a preparation(s) for parenteral administration, preferably a preparation(s) for intravenous or intravascular administration, an agent(s) for external use on mucosa or topical application to mucosa or an agent(s) for external use on skin or topical application to skin, for prevention, treatment or therapy of diseases hereinbefore described comprising containing ginsenosides, especially ginsenoside Rb₁, metabolites thereof or salts thereof, preferably at low concentrations.

The pharmaceutical composition(s) or veterinary drug composition(s) of the present invention is preferably used as a preparation(s) for intravenous administration, a preparation(s) for external use on mucosa or topical application to mucosa, a preparation(s) for external use on skin or topical application to skin, a preparation(s) for external application to skin or as a preparation(s) for external or topical spray onto skin; moreover, preparations for any route of administration such as an agent(s) for external application or use on local lesion, an injection(s) for local lesion, an oral preparation(s), nasal drops, ear drops, eye drops, eye ointment, suppository, subcutaneous injection, intracutaneous injection, intramuscular injection, inhalation, sublingual tablets, artificial salivary, intraarticular administration, percutaneous absorption, etc.can be selected. Sustained release preparation can also be used.

Further, the present invention provides an agent(s) for long term therapy, prevention or treatment of skin diseases or mucosal diseases, a promoter(s) for regeneration and/or reconstruction of skin tissue or mucosa, a wound healing-promoter(s) or cosmetics or health-promoting drugs for inhibition of senile symptoms of skin or mucosa comprising the preparation(s) for intravenous administration, the preparation(s) for external or topical application to mucosa, a preparation for external application to skin, health-promoting drugs or cosmetics, as described above.

The preparation(s) for intravenous administration of the present invention can be directly applicable intravascularly, preferably intravenously, and is used as a preparation(s) for single intravenous infusion or a preparation(s) for continuous intravenous infusion after dissolving ginsenosides, especially ginsenoside Rb₁, in physiological saline, distilled water, phosphate buffer, glucose solution, liposome or fat emulsion. A formulation used by adding to a preparation(s) for intravenous administration, such as a composition for drip infusion, is also preferable.

The preparation(s) for external use on skin or topical application to skin or the preparation(s) for external use on mucosa or topical application to mucosa for prevention, treatment or therapy of organic diseases causing histopathological changes comprising ginsenosides, especially ginsenoside Rb₁, of the present invention can be prepared by admixing ginsenosides, especially ginsenoside Rb₁, preferably at low concentrations, into any base such as water soluble base, emulsion base, combination drug (base) or fat-soluble base (ointment base). Further, as like aphtouch, low concentration(s) of ginsenosides, especially ginsenoside Rb₁, can be admixed in a preparation, which adheres on mucosa. Concretely, after ginsenosides, especially ginsenoside Rb₁, are admixed in the concentration of 100 mg (0.1% by weight) or less or lower, preferably 10 mg (0.01% by weight) or less or lower, more preferably 1 mg (0.001% by weight) or less or lower, and 1 fg (10⁻¹⁵% by weight) or more, per 100 g of water soluble base (cream, etc.), emulsion base, combination drug (base)or ointment base (fat-soluble base) such as ophthalmic white petrolatum (propel), the resulted preparation(s) can be used as an agent(s) for external use on skin or topical application to skin or an agent(s) for external use on mucosa or topical application to mucosa for prevention, treatment or therapy of the above-described diseases.

Of course, in the above-described agent(s) for external or topical application to skin or agent(s) for external or topical application to mucosa, in addition to ginsenosides, especially ginsenoside Rb₁, any pharmaceutical composition (e.g. glucose, antibiotics, vitamin E, vitamin E derivatives, vitamin D, vitamin D derivatives, vitamins, antiviral agents, immunosuppressive agent, antiallergic agents, steroids, ginseng components, components of natural substance, etc.) can be admixed. When ginsenoside Rb₁ and other pharmaceutical composition(s) or composition(s) for external or topical application to skin and/or mucosa are used in combination, the upper limit of concentration of ginsenosides Rb₁ is preferably set at levels less than 0.00002% by weight rather than at levels less than 0.001% by weight. Particularly for allergic cutaneous mucosal diseases such as atopic dermatitis, contact dermatitis or pollinosis, if steroid (preparation), antiallergic pharmaceutical composition (preparation) or immunosuppressive pharmaceutical composition (preparation) at concentrations less than those ever used or ever described in previous patent specifications is admixed into the agent(s) for external or topical application to skin or the agent(s) for external or topical application to mucosa, comprising ginsenosides, especially ginsenoside Rb₁, excellent effect can be obtained. The agent(s) for external or topical application to skin or the agent(s) for external or topical application to mucosa comprising or containing ginsenosides, especially ginsenoside Rb₁, and the agent(s) for external or topical application to skin or the agent for external or topical application to mucosa comprising or containing any other pharmaceutical composition can be used in combination. The upper limit of concentration of ginsenosides, especially ginsenoside Rb₁, in the agent(s) for external or topical application to skin or the agent(s) for external or topical application to mucosa for prevention, treatment or therapy of the above-described diseases is 10% by weight or less, preferably 1% by weight or less.

Ginsenosides, especially ginsenoside Rb₁, of the present invention can expedite wound healing as a result of promoting regeneration and/or reconstruction of deciduous skin tissue by continuous intravenous administration for about one week or by external or topical application or spray once or consecutively in every day after development of incised wound, open wound or defect of the skin. Ginsenoside Rb₁ of the present invention can expedite wound healing as a result of promoting regeneration and/or reconstruction of deciduous skin tissue by external or topical application onto the wound region 1 - 10 times a day for about 5 days after development of morsus of the mouth mucosa.

A method for administration of the pharmaceutical composition(s) or veterinary drug composition(s) of the present invention is preferably intravascular administration, especially intravenous administration, with consecutive or continuous administration of the above described dosages or doses. Ginsenosides, especially ginsenoside Rb₁, the active ingredient(s) of the present invention, are one of saponins and are formulated by conventional methods. For example, the water soluble pharmaceutical composition(s) of the present invention can be intravenously administered by dissolving lyophilized crystals in physiological saline, distilled water, phosphate buffer or glucose solution. Of course, as described hereinbefore, after dissolving, the pharmaceutical composition(s) of the present invention can be used by adding to the preparation for intravenous administration such as composition for drip infusion. Further, it can also be used as fat emulsion, liposome preparation or sustained release preparation. The concentrations of ginsenosides, especially ginsenoside Rb₁, in the preparation(s) for intravenous administration can be optionally adjusted unless so high, for example 0.001 - 100 mg/ml, preferably 0.01 - 10 mg/ml, more preferably 0.1 - 1 mg/ml. When ginsenosides, especially ginsenoside Rb₁, is utilized as the preparation(s) for external or topical application to skin or the preparation(s) for external or topical application to mucosa for prevention, treatment or therapy of the above described diseases, as described hereinbefore, ginsenosides, especially ginsenoside Rb₁, is admixed at the concentrations of 0.1% by weight or less, preferably 0.01% by weight or less, more preferably 0.001% by weight or less or at concentrations less than 0.001% by weight, and at the concentrations of 10⁻¹⁵% by weight or more in any base such as water soluble base, emulsion base, ointment base, combination drug (base) or fat soluble base. Proviso that, when the preparation(s) for external or topical application to skin comprising or containing ginsenosides, especially ginsenoside Rb₁, is administered for long term or administered to patients who suffered from mild skin wound, the concentration thereof can be reduced to 10⁻²⁰% by weight. The upper limit of concentration of ginsenosides, especially ginsenoside Rb₁, in the agent(s) for external or topical application to skin or the agent(s) for external or topical application to mucosa is 10% by weight or less, preferably 1% by weight or less. The agent(s) for external use on skin or topical application to skin comprising ginsenosides, especially ginsenoside Rb₁, can be in the form of applying to local region of skin or in the form of spray.

Further, a specific feature of ginsenosides, especially ginsenoside Rb₁, of the present invention, which can not be overlooked, is that they do not exhibit significant adverse effect. Actually, since LD50 of intraperitoneally administered ginsenoside Rb₁ is reported to be 1110 mg/kg (Kaku, T. et al., Arzneim. Forsch. Drug Res., 25, 539-547, 1975), ginsenoside Rb₁ is thought to be extremely a safe pharmaceutical composition, as far as directing therapy or treatment of incised wound, open wound and/or defect of skin tissue by continuous intravenous administration of 12 - 60 µg/day of ginsenoside Rb₁ to rat (body weight about 300 g) as shown in examples hereinbelow. The amount of administration of ginsenoside Rb₁, which is used as the preparation(s) for external or topical application to skin or the preparation(s) for external or topical application to mucosa, is far smaller than the amount of intravenous administration. Of course, in the present experiments, as far as animals administered with ginsenoside Rb₁ were carefully observed, no side effect or ill effect was noted.

Since the pharmaceutical composition(s) of the present invention is effective for prevention, improvement, treatment and/or therapy of organic diseases causing histopathological changes of skin tissue or mucosal tissues as the agent(s) for external use, external application or topical application, the pharmaceutical composition(s) of the present invention can be applied for the composition(s) for external use or external application onto skin or the composition(s) for external use or external application onto mucosa such as cosmetics, chemical peeling agent, hair restorer or pilatory, etc. for applying skin tissue or mucosal tissues. For example, it can be applied for an agent(s) for external use or external application for prevention, improvement and/or treatment of senile symptoms of skin (e.g. shrinkage, atrophy, dermatrophia, vulnerability to infection, easy infectivity, looseness, flabbiness, scurf, dandruff, alopecia, depilation, gray hair, slackening, poliosis, itching, roughness, oligosteatosis, asteatosis, exfoliation of keratinocytes, keratinized cells, cornified cells or hornified cells, exfoliation or ablation of the stratum corneum, chapped, cracks, rhagades, spots, blotches, ephelis, chloasma, lines, furrows, wrinkles, freckle, poor regeneration, aplasia, pigmentation, dryness, etc.) and depilation.

As shown in the experimental examples described hereinbefore, in open wound (defect) of skin, quite naturally the hair follicles are rapidly exfoliated, but as a result of external or topical administration to skin of low concentrations of ginsenoside Rb₁, promotion of hair restoration, hair growth and/or pilatory action is clearly observed. Judging from this fact, low dosages or low doses of extracutaneous administration of ginsenoside Rb₁ can be said to regenerate and/or reconstruct the wound region to the condition close to that of the healthy tissue by promoting hair restoration, hair growth and/or pilatory action after development of the open wound (defect of skin). Namely, it was demonstrated that low dosage or low doses of ginsenosides such as ginsenoside Rb₁ could be applied as a promoter(s) of hair restoration, hair growth and/or pilatory action or as an agent(s) for prevention of depilation progress.

As described hereinbefore, the fact that the extracutaneous spread of low concentrations of ginsenoside Rb₁ promotes regeneration and/or reconstruction of cutaneous epidermal tissue, connective tissue of the dermis, dermal papillae, subcutaneous tissue, blood vessels, pilomotor muscles, sebaceous glands, sweat glands, hair papillae, hair follicles, etc., demonstrates quite naturally that the extracutaneous spread of ginsenosides, especially, ginsenoside Rb₁, promotes regeneration and/or reconstruction of epidermal cells, epidermal keratinocytes, melanocytes, Merkel cells, Langerhans cells, cornified cells, keratinized cells, hornified cells, fibroblasts in the dermis and subcutaneous tissue, vascular endothelial cells, vascular smooth muscle cells, sebaceous gland cells, sweat gland cells, pilomotor muscular cells, hair follicle cells, mesenchymal cells, cutaneous stem cells, collagen fibers, elastic fibers, reticular fibers, extracellular or intercellular matrix(matrices), etc. Ginsenosides, especially ginsenoside Rb₁, are thought to promote regeneration and/or reconstruction of all cells and secretion thereof which constitute skin tissues. On the other hand, various symptoms of skin accompanied by aging (atrophy, shrinkage, dermatrophia, vulnerability to infection, easy infectivity, looseness, slackening, flabbiness, itching, roughness, cracks, rhagades, chapped, oligosteatosis, fissure, asteatosis, exfoliation or ablation of keratinocytes, keratinized cells, hornified cells or cornified cells, exfoliation or ablation of the stratum corneum, spots, blotches, lines, furrows, chapping, chapped, ephelis, wrinkle, freckle, poliosis, gray hair, depilation, alopecia, scurf, dandruff, pigmentation, sunburn, poor regeneration, aplasia, dryness, etc.) are thought to occur due to gradual death or dysfunction of the above mentioned cells constituting skin tissue, which is caused by ultraviolet injury or vital senility and/or by disability for the cells to regenerate to the original healthy condition. For example, roughness, dryness, depilation, alopecia, exfoliation or ablation of keratinocytes, keratinized cells, hornified cells or cornified cells, exfoliation or ablation of the stratum corneum, cracks, rhagades, chapped, oligosteatosis, asteatosis, itching, etc. accompanied by aging and senility are thought to develop due to insufficient or poor regeneration of sweat gland cells, hair follicle cells and sebaceous gland cells in the skin after their falling into dysfunction or into the state of death.

Further, sunburn, pigmentation, spots, blotches, ephelis, freckle, etc. can be produced due to insufficient or poor regeneration of cells in the skin to the original state, even if the skin cells, which are irradiated by sun light or ultraviolet ray, are gone to death. Further, it can be said that wrinkles, lines, furrows, flabbiness, slackening, atrophy, shrinkage, etc. of skin are generated as a result that fibroblasts or mesenchymal cells in the dermis and subcutaneous tissue fall into dysfunction or decrease in number depending on the aging and, thus the dermis or subcutaneous tissue can not maintain sufficient collagen fibers, elastic fibers, reticular fibers and/or extracellular matrix (matrices). On the other hand, poliosis or gray hair appear to be increased as a result of functional disorder of melanocytes. Further, as a result of dysfunction of Langerhans cells, immunofunction is reduced to develop easy infectivity or vulnerability to infection. Since the low concentrations of ginsenosides, especially ginsenoside Rb₁, of the present invention can promote regeneration and/or reconstruction of all cells constituting skin tissue and secretory component(s) thereof, if ginsenosides, especially ginsenoside Rb₁, are utilized as a cosmetic composition(s), various symptoms caused by decrease in the constituting cells of skin (cell death) and dysfunction accompanied by aging (atrophy, shrinkage, vulnerability to infection, easy infectivity, flabbiness, loosening, looseness, slackening, itching, roughness, cracks, rhagades, fissure, asteatosis, oligosteatosis, exfoliation or ablation of keratinocytes, keratinized cells, hornified cells or cornified cells, exfoliation or ablation of the stratum corneum, chapped, ephelis, chloasma, spots, blotches, furrows, lines, wrinkles, freckle, depilation, alopecia, poliosis, gray hair, scurf, dandruff, pigmentation, sunburn, poor regeneration, aplasia, dryness, etc.), can be prevented, reduced or improved. Further, ginsenoside Rb₁ is thought to increase the expression of a cell death-suppressing gene product Bcl-x_{L} and to protect skin cells including epidermal cells, epidermal keratinocytes, i.e. keratinocytes, Langerhans cells, Merkel cells, keratinized cells, cornified cells, hornified cells, corneocytes, sebaceous gland cells, hair follicle cells, sweat gland cells, fibroblasts, stem cells, mesenchymal cells, vascular endothelial cells, pilomotor muscular cells, vascular smooth muscle cells, adipocytes, etc. as described in the prior patent application (JP Appln. No. Hei 10-365560, PCT/JP99/02550, Brain cell or nerve cell-protective agent comprising ginsenoside Rb₁) of the present inventor (Sakanaka), consequently, it can also prevent death and functional disorder of constituting cells in the skin accompanied by aging.

As explained hereinabove, ginsenosides, especially ginsenoside Rb₁, are thought to protect all cells constituting the skin. Moreover, even if once cells of the skin enter death or fall into dysfunction, senile symptoms of the skin accompanied by aging (shrinkage or atrophy of skin, vulnerability to infection, easy infectivity, slackening, loosening, flabbiness, itching, roughness, cracks, rhagades, fissure, asteatosis, oligosteatosis, exfoliation or ablation of keratinocytes, keratinized cells, hornified cells or cornified cells, exfoliation or ablation of the stratum corneum, chapped, ephelis, blotches, spots, chloasma, wrinkles, lines, furrows, freckle, alopecia, depilation, gray hair, poliosis, dandruff,scurf, pigmentation, sunburn, poor regeneration, aplasia, dryness, etc.) are thought to be prevented, improved or reduced through ginsenosides-induced regeneration of the skin cells. Namely, ginsenosides, especially ginsenoside Rb₁, can be said to improve, prevent or reduce senile symptoms of the skin accompanied by aging through potent two actions: cytoprotective action and action for promoting tissue and cell regeneration. Moreover, as demonstrated in the experimental results of the present invention and the above described prior patent application (Japanese Patent Appln. No. Hei 10-365560, PCT/JP99/02550), ginsenosides, especially ginsenoside Rb₁, exhibit cytoprotective action and action for promoting tissue and cell regeneration, when the extracellular fluid concentrations in lesioned tissues and/or cutaneous tissues are 1 ng/ml or less, preferably 10 pg/ml or less, more preferably 100 fg/ml or less. Of course, ginsenosides, especially ginsenoside Rb₁, are useful as a health drug composition(s) or a composition(s) for external or topical application to mucosa in order to prevent, improve or reduce senile symptoms of mucosa (atrophy, shrinkage, epithelial exfoliation or ablation, mucosal exfoliation or ablation, poor regeneration, aplasia, chapping, chapped, dryness, etc.).

Consequently, the present invention provides a composition(s) for external or topical application to skin or a composition(s) for external or topical application to mucosa comprising containing ginsenosides, metabolites thereof or salts thereof at low concentrations or low doses, preferably low concentrations or low doses less than 0.001% by weight in the composition.

The composition(s) for external or topical application to skin or the composition(s) for external or topical application to mucosa of the present invention can be applied for every products for external use or external application such as cosmetics, hair restorer, pilatory, toiletries, sanitary goods, composition for chemical peeling, health goods, etc.

The present invention also provides a preventing method, improving method or treating method comprising using the composition(s) for external or topical application to skin or the composition(s) for external or topical application to mucosa of the present invention hereinbefore described.

Further, the present invention provides use of ginsenosides, metabolites thereof or salts thereof for production of the composition(s) for external or topical application to skin or the composition(s) for external or topical application to mucosa of the present invention hereinbefore described. Further, the present invention provides use of ginsenosides, metabolites thereof or salts thereof for the preventing method, improving method or treating method of the above described symptoms or diseases comprising using said composition, and for production thereof.

More detailed embodiment of the composition(s) for external or topical application to skin or the composition(s) for external or topical application to mucosa of the present invention can be mentioned as follows.

The composition(s) for external or topical application to skin or the composition(s) for external or topical application to mucosa of the present invention provides a cosmetic composition(s) for prevention, improvement or treatment of senile symptoms of the skin (atrophy, shrinkage, dermatrophia, vulnerability to infection, easy infectivity, flabbiness, looseness, slackening, dandruff, scurf, depilation, alopecia, gray hair, poliosis, itching, roughness, oligosteatosis asteatosis, keratic cell ablation, exfoliation of keratinocytes, keratinized cells, hornified cells or cornified cells, exfoliation of the stratum corneum, chapped, cracks, rhagades, chapping, ephelis, spots, blotches, chloasma, lines, furrows, wrinkle, freckle, poor regeneration, aplasia, pigmentation, dryness, etc. of skin), a preventing method(s), an improving method(s), a treating method(s) or a makeup method comprising using said composition(s), and use of ginsenosides, metabolites thereof or salts thereof for production thereof.

The composition(s) for external or topical application to skin or the composition(s) for external or topical application to mucosa of the present invention provides the cosmetics, health drugs and health foods for prevention, improvement or treatment of senile symptoms of the skin and mucosa, a preventing method(s), an improving method(s) or a treating method(s) or a makeup method(s) comprising using said composition(s), and use of ginsenosides, metabolites thereof or salts thereof for production thereof.

The composition(s) for external or topical application to skin or the composition(s) for external or topical application to mucosa of the present invention provides a health drug composition(s) for prevention, treatment or improvement of senile symptoms of the mucosa, especially mouth mucosa and esophageal mucosa (atrophy, shrinkage, mucosal exfoliation or ablation, chapped, cracks, rhagades, chapping, epithelial exfoliation or ablation, poor regeneration, aplasia, dryness, etc.), a preventing method(s), an improving method(s) or a treating method(s) comprising using said composition(s), and use of ginsenosides, metabolites thereof or salts thereof for production thereof.

The composition(s) for external or topical application to skin or the composition(s) for external or topical application to mucosa of the present invention provides a composition(s) for hair restoration, hair growth and/or pilatory, a preventing method(s), an improving method(s) or a treating method(s) comprising using said composition(s), and use of ginsenosides, metabolites thereof or salts thereof for production thereof.

The composition(s) for external or topical application to skin or the composition(s) for external or topical application to mucosa of the present invention provides a composition(s) for chemical peeling, a preventing method(s), an improving method(s) or a treating method(s) comprising using said composition(s), and use of ginsenosides, metabolites thereof or salts thereof for production thereof.

The composition(s) for external or topical application to skin or the composition(s) for external or topical application to mucosa of the present invention provides the toiletries or the sanitary goods.

According to results of the present experiments, the amount of intravenous administration for use of ginsenoside Rb₁ as a skin tissue regeneration and reconstruction promoter(s) is similar to the amount of intravenous administration for using said compound as a brain cell or nerve cell-protective agent(s) (Japanese Patent Appln. No. Hei 10-365560, PCT/JP99/02550, Brain cell or nerve cell-protective agents comprising ginsenoside Rb₁). Judging from this fact, the concentrations of ginsenosides, especially ginsenoside Rb₁, which act as a skin tissue or mucosal tissue regeneration and reconstruction promoter(s), are preferably low as described in Japanese Patent Appln. No. Hei 10-365560, PCT/JP99/02550 (Brain cell or nerve cell-protective agents comprising ginsenoside Rb₁), and more concretely, the extracellular fluid concentrations in lesioned regions are 1 ng/ml or less, preferably 10 pg/ml or less, more preferably 100 fg/ml or less. In case that ginsenosides, especially ginsenoside Rb₁, of the present invention are used as a preparation(s) for intravenous administration or a preparation(s) for external or topical application to skin, the preparation(s) is preferably adjusted so that the extracellular fluid concentrations of ginsenosides in lesioned tissues of patients are kept at the above levels. Sufficient effect can be achieved by the pharmaceutical composition(s) and preparation(s) of the present invention even when the extracellular fluid concentrations of ginsenosides, especially ginsenoside Rb₁ in lesioned tissues are about 1 - 100 fg/ml or less (e.g. 0.01 fg/ml). Namely, ginsenosides, especially ginsenoside Rb₁, are thought to exhibit the superior effectiveness and efficacy, when the extracellular fluid concentrations in lesioned tissues are 0.01 - 100 fg/ml or 1 - 10,000 fg/ml.

Further, when ginsenosides, especially ginsenoside Rb₁, are used in order to achieve prevention, treatment and/or improvement of senile symptoms of skin and mucosa (atrophy, shrinkage, dermatrophia, vulnerability to infection, easy infectivity, flabbiness, looseness, slackening, dandruff, scurf, depilation, alopecia, gray hair, poliosis, itching, roughness, oligosteatosis, asteatosis, exfoliation of keratinocytes, keratinized cells, hornified cells or cornified cells, keratic cell ablation, exfoliation or ablation of the stratum corneum, chapped, cracks, rhagades, chapping, ephelis, spots, blotches, chloasma, wrinkle, lines, furrows, freckle, poor regeneration, aplasia, pigmentation, dryness, etc.) by using as a composition(s) of cosmetics, health-promoting drug or health drug, the admixed amount of ginsenosides, especially ginsenoside Rb₁, in cosmetics, health-promoting drug or health drug should be adjusted so that the extracellular fluid concentrations of ginsenosides, especially ginsenoside Rb₁, in the local region of skin or mucosa are kept at the low levels as described hereinbefore. Of course, although ginsenosides can be mixed to cosmetics together with other cosmetic composition(s) described in U.S. Patent 5,663,160 or WO 99/07338, the composition(s) of the present invention has specific feature to use it at lower concentrations than the concentrations as described in U.S. Patent 5,663,160 or WO 99/07338. Ginsenosides, especially ginsenoside Rb₁, can be admixed in every cosmetics or health drug (health-promoting drug) together with any other cosmetic composition(s) or health drug composition(s), and the other cosmetic composition(s) or health drug composition(s) used with ginsenosides, especially ginsenoside Rb₁, should preferably be lower in concentration(s) than the concentration as described in the prior references and patent specifications.

Consequently, the cosmetic composition(s) or health drug composition(s) of the present invention includes not only the conventional cosmetics but also all products, which are directly or indirectly in contact with vital or viable epidermis or mucosa including oral cavity mucosa, for example quasi drugs such as tooth paste, shampoo and health goods.

Consequently, when trace amount of ginsenosides, especially ginsenoside Rb₁, is admixed into every cosmetics or drug for health (cosmetic lotion (skin lotion), beauty liquid, agent for massage, agent for pack, emulsion, milky lotion, foundation cream, hand cream, cold cream, lotion, gel, emulsion, body milk, hair dye, hair manicure, eye shadow, cleansing cream, cleansing foam, night cream, beauty cream, troches, candy for cough, sweet, water for health, isotonic water, sherbet, ice, health foods, candy, face powder, eye wash, cleansing liquid, collyrium, lipstick, cosmetic soap, gargle, shampoo, hair rinse, tooth powder, tooth paste, bath gel, lip cream, hair tonic, hair liquid, makeup base, UV liquid foundation, powder foundation, etc.) and used to maintain the extracellular fluid concentrations of ginsenosides, especially ginsenoside Rb₁, in local region of skin or local region of mucosa at the low levels hereinbefore, excellent effect can be exhibited on senile symptoms of the skin accompanied by aging (atrophy, shrinkage, vulnerability to infection, easy infectivity, flabbiness, loosening, slackening, itching, roughness, cracks, rhagades, oligosteatosis, fissure, asteatosis, poor regeneration, aplasia, epithelial ablation or exfoliation, mucosal ablation or exfoliation, corneocyte ablation, horny layer ablation, exfoliation or ablation of keratinocytes, keratinized cells, cornified cells or hornified cells, exfoliation or ablation of the stratum corneum, chap, chapped, spots, blotches, chloasma, ephelis, wrinkles, lines, furrows, freckle, depilation, alopecia, gray hair, poliosis, scurf, dandruff, pigmentation, sunburn, dryness, etc.).

For example, only lack or shortage of skin fat (i.e. sebum) accompanied by aging or senility causes roughness, itching, cracks, rhagades, fissure, exfoliation or ablation of keratinocytes, keratinized cells, cornified cells or hornified cells, exfoliation or ablation of the stratum corneum, corneocyte ablation, horny layer ablation, chapped, chapping, dryness, etc., but as a result of applying every cosmetics admixed with ginsenosides, especially ginsenoside Rb₁, at low concentrations, protection or regeneration and/or reconstruction of the sebaceous glands are promoted and the above-described senile symptoms of skin accompanied by aging are thought to be prevented, improved or reduced. Since any cosmetics comprising or containing the low concentrations of ginsenosides, especially ginsenoside Rb₁, not only protect epidermal cells (epidermal keratinocytes, keratinized cells, cornified cells, hornified cells and/or corneocytes) but also promote regeneration thereof, as the results of promoting production and secretion of intercorneocytic lipids (i.e. lipids between individual hornified cells, keratinized cells, cornified cells, corneocytes or keratinocytes) and natural humectant factors, skin dryness and roughness are suppressed to provide natural moisture in the skin. Further, as the results of admixing ginsenosides, especially ginsenoside Rb₁, a ginseng extract(s) or a crude saponin fraction(s) of ginseng into mineral water, injury of mouth mucosa or digestive tract mucosa (especially esophageal mucosa) caused by alcoholic beverage or high temperature irritation can be improved, prevented or treated. Low concentrations of ginsenosides, especially ginsenoside Rb₁, can be used as a composition(s) for promoting regeneration and/or reconstruction of skin tissue in the chemical peeling. Ginsenosides, especially ginsenoside Rb₁, is useful for prevention, treatment and/or improvement of senility or disease of skin by using as bath gel in the nursing facilities, hot spa health facilities, hospitals, etc. As a result of mixing ginsenosides, especially ginsenoside Rb₁, with dressing agents, antiseptics, washing lotion, plaster, coating agents, etc. wound healing is promoted or deterioration of wound is prevented. Bases for the composition(s) for external or topical application to skin of the present invention and the other composition(s), which can be combined, are already described.

In case that the composition(s) for external or topical application to skin or the composition(s) for external or topical application to mucosa is used as a composition(s) for hair restoration, hair growth or pilatory, after admixing the effective component(s) with optional or known base, the mixture can be used independently or can be used in combination with other effective or pharmaceutical composition(s) (e.g. composition(s) for promoting blood flow, composition(s) for local stimulation, composition(s) for activating hair follicle, antiandrogen(s), antiseborrheic composition(s), composition(s) for keratolysis, antibiotics, galenical extract(s), vitamins, amino acids, etc.). In that occasion, the upper limit of concentration of ginsenoside Rb₁ is preferably set at levels less than 0.00002% by weight or under 0.00002% by weight. Concretely, intravenous administration or local external application of ginsenosides, especially ginsenoside Rb₁, in low dosages or low doses appears to be effective for alopecia areata, androgenic alopecia, common baldness and/or diffuse alopecia.

When ginsenosides, especially ginsenoside Rb₁, are admixed into liquid cosmetics such as conventional UV liquid foundation, concentration thereof is adjusted to be at 1000 ng/ml or less, preferably 10 ng/ml or less, more preferably 0.01 fg/ml-100 pg/ml; and if the mixture is applied externally or sprayed externally onto skin every day, the extracellular fluid concentrations of ginsenosides, especially ginsenoside Rb₁, in the local region of skin can be maintained at low levels as described hereinbefore, then the liquid cosmetics is useful for improvement, prevention of progress or prevention of deterioration of senile symptoms of the skin (atrophy, shrinkage, dermatrophia, vulnerability to infection, easy infectivity, looseness, flabbiness, slackening, itching, alopecia, depilation, dandruff, scurf, gray hair, poliosis, roughness, cracks, rhagades, fissure, oligosteatosis, asteatosis, keratic cell ablation, exfoliation or ablation of keratinocytes, keratinized cells, hornified cells or cornified cells, exfoliation or ablation of the stratum corneum, corneum ablation, chapped, chapping, dryness, spots, blotches, chloasma, ephelis, wrinkles, lines, furrows, freckle, pigmentation, poor regeneration, aplasia, sunburn, etc. of skin). When ginsenosides, especially ginsenoside Rb₁, is admixed into solid, gelled or creamy cosmetics, such as conventional makeup base or night cream, the amount of admixed ginsenosides, especially ginsenoside Rb₁, is controlled to be 1000 ng or less, preferably 10 ng or less, more preferably 0.01 fg - 100 pg per g of cream; and if the mixture is applied topically or externally onto skin for consecutive days, the external fluid concentrations of ginsenosides, especially ginsenoside Rb₁, in the local region of skin are maintained at low levels as described hereinbefore, then the cosmetics is useful for improvement, prevention and/or treatment of senile symptoms of the skin (atrophy, shrinkage, dermatrophia, vulnerability to infection, easy infectivity, looseness, flabbiness, slackening, itching, roughness, cracks, rhagades, fissure, oligosteatosis, asteatosis, keratic cell ablation, exfoliation or ablation of keratinocytes, keratinized cells, cornified cells or hornified cells, exfoliation or ablation of the stratum corneum, corneum ablation, chapped, chapping, dryness, spots, blotches, ephelis, chloasma, wrinkles, lines, furrows, freckle, poliosis, gray hair, scurf, dandruff, depilation, alopecia, pigmentation, poor regeneration, aplasia, sunburn, etc. of skin).

Proviso that when ginsenosides, especially ginsenoside Rb₁, are admixed in any cosmetics and used for extremely long term, the content of ginsenosides, especially ginsenoside Rb₁, per 1 g of cosmetics can be reduced to 0.0000001 fg. The upper limit of concentration of ginsenosides, especially ginsenoside Rb₁, admixed in the above cosmetics for the purpose of prevention, improvement or treatment of senile symptoms of the skin, is 10 µg/ml or less by weight or less in case of the liquid cosmetics, and 10 µg/g or less in case of gelled or creamy cosmetics. In other words, ginsenosides, especially ginsenoside Rb₁, are admixed preferably in the above described cosmetics at concentrations of 0.001% by weight or less, preferably at concentrations less than 0.001% by weight. If not, membrane of normal cells in the skin may be damaged. Namely, when ginsenosides, especially ginsenoside Rb₁, are admixed into the cosmetics or health drugs, which are used for long term, to make lower the concentrations of ginsenosides, especially ginsenoside Rb₁, than those in the agent(s) for external application for prevention, treatment or therapy of skin diseases and mucosal diseases as previously described in the present invention is thought to be safe. Of course, as described hereinabove, the cosmetics comprising or containing trace amount of ginsenosides, especially ginsenoside Rb₁, can be applied or sprayed externally onto the face and also applied or sprayed externally to the other skin regions(e.g. extremities, trunk, neck, head, etc.) which are frequently irradiated by sun light. As described, when the cosmetics or health drugs comprising or containing ginsenosides, especially ginsenoside Rb₁, are used usually for long term, symptoms accompanied by senility of skin (atrophy, shrinkage, vulnerability to infection, easy infectivity, flabbiness, loosening, slackening, itching, roughness, cracks, rhagades, fissure, asteatosis, oligosteatosis, keratic cell ablation, corneum ablation, exfoliation or ablation of keratinocytes, keratinized cells, cornified cells or hornified cells, exfoliation or ablation of the stratum corneum, chapped, chapping, spots, blotches, chloasma, ephelis, lines, furrows, wrinkles, freckle, gray hair, poliosis, dandruff, scurf, alopecia depilation, pigmentation, sunburn, poor regeneration aplasia, dryness, etc. of skin) can be prevented or improved. Proviso that, the amount of ginsenosides, especially ginsenoside Rb₁, admixed into the agent(s) for external or topical application to skin, agent(s) for external or topical application to mucosa, health drugs or cosmetics, is tentative value and it should be actually adjusted so that the extracellular fluid concentrations of ginsenosides, especially ginsenoside Rb₁, in the skin or mucosa are kept at 1 ng/ml or less, preferably 10 pg/ml or less, more preferably 100 fg/ml or less.

Of course, senility of mucosa (shrinkage, epithelial ablation or exfoliation, poor regeneration, aplasia, mucosal ablation or exfoliation, chapped, rhagades, chapping, dryness, etc.) can be prevented, improved or reduced by admixing ginsenosides, especially ginsenoside Rb₁, into health drugs (e.g. gargle, eye wash, etc.) at low concentrations. When ginsenosides, especially ginsenoside Rb₁, or later described crude saponin fraction(s) of ginseng, ginseng extract(s) or ginseng are used as a composition(s) of heath drugs (health-promoting drugs) such as health beverages and foods, gargle, bath gel, eye wash, etc., undiluted solution of health drug is prepared with the concentration(s) of preferably 0.001% by weight or lower or the concentration(s) less than 0.001% by weight, and the concentrations of ginsenosides, especially ginsenoside Rb₁, or crude saponin fraction(s) of ginseng, ginseng extract(s) or ginseng at use are diluted to be 1 ng/ml or less or 14.5 ng/ml or less, preferably 10 pg/ml or less or 145 pg/ml or less, more preferably 100 fg/ml or less or 1450 fg/ml or less. Of course, after freeze drying the above-described undiluted solution, it may be used as powdery bulk for health drug. In case of lyophilized powder of the undiluted solution, quite naturally, per cent by weight of the health drug composition of said powder is increased, but the lyophilized powder may be diluted at use to low concentrations of the health drug composition as described above. Generally, the optimum extracellular fluid concentrations of crude saponin fraction of ginseng in lesioned tissue are thought to be 14.5-fold of the optimum extracellular fluid concentrations of ginsenoside Rb₁.

As demonstrated from the culture experiments described hereinbefore, the pharmaceutical composition(s) or the drug composition(s) of the present invention can be applied not only to human but also to the vital or viable tissues of plants, livestock and pets, and can regulate growth of animals or plants.

Consequently, the present invention provides a composition(s) for growth-regulation for promoting generation, regeneration, growth, reconstruction, differentiation, preservation, stock, nourishment or cultivation of tissues or cells of plants or animals comprising ginsenosides, metabolites thereof or salts thereof, preferably ginseng, a ginseng extract(s), a crude saponin fraction(s) of ginseng, ginsenosides or salts thereof.

Further, the present invention provides a method(s) for cultivation of plants or a method(s) for rearing or raising animals comprising using the above-described composition(s) for growth-regulation of the present invention.

The composition(s) for growth-regulation of the present invention includes a composition(s) for growth-regulation of plants for promoting generation, regeneration, growth, reconstruction, differentiation, preservation, stock, nourishment or cultivation of tissues or cells of plants, and a composition(s) for growth-regulation of animals for growth, raising, nourishment, protection or culture of marine products, marine resources, aquatic products, fisheries resources, marine animals, aquatic animals, pets or livestock.

More particularly, examples of the composition(s) for growth-regulation of the present invention are growth promoter(s), fertilizer additive(s), fertilizer composition(s), etc. of plants and growth promoter(s), feed additive(s), feed composition(s), etc. of animals.

Low concentrations, low doses and/or low dosages of ginsenosides, especially ginsenoside Rb₁, of the present invention can be utilized for prevention, therapy and/or treatment of disease, trauma or wound of not only human but also pets and livestock.

Further, low concentrations of ginsenosides, especially ginsenoside Rb₁, can be utilized for cultivation of sea products (fish and shellfish, crustacean, eel, conger, sea urchin, oyster, wakame, pearl shell, pearl oyster, etc.) and aquatic products or for cultivation of farm products. Of course, low concentrations of ginsenosides, especially ginsenoside Rb₁, can be utilized for protection, raising and/or rearing of fish and shellfish cultivated in aquaria and others. In this case, ginsenosides, especially ginsenoside Rb₁, are thought to protect marine resources and farm products from endocrine disrupters, toxins, trauma, micro-organisms, microbes, biohazards, environmental pollutions, etc. Further, in the hydroponics, ginsenosides, especially ginsenoside Rb₁, are added into water of cultivation for an increased yield of vegetables.

Embodiments of the composition(s) for growth-regulation of the present invention can be mentioned as follows.

The present invention provides a composition(s) for promoting regeneration, generation or reconstruction of plant tissues, a composition(s) for growth-regulation or a fertilizer composition(s) comprising ginsenosides, metabolites thereof or salts thereof, preferably ginseng, its extract(s), its components, metabolites thereof or salts thereof.

The present invention provides a fertilizer additive(s) or a fertilizer composition(s) for prevention, treatment, restoration or therapy of injury, trauma, cutting or defect of plant tissues comprising ginsenosides, metabolites thereof or salts thereof, preferably ginseng, its extract(s), its components, metabolites thereof or salts thereof useful for promoting regeneration, generation or reconstruction of plant tissues.

The present invention provides a fertilizer additive(s) or a fertilizer composition(s) comprising ginsenosides, metabolites thereof or salts thereof, preferably ginseng, its extract(s), its components, metabolites thereof or salts thereof useful for cuttings or hydroponics of plant tissues such as stem or branch of pothos.

The present invention provides a feed additive(s) or a feed composition(s) comprising ginsenosides, metabolites thereof or salts thereof, preferably ginseng, its extract(s), its components, metabolites thereof or salts thereof.

More particularly, the present invention relates to low concentrations of a crude saponin fraction(s) of ginseng or ginsenosides, especially ginsenoside Rb₁, useful for cuttings or hydroponics of plant tissues such as stem or branch of pothos. Namely, the present invention provides a composition(s) or a fertilizer additive(s) for promotion of rooting, budding or growth comprising ginsenosides, metabolites thereof or salts thereof, preferably ginseng, its extract(s), its components, metabolites thereof or salts thereof useful for cultivation, growth, preservation or improvement of plant, preservation of fresh flower, hydroponics, cultivation or growth of farm products, cultivation or growth of vegetables, cultivation and/or growth of fruits or fruit tree, cultivation and/or growth of tobacco, cultivation, growth and/or improvement of garden plants, cultivation of medicinal plants, cultivation of mushrooms, or cultivation and/or growth of tea-leaves.

The fertilizer additive(s) or fertilizer composition(s) of the present invention is preferably comprising low concentrations of ginseng, its extract(s), its components, metabolites thereof or salts thereof. When ginseng components such as ginsenosides, especially ginsenoside Rb₁, is used as a fertilizer composition(s) in hydroponics independently or together with other effective component(s), the concentrations of ginsenosides, especially ginsenoside Rb₁, in solution of the hydroponics is adjusted to 1 ng/ml or less, preferably 10 pg/ml or less, more preferably 100 fg/ml or less. Ginsenosides, especially ginsenoside Rb₁, of the present invention promote sufficiently rooting, budding, growth, differentiation, regeneration, generation or reconstruction of plant tissues in the hydroponics, even in the concentration range around 0.01 fg/ml, and are utilized for preservation, growth, cultivation or improvement of all farm products and plants including vegetables, fruits and fresh flowers. Further, when ginsenosides, especially ginsenoside Rb₁, are added or admixed into any solid fertilizer, gel fertilizer, liquefied fertilizer, liquid fertilizer, powdery fertilizer (e.g. straight fertilizer, soil amendment matter or fertilizer, seedling fertilizer, fertilizer for paddy and barley, slow-release fertilizer, high-analysis compound fertilizer, low-analysis compound fertilizer, organic compound fertilizer, NK-PK-PM compound fertilizer, organic mixed fertilizer, liquid fertilizer, etc.), concentration thereof is, as same in the cosmetics, preferably set to 0.001% by weight or less or lower, 10⁻²⁰% or more. The upper limit of concentration of ginsenosides, especially ginsenoside Rb₁, as the fertilizer composition(s) is 0.1% by weight or less, preferably 0.001% by weight or less. When a crude saponin fraction(s) of ginseng of the present invention is used as a fertilizer additive(s) or a fertilizer composition(s) or is used independently in hydroponics, the concentration of the crude saponin fraction(s) in the solution of hydroponics is adjusted to 14.5 ng/ml or less, preferably 145 pg/ml or less, more preferably 1450 fg/ml or less. The crude saponin fraction(s) of ginseng of the present invention promotes sufficiently rooting, budding, growth, differentiation, regeneration, generation or reconstruction of plant tissues in the hydroponics, even in the concentration range about 0.145 - 1450 fg/ml, and are utilized for preservation, growth, cultivation or improvement of all farm products and plants including vegetables, fruits and fresh flowers. Further, when a crude saponin fraction(s) of ginseng is added or admixed into any solid fertilizer, gel fertilizer, liquefied fertilizer, liquid fertilizer, powdery fertilizer (e.g. straight fertilizer, soilamendment matter or fertilizer, seedling fertilizer, fertilizer for paddy and barley, slow-release fertilizer, high-analysis compound fertilizer, low-analysis compound fertilizer, organic compound fertilizer, NK-PK-PM compound fertilizer, organic mixed fertilizer, liquid fertilizer, etc.), concentration thereof is, as same in the cosmetics, preferably set at 0.01% by weight or less, preferably 0.001% by weight or less, more preferably 0.0001% by weight or less, and 10⁻²⁰% by weight or more. of course, amount of ginseng or ginseng extract equal to or 5 - 6-fold or less of the crude saponin fraction(s) of ginseng may be used as a fertilizer composition(s). The upper limit of concentration of the crude saponin fraction(s) and a ginseng extract(s) as a fertilizer composition(s) is 1% by weight or less.

As demonstrated in the experiments hereinafter, when plant tissue such as cutting of pothos is cultured in aqueous solution containing 100 fg/ml of ginsenoside Rb₁ or 1450 fg/ml of a crude saponin fraction of ginseng, generation of root (i.e. rooting and/or growth) is obviously promoted as compared with the control cutting. Judging from this case, ginsenosides, especially ginsenoside Rb₁, a crude saponin fraction(s) of ginseng, a ginseng extract(s) or ginseng containing the crude saponin fraction(s) can promote generation, regeneration or reconstruction of not only animal tissues but also plant tissues.

Furthermore, it was found in the present invention that the extracellular fluid concentrations of ginsenoside Rb₁ or crude saponin fraction which promotes generation, regeneration or reconstruction of plant tissues were, as same manner in case of regeneration and/or reconstruction of animal tissues (skin tissue and mouth mucosal tissue), extremely low. Consequently, ginseng, a ginseng extract(s), a crude saponin fraction(s) of ginseng and ginsenosides, especially ginsenoside Rb₁, can be applied for cultivation, growth or preservation of plant, preservation, cultivation or growth of fresh flower, hydroponics, cultivation or growth of farm products, cultivation or growth of vegetables, cultivation and/or growth of fruits, cultivation and/or growth of tobacco, cultivation of mushrooms, cultivation of medicinal plant or cultivation and/or growth of tea-leaves. The fertilizer additive(s) or fertilizer composition(s) comprising the above described ginseng, ginseng extract, crude saponin fraction of ginseng or ginsenosides, especially ginsenoside Rb₁, can be admixed to any fertilizers, preferably at low concentrations, or can be used as a rooting, budding, generation, regeneration, growth or reconstruction promoter(s) for plant tissues independently.

The fact that ginsenosides, especially ginsenoside Rb₁, and the crude saponin fraction(s) of ginseng can promote generation, regeneration or reconstruction of not only animal tissues (skin tissue and mouth mucosal tissue) but also plant tissues, indicates that ginseng, a ginseng extract(s), a crude saponin fraction(s) of ginseng or ginsenosides such as ginsenoside Rb₁, can promote generation, regeneration or reconstruction of all vital, living or viable tissues. Consequently, ginseng, a ginseng extract(s), a crude saponin fraction(s) of ginseng or ginsenosides, especially ginsenoside Rb₁, can be utilized as an additive(s) to feeds for livestock, cultivated fish and shellfish and pet animals, i.e. feed composition. For example, in the cultivation of fish and shellfish, crustacean, eel, sea urchin, conger, pearl shell, pearl oyster, etc., addition of low concentrations of ginseng, a ginseng extract(s), a crude saponin fraction(s) of ginseng and ginsenosides, especially ginsenoside Rb₁, to sea water or fresh water together with conventional feeds is thought to promote.growth of these aquatic or marine resources. Of course, ginseng, a ginseng extract(s), a crude saponin fraction(s) of ginseng and ginsenosides, especially ginsenoside Rb₁, can protect marine and aquatic resources such as fish and shellfish, crustacean, eel, conger, etc. from trauma, wound, pathogenic microorganisms, pathological microbes, biohazards, endocrine disrupters, environmental pollutions, toxins, etc. through their cytoprotective actions. Namely, the feed additive(s) or the feed composition(s) of the present invention will be essential for secure human from forthcoming food crisis. As explained hereinabove, a crude saponin fraction(s) of ginseng, a ginseng extract(s), ginseng or ginsenosides can be used in the concentration range from 10⁻²⁰% by weight to 0.1% by weight or 1% by weight as a composition(s) for growth modulation or growth regulation.

The fact that ginsenosides such as ginsenoside Rb₁ or a crude saponin fraction(s) can promote regeneration, generation or reconstruction of plant tissues supports that low concentrations of crude saponin fraction(s) or ginseng extract or ginseng containing the crude saponin fraction(s) can be utilized as a composition(s) for chemical peeling, a cosmetic composition(s), a health drug composition(s), a composition for hair restorer, hair growth and/or pilatory, a pharmaceutical composition or as a veterinary drug composition(s) by promoting regeneration or reconstruction of skin tissue as well.

Individual compositions of the present invention as described above, i.e. pharmaceutical composition, veterinary drug composition, composition for external or topical application to skin, composition for external or topical application to mucosa, health drug composition, cosmetic composition, composition for hair restoration, hair growth and/or pilatory, composition for growth regulation or growth modulation, etc. are preferably comprising ginsenosides such as ginsenoside Rb₁, metabolites thereof or salts thereof at low concentrations.

They can be in the form of parenteral administration such as intravenous administration, mucosal administration and external cutaneous administration. More precisely, the pharmaceutical composition(s), veterinary drug composition(s), health drug composition(s) or cosmetic composition(s) of the present invention is preferably used as a preparation(s) for parenteral administration, an agent(s) for external or topical application to mucosa, an agent(s) for external spray on mucosa, an agent(s) for external or topical application to skin or as an agent(s) for external spray on skin comprising containing ginsenosides derivatives, especially dihydroginsenoside Rb₁, metabolites thereof or salts thereof at low concentrations.

Further, the present invention provides a preparation(s) for parenteral administration, preferably a preparation(s) for intravenous or intravascular administration, an agent(s) for external or topical application to mucosa or an agent(s) for external or topical application to skin, for prevention, treatment or therapy of diseases causing histopathological changes of vital or viable tissues comprising ginsenoside derivatives, especially dihydroginsenoside Rb₁, metabolites thereof or salts thereof at low concentrations.

Further, the present invention provides a cosmetics or health drug for prevention, treatment or improvement of senile symptoms of skin or mucosa comprising ginsenoside derivatives, especially dihydroginsenoside Rb₁, metabolites thereof or salts thereof, preferably in low concentration.

These compositions of the present invention are preferably comprised or contained in a preparation(s) for intravenous administration, a preparation(s) for external or topical application to mucosa, a preparation(s) for external use on skin, a preparation(s) for external or topical application to skin or a preparation(s) for external spray on skin, and an agent(s) for external use on local lesion, an injection(s) for local lesion, an oral preparation(s), nasal drops, ear drops, eye drops, eye ointment, suppository, subcutaneous injection, intracutaneous injection, intramuscular injection, inhalation, sublingual tablets, artificial salivary, an agent(s) for intraarticular administration, an agent(s) for percutaneous absorption, etc., and preparation for any route of administration can be selected. Sustained release preparation can also be used.

As explained hereinabove, intravenous administration, extramucosal administration or extracutaneous administration of ginsenosides, especially ginsenoside Rb₁, is thought to exhibit effectiveness and efficacy for prevention, therapy or treatment of all skin diseases or oral cavity diseases described hereinbefore through regeneration and reconstruction-promoting action or wound healing-promoting action on skin tissue and mucosal tissues. Accordingly, low concentrations and/or low doses of ginsenosides, especially ginsenoside Rb₁, are thought to be the first compound, which can regenerate and/or reconstruct the injured skin tissue or mucosal tissues nearly to the condition before injury, in the human history.

Namely, we have found for the first time that ginsenosides, especially ginsenoside Rb₁, or metabolites thereof have action for promoting regeneration and/or reconstruction of skin tissue or mucosal tissues, especially action for promoting regeneration and/or reconstruction of skin tissue after incised wound, open wound or defect of skin, action for promoting regeneration and/or reconstruction of mucosal tissues after morsus of human mouth mucosa, action for promoting wound healing or action for promoting regeneration and/or reconstruction of epidermis, dermis, dermal papillae, subcutaneous tissue, lamina propria, muscular tissue, connective tissue, hair follicles, hair papillae, sebaceous glands, sweat glands, salivary glands, mucous glands, mixed glands, peripheral nerves, blood vessels, fibroblasts, stem cells, mesenchymal cells, epithelial cells, glandular cells, myoepithelial cells, epidermal cells, epidermal keratinocytes, keratinized cells, corneocytes, cornified cells, hornified cells, melanocytes, Merkel cells, vascular endothelial cells, pilomotor muscle cells, vascular smooth muscle cells, smooth muscle cells, muscular cells, collagen fibers, elastic fibers, reticular fibers or extracellular matrices (matrix).

Consequently, the present invention provides use of ginsenosides, especially ginsenoside Rb₁, or metabolites thereof as a leading compound(s) for exploring or screening other effective compounds for prevention, treatment or therapy of the above described diseases of skin tissue, mouth tissue or the other organs or tissues. Further, it is possible to select any route of administration after preparing a prodrug(s), which is prepared by modifying a part(s) of chemical structure of ginsenosides, especially ginsenoside Rb₁. Furthermore, as a result of identifying the target molecule of ginsenosides, especially ginsenoside Rb₁, or metabolites thereof, it is possible to aim at the development of agents for treatment or therapy of skin diseases, wound healing promoters, skin tissue regeneration and reconstruction promoters, agents for treatment or therapy of oral diseases, mucosal tissue regeneration and reconstruction promoters or cosmetics for suppressing senile symptoms of skin or mucosa by synthesizing a compound(s), which modifies function of the target molecule.

The present invention provides ginsenosides, especially ginsenoside Rb₁, or metabolites thereof as a leading compound(s) for exploring or screening a new effective component(s) or compound(s) for prevention, treatment or therapy of these diseases mentioned above. Of course, a composition(s) or fertilizer composition(s) for promoting rooting, budding, growth, differentiation, generation, regeneration or reconstruction of plant tissues can be newly developed by utilizing ginseng or extract(s) thereof, or components of ginseng including ginsenoside Rb₁, or metabolites thereof as a leading compound(s).

Accordingly, in future, large numbers of agent for prevention, therapy or treatment of diseases caused by injury, wound, trauma or defect of skin tissue or mucosal tissues, or diseases causing histopathological changes of skin, oral cavity and the other organs or mucosa can be prepared.

Namely, the present invention is to find out that ginsenosides, especially ginsenoside Rb₁, or metabolites thereof are extremely effective for prevention, treatment or therapy for all diseases of skin tissues. Consequently, the present invention relates to a method for exploring or screening effective components or compounds for prevention, treatment or therapy of skin diseases or mouth mucosa diseases hereinafter explained or all mucosa diseases comprising applying ginsenosides, especially ginsenoside Rb₁, or metabolites thereof as a leading compound(s). Further, the present invention relates to use of ginsenosides, especially ginsenoside Rb₁, or metabolites thereof as a leading compound(s) for exploring or screening effective components or compounds for prevention, treatment or therapy of diseases of skin tissue or mucosa. The present invention also relates to an agent(s) for prevention, treatment or therapy of diseases of skin tissue or mouth mucosa obtained by the method or use as described hereinbefore.

Next, an example for creating novel derivatives with the use of ginsenosides, especially ginsenoside Rb₁, or metabolites thereof as a leading compound(s) is exhibited. This example relates to dihydroginsenoside Rb₁ chemically modified by hydrogen reduction of ginsenoside Rb₁.

We have found for the first time that ginsenoside derivatives, especially dihydroginsenoside Rb₁, or metabolites thereof have action for promoting regeneration and/or reconstruction of skin tissue, especially action for promoting regeneration and/or reconstruction of skin tissue after open wound or defect of skin, action for promoting wound healing or action for promoting regeneration and/or reconstruction of epidermis, dermis, dermal papilla, subcutaneous tissue, connective tissue, hair follicles, hair papillae, sebaceous glands, sweat glands, peripheral nerves, blood vessels, fibroblasts, stem cells, mesenchymal cells, epithelial cells, glandular cells, myoepithelial cells, epidermal cells, epidermal keratinocytes, keratinized cells, corneocytes, hornified cells, cornified cells, melanocytes, Merkel cells, vascular endothelial cells, pilomotor muscle cells, vascular smooth muscle cells, smooth muscle cells, muscular cells, collagen fibers, elastic fibers, reticular fibers or extracellular matrix (matrices). Consequently, the present invention proves use of ginsenosides, especially ginsenoside Rb₁, or metabolites thereof as a leading compound(s) for exploring or screening other effective compounds for prevention, treatment or therapy of diseases of the above described skin tissue or mouth tissue and the. other organs or tissues. Further, it is possible to select any route of administration after preparing a prodrug(s), which is prepared by modifying a part(s) of chemical structure of ginsenoside derivatives, especially dihydroginsenoside Rb₁. Of course, the present invention demonstrates that a growth-regulating composition(s) or fertilizer composition(s) for promoting rooting, budding, growth, differentiation, generation, regeneration or reconstruction of plant tissues can be newly developed by utilizing ginseng or extract(s) thereof, or components of ginseng such as ginsenoside Rb₁, or metabolites thereof as a leading compound(s).

Dihydroginsenoside Rb₁ of the present invention is represented by the following formula: 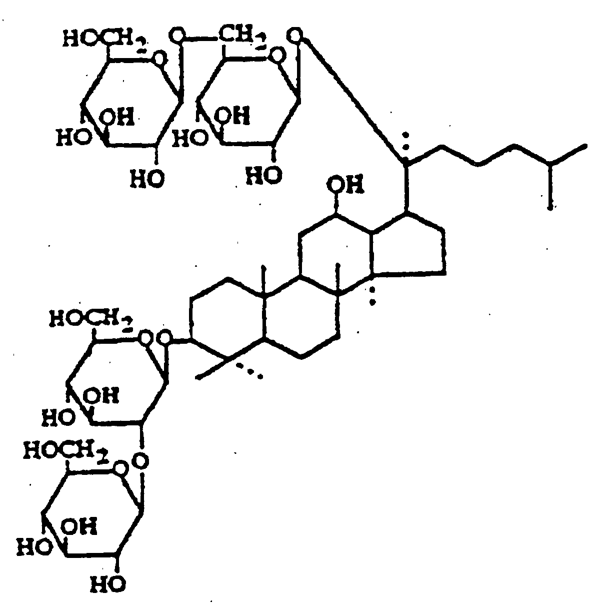 It is described in PCT/JP00/04102 (Brain cell or nerve cell protecting agents comprising ginseng) that dihydroginsenoside Rb₁ can be produced by the following method. Firstly, 10% Pd/c (palladium charcoal) 10.2 mg was weighed, and poured into a two-neck flask with stop cock. Methanol (GR) (1 ml) was added to suspend. Hydrogen balloon (appx. 1.1 atom.) was attached to the flask and the catalyst was activated at 0 C for 30 minutes. Ginsenoside Rb₁ (19.9 mg) dissolved in methanol (1 ml) was injected into the flask through a syringe. The mixture was vigorously stirred at 0 C for 10 hours and 30 minutes with the use of a magnetic stirrer. The reaction mixture was filtered by using filter paper and membrane filter with pores 0.45 µm in diameter. The product was dissolved in pure water (10 ml) and freeze-dried to obtain 19.1 mg of dihydroginsenoside Rb₁ (yield: 97%) as white powder. The melting point of dihydroginsenoside Rb₁ is 193 - 195 C. Incidentally, the melting point of ginsenoside Rb₁ is 197-198 C (reference value). NMR chart of dihydroginsenoside Rb₁ is shown in PCT/JP00/04102. The thus produced dihydroginsenoside Rb₁ is confirmed to have purity of 98% or more by NMR spectrum and HPLC. Ginsenosides other than ginsenoside Rb₁ can be dihydrogenated by the similar method of reduction.

Ginsenoside derivatives of the present invention, especially dihydroginsenoside Rb₁ can be used in the free form as described in PCT/JP00/04102, but can also be used together with proper salts. These can also be used in the form of solvate such as hydrate. Ginsenoside derivatives of the present invention, especially dihydroginsenoside Rb₁ can be admixed, preferably at low concentrations, as same manner in ginsenoside Rb₁, into every cosmetics or drugs for health (cosmetic lotion (skin lotion), milky lotion, beauty liquid, agent for massage, agent for pack, emulsion, foundation cream, gel, lotion, emulsion, powder, hair dye, hair manicure, hand cream, cold cream, eye shadow, cleansing cream, cleansing foam, night cream, beauty cream, troches, face powder, lipstick, cosmetic soap, bath gel, gargle, water for health, isotonic water, ice, sherbet, ice cream, eye wash, collyrium, cleansing liquid, shampoo, hair rinse, tooth powder, tooth paste, lip cream, makeup base, hair liquid, hair tonic, UV liquid foundation, powder foundation, etc.). Base for ginsenoside derivatives and composition for external or topical application to skin, which can be used in combination with ginsenoside derivatives, are the same as in ginsenoside Rb₁. According to the experimental results of the present invention, the extracellular fluid concentrations of dihydroginsenoside Rb₁ used as a cytoprotective agent is 100 ng/ml or less, preferably 10 pg/ml or less, more preferably 100 fg/ml or less. It is demonstrated in the example hereinbelow that the agent(s) or preparation(s) for external or topical application to skin comprising dihydroginsenoside Rb₁ at concentrations of 0.00001% by weight (10⁻⁵% by weight) - 0.0000001 % by weight (10⁻⁷% by weight), significantly reduced the open wound. In addition, dihydroginsenoside Rb₁ at a concentration of 0.00001% by weight is thought to be almost equivalent to about 100 ng/g or 100 ng/ml of dihydroginsenoside Rb₁. Judging from these facts, the concentrations of dihydroginsenoside Rb₁ to act as a regeneration and reconstruction promoter(s) for skin tissue are preferably low as described in PCT/JP00/04102 (Brain cell or nerve cell-protecting agents comprising ginseng) , and more concretely, the extracellular fluid concentrations of dihydroginsenoside Rb₁ in lesioned region are 100 ng/ml or less, preferably 10 pg/ml or less, more preferably 100 fg/ml or less. In case of using ginsenoside derivatives, especially dihydroginsenoside Rb₁, of the present invention as a preparation(s) for intravenous administration or a preparation(s) for external or topical application to skin, the preparation(s) is preferably adjusted so that the extracellular fluid concentrations of ginsenoside derivatives, especially dihydroginsenoside Rb₁, in lesioned tissues of patients are kept at the above described levels. Sufficient effectivity of the pharmaceutical composition(s) or preparation(s) of the present invention can be obtained, if the extracellular concentrations in lesioned tissues are about 0.01 - 100 fg/ml or less (e.g. 0.00001 fg/ml). Further, in case of directing prevention, treatment or improvement of senile symptoms of skin or mucosa (atrophy, shrinkage, dermatrophia, vulnerability to infection, easy infectivity, slackening, looseness, flabbiness, scurf, dandruff, depilation, alopecia, poliosis, gray hair, itching, roughness, oligosteatosis, asteatosis, keratic cell ablation, corneum ablation, exfoliation or ablation of keratinocytes, keratinized cells, cornified cells or hornified cells, exfoliation or ablation of the stratum corneum, chapping, chapped, cracks, rhagades, spots, blotches, chloasma, ephelis, lines, furrows, wrinkle, freckle, aplasia, poor regeneration, pigmentation or dryness, or shrink of mucosa, epithelial ablation or exfoliation, mucosal ablation or exfoliation, aplasia, poor regeneration, chapping, chapped or driness), the amount of ginsenoside derivatives, especially dihydroginsenoside Rb₁, admixed in cosmetics or health drugs should be adjusted in order to maintain the extracellular fluid concentrations of ginsenoside derivatives, especially dihydroginsenoside Rb₁, in the local region of the skin or in the local region of the mucosa at the low levels as described hereinbefore. Of course, although ginsenoside derivatives, especially dihydroginsenoside Rb₁, can be admixed to cosmetics together with other cosmetic compositions described in U.S. Patent 5,663,160, the said other cosmetic compositions has specific feature to be used at lower concentrations than the concentrations as described in U.S. Patent 5,663,160. Although ginsenoside derivatives, especially dihydroginsenoside Rb₁, can be admixed in every cosmetics or health drugs together with any other cosmetic composition(s) or health drug composition(s), the said other cosmetic composition(s) or said health drug composition(s) used together with ginsenoside derivatives, especially dihydroginsenoside Rb₁, should preferably be used at lower concentrations than the concentrations described in the prior references and patent specifications.

Consequently, the cosmetic composition(s) or the health drug composition(s) of the present invention can be contained or comprised in every products, which include not only the conventional cosmetics but also quasi drugs such as tooth paste and shampoo and health goods-for direct or indirect contact with surface of the vital epidermis or mucosa including oral cavity mucosa.

As shown in the examples hereinbelow, dihydroginsenoside Rb₁ suppresses apoptosis of neurons or apoptosis-like nerve cell death at almost the same low concentrations and low concentration range as those of ginsenoside Rb₁, and promotes healing of open wound in the skin by external or topical administration. Furthermore, as invented in PCT/JP00/04102 (Brain cell or nerve cell protecting agents comprising ginseng), a preparation(s) for intravenous administration comprising dihydroginsenoside Rb₁ exhibits superior therapeutic effect on cerebral infarct in almost the same dosage as that of ginsenoside Rb₁. Namely, effects, efficacy and usages of ginsenosides, especially ginsenoside Rb₁, are similar to those of ginsenosides derivatives, especially dihydroginsenoside Rb₁. Based on such estimation, effects, efficacy and usages of ginsenoside Rb₁ demonstrated in the present invention, can be applied to ginsenoside derivatives, especially dihydroginsenoside Rb₁. Consequently, ginsenoside derivatives, especially dihydroginsenoside Rb₁ can be utilized as a pharmaceutical composition(s) for promoting regeneration or reconstruction of mucosal tissues, a composition(s) for growth regulation or a fertilizer composition(s) to promote rooting, budding, growth, generation, differentiation, regeneration or reconstruction of plant tissues, a feed composition(s) or a composition(s) for growth regulation for growth, protection or cultivation of fish, shellfish, marine resources, aquatic resources, livestock or pet. Quite naturally, ginsenoside derivatives, especially dihydroginsenoside Rb₁ can be used as a preparation(s) for intravenous administration or a preparation(s) for external or topical application to mucosa as described hereinbefore. Hereinbelow these will be described.

The preparation(s) for intravenous administration of the present invention comprising ginsenoside derivatives, especially dihydroginsenoside Rb₁ can be directly applied intravascularly, preferably intravenously, and is used as a preparation(s) for single intravenous infusion or a preparation(s) for continuous intravenous infusion after dissolving them in physiological saline, distilled water, phosphate buffer, glucose solution, liposome or fat emulsion. It can also be a formulation such as a composition for drip infusion to be added to a preparation(s) for intravenous administration. Further a part(s) of chemical structure of ginsenoside derivatives, especially dihydroginsenoside Rb₁, can be modified to prepare a prodrug(s) and any route of administration and any method for administration may be selected. For example, the prodrug(s) is prepared by esterification of a hydroxyl(s) or a hydroxyl group(s) of dihydroginsenoside Rb₁, administered and hydrolyzed by endogenous esterase to work as dihydroginsenoside Rb₁ in the vital or viable tissues. The preparation(s) for external or topical application to skin or the preparation(s) for external or topical application to mucosa for prevention, treatment or therapy of organic diseases causing histopathological changes comprising ginsenoside derivatives, especially dihydroginsenoside Rb₁, of the present invention can be prepared by admixing ginsenoside derivatives, especially dihydroginsenoside Rb₁, preferably at low concentrations, into any base such as water soluble base (cream), emulsion base, combination base or fat-soluble base (ointment base). Further, as like aphtouch, low concentrations of ginsenoside derivatives, especially dihydroginsenoside Rb₁, can be admixed in a preparation(s), which adhere on mucosa. Concretely, after ginsenoside derivatives, especially dihydroginsenoside Rb₁, are admixed at the concentration of 1 g (1% by weight) or less or lower, preferably 10 mg (0.01% by weight) or less or lower, more preferably 0.1 mg (0.0001% by weight) or less or lower, and 1 fg (10⁻¹⁵% by weight) or more, per 100 g of water soluble base, emulsion base, combination base or ointment base (fat-soluble base) such as ophthalmic white petrolatum (propet), the resulted preparation(s) can be used as an agent(s) for external or topical application to skin or an agent(s) for external or topical application to mucosa for prevention, treatment or therapy of the above-described diseases. Of course, in the above-described agent(s) or preparation(s) for external or topical application to skin or agent(s) or preparation(s) for external or topical application to mucosa, in addition to ginsenoside derivatives, especially dihydroginsenoside Rb₁, any pharmaceutical composition (e.g. glucose, antibiotics, vitamin E, vitamin E derivatives, vitamin D, vitamin D derivatives, vitamins, antiviral agents, immunosuppressive agents, antiallergic agents, steroids, ginseng components, natural product components, etc.) can be admixed. Especially for allergic cutaneous mucosal diseases such as atopic dermatitis, contact dermatitis or pollinosis, if steroid(s) (preparation), antiallergic pharmaceutical composition(s) (preparation) or immunosuppressive pharmaceutical composition(s) (preparation) is admixed into the agent(s) for external or topical application to skin or the agent(s) for external or topical application to mucosa comprising ginsenoside derivatives, especially dihydroginsenoside Rb₁, excellent effect can be obtained. The agent(s) for external or topical application to skin or the agent(s) for external or topical application to mucosa comprising ginsenoside derivatives, especially dihydroginsenoside Rb₁, can be combined with the agent(s) for external or topical application to skin or the agent(s) for external or topical application to mucosa comprising any other pharmaceutical composition(s). The upper limit of concentration of ginsenoside derivatives, especially dihydroginsenoside Rb₁, in the agent(s) for external or topical application to skin or the agent(s) for external or topical application to mucosa for prevention, treatment or therapy of the above-described diseases is 10% by weight or less, preferably 1% by weight or less.

When ginsenoside derivatives, especially dihydroginsenoside Rb₁, are admixed into liquid cosmetics such as conventional UV liquid foundation, concentration thereof is adjusted to be at 100 µg/ml or less, preferably 10 ng/ml or less, more preferably 0.01 fg/ml, and if the mixture is applied externally or sprayed externally onto skin every day, the extracellular fluid concentrations of ginsenoside derivatives, especially dihydroginsenoside Rb₁, in the local region of skin can be maintained at low levels as described hereinbefore. Thus the mixture is useful for improvement, prevention of progress or prevention of deterioration of senile symptoms of skin (atrophy, shrinkage, dermatrophia, vulnerability to infection, easy infectivity, flabbiness, slackening, looseness, itching, depilation, alopecia, dandruff, scurf, poliosis, gray hair, roughness, rhagades, fissure, cracks, oligosteatosis, asteatosis, exfoliation or ablation of keratinocytes, keratinized cells, hornified cells or cornified cells, keratic cell ablation, corneum ablation, exfoliation or ablation of the stratum corneum, chapping, chapped, dryness, ephelis, chloasma, spots, blotches, lines, furrows, wrinkles, freckle, pigmentation, poor regeneration, aplasia, sunburn, etc. of skin). When ginsenoside derivatives, especially dihydroginsenoside Rb₁, are admixed into solid, gelled or creamy cosmetics, such as conventional makeup base or night cream, the amount of admixed ginsenoside derivatives, especially dihydroginsenoside Rb₁, is adjusted to 100 µg or less, preferably 10 ng or less, more preferably 0.01 fg - 100 pg per g of cream, then if the mixture is applied or sprayed externally onto skin for consecutive days, it is useful for prevention, improvement or treatment of senile symptoms of skin (atrophy, shrinkage, dermatrophia, vulnerability to infection, easy infectivity, slackening, looseness, flabbiness, itching, roughness, cracks, rhagades, oligosteatosis, fissure, asteatosis, keratic cell ablation, exfoliation or ablation of keratinocytes, keratinized cells, hornified cells or cornified cells, corneum ablation, exfoliation or ablation of the stratum corneum, chapping, chapped, dryness, dry, ephelis, spots, chloasma, blotches, wrinkle, furrows, lines, freckle, gray hair, poliosis, dandruff, scurf, alopecia, depilation, pigmentation, aplasia, poor regeneration, sunburn, etc. of skin). Proviso that when ginsenoside derivatives, especially dihydroginsenoside Rb₁, are admixed in any cosmetics and used for extremely long term, the content of ginsenoside derivatives, especially dihydroginsenoside Rb₁, per 1 g of cosmetics can be reduced to 0.0000001 fg. The upper limit of concentration of ginsenoside derivatives, especially dihydroginsenoside Rb₁, admixed in the above cosmetics for the purpose of prevention, improvement or treatment of the senile symptoms of skin, is 10 mg/ml or less in case of the liquid cosmetics, and 10 mg/g or less in case of solid, gelled or creamy cosmetics, namely 1% by weight or less. However, high concentrations of ginsenoside derivatives as like 1% by weight may damage membrane of normal cells in the skin. Namely, when ginsenoside derivatives, especially dihydroginsenoside Rb₁, are admixed into the cosmetics or health drugs, which are used for long term, it is thought to be safe to lower the optimum concentration in comparison with the concentration in the agent(s) for external application for prevention, treatment or therapy of skin diseases and mucosal diseases as previously described in the present invention. Of course, as described hereinabove, the cosmetics comprising or containing trace amount of ginsenoside derivatives, especially dihydroginsenoside Rb₁, can be applied or sprayed externally onto the face and also applied or sprayed externally to the other skin regions (e.g. extremities, trunk, neck, head, etc.) which are frequently irradiated by sun light. As described, when the cosmetics or health drugs comprising or containing ginsenoside derivatives, especially dihydroginsenoside Rb₁, are used usually for long term, symptoms accompanied by senility of the skin (shrinkage, atrophy, vulnerability to infection, easy infectivity, flabbiness, slackening, loosening, itching, roughness-, cracks, rhagades, fissure, asteatosis, oligosteatosis, keratic cell ablation, exfoliation or ablation of keratinocytes, keratinized cells, cornified cells or hornified cells, exfoliation or ablation of the stratum corneum, corneum ablation, chapped, chapping, spots, blotches, ephelis, lines, furrows, wrinkle, freckle, gray hair, poliosis, dandruff, scurf, alopecia, depilation, pigmentation, sunburn, poor regeneration, aplasia, dryness, etc. of skin) can be prevented or improved. Proviso that, the amount of ginsenoside derivatives, especially dihydroginsenoside Rb₁, admixed into the agent(s) for external or topical application to skin, agent(s) for external or topical application to mucosa, health drugs or cosmetics, is tentative value and actually the admixed amount of ginsenoside derivatives, especially dihydroginsenoside Rb₁ should be adjusted so that the extracellular fluid concentrations of ginsenoside derivatives, especially dihydroginsenoside Rb₁, are kept at 100 ng/ml or less, preferably 10 pg/ml or less, more preferably 100 fg/ml or less. Of course, senility of mucosa (shrinkage, atrophy, epithelial exfoliation or ablation, mucosal exfoliation or ablation, aplasia, poor regeneration, chapped, cracks, chapping, dryness, etc.) can be prevented, improved or reduced by admixing ginsenoside derivatives, especially dihydroginsenoside Rb₁, into health drugs (e.g. gargle, eye wash, water for health, health beverage or health food, etc.) at low concentrations.

As like ginsenoside Rb₁, ginsenoside derivatives, especially dihydroginsenoside Rb₁, of the present invention are thought to be able to expedite wound healing as a result of promoting regeneration and/or reconstruction of defected skin tissue by continuous intravenous administration for about one week after development of open wound or defect, or by external application or spray onto the skin once or consecutively in every day. Dihydroginsenoside Rb₁ of the present invention is thought to be able to expedite, as like ginsenoside Rb₁, wound healing as a result of promoting regeneration and/or reconstruction of defected oral mucosal tissue by external application onto the wound region 1 - 10 times a day for about one week after development of morsus of the mouth mucosa. Consequently, intravenous administration, extramucosal administration or extracutaneous administration of ginsenoside derivatives, especially dihydroginsenoside Rb₁, is thought to exhibit, as like ginsenoside Rb₁, effectiveness and efficacy for prevention, therapy or treatment of the above described all skin diseases or oral diseases through regeneration and reconstruction-promoting action or wound healing-promoting action on skin tissue or mucosal tissues. The present invention, which is completed by using dihydroginsenoside Rb₁, is to demonstrate that many agents for prevention, therapy or treatment of diseases caused by injury, wound, trauma or defect of skin tissue or mouth mucosa, or diseases causing histopathological changes of skin, oral cavity and the other organs or mucosa can be prepared in future by utilizing ginsenosides, especially ginsenoside Rb₁, or metabolites thereof as a leading compound(s).

Further, intravenous continuous administration, extramucosal administration on mouth or extracutaneous administration of ginsenoside derivatives, especially dihydroginsenoside Rb₁, is thought to facilitate wound healing by promoting regeneration and/or reconstruction of skin tissue or oral mucosal tissue receiving injury, wound, trauma or defect. Based on this fact, intravenous administration or local administration of ginsenoside derivatives, especially dihydroginsenoside Rb₁, is thought to exhibit effectiveness and efficacy, as same in case of injury, wound, trauma or defect of skin tissue or oral mucosal tissue, through tissue regeneration and reconstruction-promoting action or wound healing-promoting action, for diseases caused by injury, trauma, wound or defect of abdominal or thoracic visceral organs, head and neck organs, bone, joint, ligament, muscle, blood vessel or nerve, or for all diseases causing histopathological changes of said organs. Consequently, intravenous administration, local administration during operation, nasal administration or intrarectal administration, of ginsenoside derivatives, especially dihydroginsenoside Rb₁, in low dosage or low concentration is effective for: promoting recovery of sutured wound after organ transection or incision; prevention of incomplete suture in surgical treatment and operation; prevention, therapy or treatment of peptic ulcer lesion; promoting regeneration and/or reconstruction of organ after excision or transection of liver, kidney, spleen, pancreas, lung, intestine, digestive tract, urogenital organ, endocrine gland, exocrine gland, uterus, bladder or gallbladder; promoting regeneration and/or reconstruction of tissue in reconstructive surgery of bone, joint, ligament, tendon, peripheral nerve and meninges (orthopedic surgery and neurosurgery); and prevention, therapy or treatment of injury, wound, trauma or defect of organs such as liver, kidney, spleen, pancreas, lung, intestine or urogenital system. Further, ginsenoside derivatives, especially dihydroginsenoside Rb₁, in low dosage or low concentration is effective for endogenous or exogenous diseases causing histopathological changes of the organs and tissues described hereinbefore. Such diseases include all diseases described in the book ("Today's therapy", Ed. Hinohara, Shigeaki and Abe, Masakazu, Igaku Shoin Publ., 1995; "Today's therapy", Ed. Tagasu, Yukio and Ogata, Etsuro, Igaku Shoin Publ., 2000; or "Today's therapy in orthopaedics", Ed. Ninomiya, Setsuo, Fujikawa, Ryosuke, Ochi, Takahiro and Kokubu, Shoichi. Igaku Shoin Publ., 2000). Low concentrations, low doses or low dosages of ginsenoside derivatives, especially dihydroginsenoside Rb₁, can be utilized for prevention, therapy or treatment of diseases, trauma or wound of not only human but also pet and livestock. Further, low concentrations of ginsenoside derivatives, especially dihydroginsenoside Rb₁, can be utilized for cultivation of sea products (fish and shellfish, crustacean, eel, conger, sea urchin, oyster, wakame, pearl shell, pearl oyster, etc.) and aquatic products or cultivation of farm products. In this case, ginsenoside derivatives, especially dihydroginsenoside Rb₁, are thought to protect marine resources and farm products from endocrine disrupters, toxins, trauma, microorganisms, biohazards, environmental pollution, etc. Further, in the hydroponics, ginsenoside derivatives, especially dihydroginsenoside Rb₁, can be added into water of cultivation for increased yield of vegetables.

Further, a specific feature of ginsenoside derivatives, especially dihydroginsenoside Rb₁, of the present invention, which can not be overlooked, is that they do not exhibit significant side effect or adverse effect. Actually, as indicated in PCT/JP00/04102 (Brain cell or nerve cell protecting agents comprising ginseng), as far as directing treatment or therapy of cerebral infarction by continuous intravenous administration in a dose of 6 µg/day/rat (weighing 300 g), dihydroginsenoside Rb₁ is thought to be a quite safe pharmaceutical composition. The amount of administration of ginsenoside Rb₁, which is used as the preparation(s) for external or topical application to skin or the preparation(s) for external or topical application to mucosa, is far smaller than the amount of intravenous administration. Of course, in the present experiments, as far as animals administered with dihydroginsenoside Rb₁ were carefully observed, no side effect was noted.

As shown in examples hereinbelow, by applying the agent(s) for external or topical application to skin comprising or containing dihydroginsenoside Rb₁ at concentrations of 0.00001% by weight (10⁻⁵% by weight), 0.000001% by weight (10⁻⁶% by weight) or 0.0000001% by weight (10⁻⁷% by weight) to open wound of rats, wound healing and skin tissue regeneration and/or reconstruction were obviously promoted and area of open wound region was reduced to about 1/2 - 1/4 of the control group. However, since the therapeutic effect of 0.0001% by weight (10⁻⁴% by weight) of dihydroginsenoside Rb₁ on open wound in rats was trivial, ginsenoside derivatives, especially dihydroginsenoside Rb₁, should preferably be admixed at a concentration of 0.0001% by weight or less or at lower concentrations in the agent(s) for external or topical application to skin. These facts indicate that ginsenoside derivatives, especially dihydroginsenoside Rb₁, is useful for prevention, therapy or treatment of diseases showing injury, defect or degenerative exfoliation of skin tissue (ulcer, trauma, burn, frostbite, perniosis, chilblain, congelation, ultraviolet injury, electric burn, bedsore, decubitus, wound, bullous skin diseases, etc.) and diseases causing histopathological changes of skin (e.g. diseases described in "Today's therapy in dermatology", Ed. Ikeda, Shigeo, et al, Igaku Shoin Publ., 1996, and wound, burn, radiation injury, perniosis, chilblain, congelation, frostbite, ultraviolet injury, electric injury, trauma, skin ulcer, decubitus, bedsore, contact dermatitis, bullous dermatitis, atopic dermatitis, xeroderma, autosensitization dermatitis, erythroderma, exfoliative dermatitis, epidermolysis bullosa, epidermal hydroa, photosensitivity, chronic pigmentary purpura (Schamberg's disease), strophulus, insect bite, prurigo, erythema multiforme, erythema annulare, erythema nodosum, pemphigus, pemphigoid, herpetic dermatitis, palmoplantar pustulosis, psoriasis, lichen planus, ichthyosis, lichen pilaris, xanthomatosis, cutaneous amyloidosis, herpes simplex, viral wart, molluscum contagiosum, pyoderma, skin tuberculosis, atypical mycobacteriosis of the skin, trichophytia, tinea, oral or cutaneous candidiasis, scabies, pediculosis, pediculosis pubis, syphilis, keloid, hypertrophic scar, angioma, hemangioma, lymphoma, nevus, vitiligo vulgaris, ephelides, chloasma, moth patches, melanosis, pompholyx, miliaria, acne vulgaris, rosacea, rosacea-like dermatitis, oral mucosa ulcer, stomatitis, perioral dermatitis, senile symptoms of skin, alopecia, depilation, perionychia, dry eye, ingrown nail, etc). Quite naturally, diseases exhibiting injury, defect, degenerative exfoliation of skin tissue are also included in diseases causing histopathological changes of skin. Of course, ginsenoside derivatives, especially dihydroginsenoside Rb₁, are, as like low concentrations, low doses and low dosages of ginsenoside Rb₁, useful for prevention, therapy or treatment of all diseases and pathological state causing histopathological changes of mucosal tissues such as diseases caused by injury, morsus, wound, burn, trauma or defect of mucosal tissues including oral mucosa, caries, pulpitis, marginal periodontitis, stomatitis, glossitis, recurrent aphtha, intraoral aphtha, halitosis, mouth odor, oral dysesthesia, odontogenic infection, oral mucosa morsus, lingual morsus, oral mucosa burn, lingual burn, lingual injury, oral mucosa injury, gingivitis, alveolar pyorrhea, catarrhal stomatitis, gangrenous stomatitis, Vincent stomatitis, aphthous stomatitis, acute herpetic gingival stomatitis, herpangina, herpes zoster, oral mucosal erosion, oral mucosal ulcer, decubitus ulcer, radiation stomatitis, pemphigus, oral candidiasis, mucosal defect, mucosal erosion, mucosal ulcer, lichen planus, Riga-Fede disease, bald tongue, red plain tongue, Sjoegren's syndrome, etc.

In PCT/JP00/04102 (Brain cell or nerve cell protecting agents comprising ginseng), we (Sakanaka, Tanaka and Nakata) have found that intravenous administration of dihydroginsenoside Rb₁ at doses of 6 µg/day - 60 µg/day in rats (weighing 300 g) with brain infarction, showed excellent therapeutic effect on brain infarction. Also, intravenous administration of ginsenoside Rb₁ at the same dosage exhibited excellent therapeutic effect on brain infarction, wound healing-promoting action or skin tissue regeneration and reconstruction-promoting action. Consequently, intravenous administration of dihydroginsenoside Rb₁ at the same dosage is likely to exhibit excellent wound healing-promoting action or skin tissue regeneration and reconstruction-promoting action. Based on the experimental results hereinbefore, an intravenous optimum dose of drug (ginsenoside derivatives, especially dihydroginsenoside Rb₁) in patient or vertebrate (estimated body weight 60 kg) suffering from diseases caused by injury, wound, trauma or defect of skin tissue or mucosal tissue or diseases causing histopathological changes of skin or mucosa is calculated to be from 1.2 mg to 12 mg a day. Consequently, in case of using the pharmaceutical composition(s) of the present invention for prevention, therapy or treatment of human skin diseases or mucosal diseases, a systemic dose per day is, though depending on individual difference or disease state of patients, 0.001 mg or more, preferably 0.1 mg or more, more preferably 1 mg or more, further more preferably 10 mg or more. However, since, generally, necessary amount of drug for administration per 1 kg body weight is decreased depending on increase in body weight of animals, the possibility is left open that an amount of 1/10 or less of that dose exhibits sufficient effectiveness and efficacy in human. The pharmaceutical composition(s) of the present invention has less adverse effect and the upper limit of administration for prevention, treatment or therapy of the skin diseases hereinbefore described can be set considerably high; it is 1 g/day or less, preferably 0.1 g/day or less. The amount of ginsenosides derivatives, especially dihydroginsenoside Rb₁, in 10 g of an agent(s) for external or topical application to skin or an agent(s) for external or topical application to mucosa can be 100 mg or less, preferably 1 mg or less, more preferably 0.001 mg or less, further more preferably 0.00001 mg or less. Namely, the amount of ginsenoside derivatives, especially dihydroginsenoside Rb₁, for external or topical administration to skin or mucosa per day for human with skin diseases or mucosal diseases is thought to be, though depending on individual difference or disease state of patients, usually 100 mg or less, preferably 1 mg or less, more preferably 0.001 mg or less, further more preferably 0.00001 mg or less.

A method for administration of the pharmaceutical composition(s) or veterinary drug composition(s) of the present invention is preferably intravascular administration, especially intravenous administration, with consecutive or continuous administration of the above described dosages. Ginsenoside derivatives, especially dihydroginsenoside Rb₁, as the active component(s) of the present invention are one of saponins and are formulated by conventional methods. For example, the water-soluble pharmaceutical composition(s) of the present invention can be intravenously administered by dissolving lyophilized crystals in physiological saline, distilled water, phosphate buffer or glucose solution. Of course, as described hereinbefore, after dissolving, it can be used by adding into the preparation(s) for intravenous administration such as a composition(s) for drip infusion. Further, it can also be used as fat emulsion, liposome preparation or sustained release preparation. The concentrations of ginsenoside derivatives, especially dihydroginsenoside Rb₁, in the preparation(s) for intravenous administration can be optionally adjusted unless so high, for example 0.001 - 100 mg/ml, preferably 0.01 - 10 mg/ml, more preferably 0.1 - 1 mg/ml. When ginsenoside derivatives, especially dihydroginsenoside Rb₁, are utilized as the preparation(s) for external or topical application to skin or the preparation(s) for external or topical application to mucosa for prevention, treatment or therapy of the above described diseases, ginsenoside derivatives, especially dihydroginsenoside Rb₁, are admixed at the concentrations of 1% by weight or less, preferably 0.01% by weight or less, more preferably 0.0001% by weight or less, further more preferably 0.000001% by weight or less, and 10⁻¹⁵% by weight or more in any base such as water soluble base, emulsion base, ointment base, combination base or fat soluble base, as described hereinbefore. Proviso that, when the preparation(s) for external or topical application to skin comprising or containing ginsenoside derivatives, especially dihydroginsenoside Rb₁, is administered for long term or administered to patients who suffer from mild skin wound, the concentration thereof can be reduced to 10⁻²⁰% by weight. The upper limit of concentration of ginsenoside derivatives, especially dihydroginsenoside Rb₁, in the agent(s) for external or topical application to skin or the agent(s) for external or topical application to mucosa is 10% by weight or less. The agent(s) for external or topical application to skin comprising ginsenoside derivatives, especially dihydroginsenoside Rb₁, can be in the form of applying to local region of skin or in the form of spray. Ginsenosides derivatives, especially dihydroginsenoside Rb₁, can be admixed in any conventional cosmetics and novel cosmetics containing any active ingredient, and the concentrations of ginsenoside derivatives, especially dihydroginsenoside Rb₁, in the cosmetics is preferably kept lower than 0.001% by weight (10⁻³% by weight), and 10⁻¹⁵% by weight or more. Proviso that if the cosmetics or health drugs comprising or containing ginsenoside derivatives, especially dihydroginsenoside Rb₁, are used for extremely long term, the concentration may be reduced to 10⁻²⁰% by weight. Further, the above described agent(s) for external or topical application to skin, agent(s) for external or topical application to mucosa, health drugs or cosmetics comprising ginsenoside derivatives, especially dihydroginsenoside Rb₁, can be externally or topically applied onto the skin or mucosa once or consecutively, or externally applied or sprayed on the skin or mucosa in a continuous manner at any time, if the extracellular fluid concentrations of ginsenoside derivatives, especially dihydroginsenoside Rb₁, in local lesions or local skin regions can be maintained at 100 ng/ml or less, preferably 10 pg/ml or less, more preferably 100 fg/ml or less.

Continuous intravenous infusion of ginsenoside derivatives, especially dihydroginsenoside Rb₁, exhibits superior effect as like ginsenoside Rb₁, but when human or animal suffering from diseases caused by injury, wound, trauma or defect of skin tissue or diseases causing histopathological changes of skin, are treated in actual general outpatient department, ginsenoside derivatives, especially dihydroginsenoside Rb₁, can be intravenously administered once a day for consecutive days until the effectiveness is observed. Alternatively, ginsenoside derivatives, especially dihydroginsenoside Rb₁, is admixed with a composition(s) of drip infusion in every consecutive day and intravenously administered for about one hour until the effectiveness is observed. Of course, among human patients with wound of skin, burn, radiation injury, pernio, chilblain, congelation, frostbite, ultraviolet injury, electric injury, trauma, skin ulcer, decubitus, bedsore, contact dermatitis, bullous dermatitis, atopic dermatitis, xeroderma, autosensitization dermatitis, erythroderma, exfoliative dermatitis, epidermolysis bullosa, epidermal hydroa, photosensitivity, chronic pigmentary purpura (Schamberg's disease), strophulus, insect bite, prurigo, erythema multiforme, erythema annulare, erythema nodosum, pemphigus, pemphigoid, herpetic dermatitis, palmoplantar pustulosis, psoriasis, lichen planus, ichthyosis, lichen pilaris, xanthomatosis, cutaneous amyloidosis, herpes simplex, viral wart, molluscum contagiosum, pyoderma, skin tuberculosis, atypical mycobacteriosis of the skin, trichophytia, tinea, oral or cutaneous candidiasis, scabies, pediculosis, syphilis, keloid, hypertrophic scar, angioma, hemangioma, lymphoma, nevus, vitiligo vulgaris, ephelides, chloasma, moth patches, melanosis, pompholyx, miliaria, acne vulgaris, rosacea, rosacea-like dermatitis, oral mucosa injury, stomatitis, perioral dermatitis, senile symptoms of skin, alopecia, depilation, perionychia, dry eye, ingrown nail, etc. and among patients before or after undergoing operation, the human patients who require medical treatment or therapy for long term may be intravenously administered with ginsenoside derivatives, especially dihydroginsenoside Rb₁, admixed, for example, with an intravenous hyperalimentation (IVH) preparation(s) for more than one month. Of course, the agent(s) for external or topical application to skin or agent(s) for external or topical application to mucosa comprising ginsenoside derivatives, especially dihydroginsenoside Rb₁, can be applied or sprayed onto local lesion for required times (approximately 1 - 10 times a day) for consecutive days for prevention, treatment or therapy of the above described diseases.

The present invention is to report initiative in the world that low dosages and low concentrations of ginsenoside derivatives, especially dihydroginsenoside Rb₁, regenerate and reconstruct epidermal tissue, connective tissue in the dermis, dermal papillae, subcutaneous tissue, hair follicles, hair papillae, pilomotor muscles, sweat glands, sebaceous glands, peripheral nerves and blood vessels of skin with open wound (defect) rapidly and nearly to the condition prior to injury. The fact that ginsenoside derivatives, especially dihydroginsenoside Rb₁, promote regeneration and/or reconstruction of epithelial (epidermal) tissue, connective tissues, blood vessels, peripheral nerves and glandular tissues indicates that ginsenoside derivatives, especially dihydroginsenoside Rb₁, is effective for regeneration and/or reconstruction of not only the skin but also the other tissues (e.g. central nervous tissue, liver, kidney, spleen, hematopoietic tissue, digestive tract, lung, pancreas, cornea, endocrine glands, exocrine glands, salivary glands, gonad, bladder, etc.). Namely, intravenous administration, intrarectal administration, local administration during surgical operation or administration to local lesion of ginsenoside derivatives, especially dihydroginsenoside Rb₁, is effective for regeneration and/or reconstruction of the liver after hepatitis, hepatectomy or hepatic ischemia and reperfusion, regeneration and/or reconstruction of the central nervous tissue after neurotrauma (head injury and spinal cord injury), regeneration, reconstruction or attachment to original position of an amputated finger(s), regeneration and/or reconstruction of the kidney after nephritis or acute tubular necrosis, regeneration and/or reconstruction of the spleen, pancreas or lung after their excision, regeneration and/or implantation of grafted bone marrow, injury to tympanic membrane, corneal injury, corneal erosion, corneal ulcer, injury or diseases of nail, peptic ulcer, regeneration and/or reconstruction of injured or damaged organs and tissues after surgical operations (thoracic or peritoneal surgical operation, orthopaedic surgery, plastic surgery, vanity surgery, gynecotocological surgery, urological surgery, ophthalmological surgery, head and neck surgery, dental oral surgery, otorhinolaryngological surgery, veterinary surgery, neurological surgery, etc.). Of course,. ginsenoside derivatives, especially dihydroginsenoside Rb₁, exhibit effectiveness and efficacy for prevention, treatment or therapy of diseases causing histopathological changes of all organs and tissues in addition to the above described diseases and trauma, through tissue regeneration and reconstruction-promoting action. Examples of diseases and pathological states, for which application of ginsenoside derivatives, especially dihydroginsenoside Rb₁, is expected, appear to be all diseases and pathological states described in the books ("Today's therapy", Ed. Hinohara, Shigeaki and Abe, Masakazu, Igaku Shoin Publ., 1995; "Today's therapy", Ed. Tagasu, Yukio and Ogata, Etsuro, Igaku Shoin Publ., 2000). Of course, even if organic diseases with unknown origin or etiology newly appear in future, and if such diseases are causing histopathological changes of vital or viable tissues, application of ginsenoside derivatives, especially dihydroginsenoside Rb₁, will be expected. The effectiveness, efficacy and usages of ginsenoside derivatives, especially dihydroginsenoside Rb₁, as described hereinabove are identical with those of ginsenoside Rb₁. As shown in examples hereinbelow, ginsenoside derivatives, especially dihydroginsenoside Rb₁, can suppress apoptosis or apoptosis-like death of cells such as nerve cells. Consequently, ginsenoside derivatives, especially dihydroginsenoside Rb₁, exhibit effectiveness and efficacy for all diseases accompanied by cell death. Diseases causing cell death are described in PCT/JP00/04102. Further, there is a description in PCT/JP00/04102 that dihydroginsenoside Rb₁ exhibits superior therapeutic effect on cerebral apoplexy and cerebral infarction by intravenous administration.

Further, in open wound (defect) of skin, quite naturally the hair follicles are rapidly exfoliated, but as a result of extracutaneous administration of low dosages and low concentrations of dihydroginsenoside Rb₁, hair restoration, hair growth and/or pilatory are obviously promoted. Judging from this fact, extracutaneous administration or external application to skin of dihydroginsenoside Rb₁ at low dosages and low concentrations can be said to regenerate, reconstruct, recover and/or repair the wound region nearly to the original healthy condition by promoting hair restoration, hair growth and/or pilatory after development of the open wound (defect of skin). Namely, it was demonstrated that low dosages or low doses of ginsenoside derivatives, especially dihydroginsenoside Rb₁ could be applied as a hair restoration and pilatory action promoter(s) or as an agent(s) for prevention of progress of depilation. Of course, ginsenoside derivatives, especially dihydroginsenoside Rb₁ can be used independently as a composition(s) for hair growth, hair restorer or pilatory after admixing them with any known base or carrier or can be used in combination with other pharmaceutical compositions (e.g. composition for promoting blood flow, composition for local stimulation, composition for activating hair follicles, antiandrogen, antiseborrheic composition, composition for keratolysis, antibiotics, galenical extract, vitamins, amino acids, etc.). Concretely, intravenous administration or local external application of low dosage, low doses and low concentrations of ginsenoside derivatives, especially dihydroginsenoside Rb₁ appears to be effective for alopecia areata, common baldness, androgenic alopecia, male pattern alopecia and/or diffuse depilation. Further, in the open wound (defect) of skin, although peripheral nerves and blood vessels distributed in the skin defect region are destroyed and incised, these peripheral nerves and blood vessels can obviously be regenerated, reconstructed, recovered and/or restored rapidly by intravenous administration or extracutaneous administration of ginsenoside Rb₁ nearly to the original healthy condition. Consequently, ginsenoside derivatives, especially dihydroginsenoside Rb₁, can be utilized as a pharmaceutical composition(s) for promoting regeneration and/or reconstruction of nervous tissues and vascular tissues. Namely, in any of pathological states of diabetic neuropathy, intervertebral disk hernia, spinal canal stenosis, spondylolysis, spondylolisthesis, spondylopathy, cervical spondylotic myelopathy, myelopathic radiculopathy, ossification of the posterior longitudinal ligament, spinal cord injury, peripheral neuropathy, compression neuropathy, head injury, neurotrauma, neuralgia, neurodegenerative diseases, peripheral nerve paralysis and cerebral apoplexy, intravenous administration, local administration, nasal administration, rectal administration, etc. of ginsenoside derivatives, especially dihydroginsenoside Rb₁, appears to exhibit effectiveness and efficacy by promoting regeneration and/or reconstruction of once injured nervous tissues. On the other hand, ginsenoside derivatives, especially dihydroginsenoside Rb₁, appear to exhibit effectiveness and efficacy, through promoting regeneration and/or reconstruction of blood vessels, for diseases with a main symptom of blood flow failure or blood flow disturbance (aortitis syndrome, collagen diseases, peripheral arterial embolism, thromboangitis obliterans, arteriosclerosis obliterans, thrombophlebitis, diabetic skin ulcer, diabetic retinopathy, diabetic nephropathy, occlusion of the central retinal artery or vein, Raynaud's disease, Raynaud's syndrome, myocardial infarct, decubitus, bedsore, pile, hemorrhoids, periproctitis, osteonecrosis, epiphysiopathy, peripheral circulation failure, angina pectoris, ischemia reperfusion injuries of liver, kidney and heart, cerebrovascular diseases, bone atrophy, malunited bone fracture, delayed cure fracture of bone, etc.). The effects, efficacy and usages of ginsenoside derivatives, especially dihydroginsenoside Rb₁, described hereinabove coincide with those of ginsenoside Rb₁.

As described hereinbefore, the fact that the extracutaneous spread of dihydroginsenoside Rb₁, promotes regeneration and/or reconstruction of cutaneous epidermal tissue, connective tissue in the dermis, dermal papillae, subcutaneous tissue, blood vessels, pilomotor muscles, sebaceous glands, sweat glands, hair papillae, hair follicles, etc., demonstrates quite naturally that extracutaneous spread of ginsenoside derivatives, especially dihydroginsenoside Rb₁ promotes regeneration and/or reconstruction of epidermal cells, epidermal keratinocytes, melanocytes, Merkel cells, Langerhans cells, corneocytes, hornified cells, cornified cells, keratinized cells, fibroblasts in the dermis and subcutaneous tissue, vascular endothelial cells, vascular smooth muscle cells, sebaceous gland cells, sweat gland cells, pilomotor muscular cells, hair follicle cells, mesenchymal cells, myoepithelial cells, skin stem cells, collagen fibers, elastic fibers, reticular fibers, extracellular or intercellular matrix (matrices), etc. Namely, ginsenoside derivatives, especially dihydroginsenoside Rb₁, are thought to promote regeneration and/or reconstruction of all cells and secretion thereof which constitute skin tissues. On the other hand, various symptoms of skin accompanied by aging (atrophy, shrinkage, dermatrophia, vulnerability to infection, easily infectivity, flabbiness, loosening, slackening, itching, roughness, cracks, rhagades, fissure, asteatosis, oligosteatosis, keratic cell ablation, corneum ablation, exfoliation or ablation of keratinocytes, hornified cells, cornified cells or keratinized cells, exfoliation or ablation of the stratum corneum, chapped, chapping, spots, blotches, chloasma, ephelis, wrinkles, furrows, lines, freckle, poliosis, gray hair, depilation, alopecia, dandruff, scurf, pigmentation, sunburn, poor regeneration, aplasia, dryness, etc.) are thought to occur due to gradual death or dysfunction of the above skin tissue-constituting cells, which are damaged by ultraviolet ray or vital senility and impossible to regenerate to the original healthy condition. For example, roughness, dryness, depilation, alopecia, keratic cell ablation, corneum ablation, exfoliation or ablation of keratinocytes, hornified cells, cornified cells or keratinized cells, exfoliation or ablation of the stratum corneum, chapping, chapped, asteatosis, oligosteatosis, itching, etc. accompanied by aging and senility are thought to develop due to poor regeneration after dysfunction or death of sweat gland cells, hair follicle cells and sebaceous gland cells in the skin. Further, sunburn, pigmentation, spots, blotches, chloasma, ephelis, freckle, etc. can be produced due to insufficient regeneration of cells to the original state, even after skin cells, which are irradiated by sun light or ultraviolet ray, are gone to death. Further, it can be said that lines, wrinkles, furrows, flabbiness, shrinkage, slackening, looseness, atrophy, etc. of skin are generated as a result that fibroblasts or mesenchymal cells in the dermis and subcutaneous tissue fall into dysfunction or decrease in number depending on the aging and the dermis or subcutaneous tissue can not maintain sufficient collagen fibers, elastic fibers, reticular fibers and/or extracellular matrix (matrices). On the other hand, poliosis or gray hair may be increased as a result of functional disorder of melanocytes. Further, as a result of dysfunction of Langerhans cells, immunofunction of the skin is reduced to develop easy infectivity or vulnerability to infection. Since low concentrations, low doses and/or low dosages of ginsenoside derivatives, especially dihydroginsenoside Rb₁, of the present invention can promote regeneration and/or reconstruction of all cells constituting skin tissue and secretory components thereof, if ginsenoside derivatives, especially dihydroginsenoside Rb₁, are utilized as a cosmetic composition(s), various symptoms caused by decrease in the constituting cells of skin (cell death) and dysfunction accompanied by aging (shrinkage, atrophy, easy infectivity, vulnerability to infection, looseness, slackening, flabbiness, itching, roughness, fissure, cracks, rhagades, asteatosis, oligosteatosis, corneocyte ablation, horny layer ablation, exfoliation or ablation of keratinocytes, hornified cells, cornified cells or keratinized cells, exfoliation or ablation of the stratum corneum, chapped, spots, blotches, ephelis, chloasma, lines, furrows, wrinkles, freckle, alopecia, depilation, gray hair, poliosis, dandruff, scurf, pigmentation, sunburn, poor regeneration, aplasia, dryness, etc.), can be prevented, reduced or improved. Further, dihydroginsenoside Rb₁ is, as like ginsenoside Rb₁, thought to protect all skin cells including epidermal cells, epidermal keratinocytes, i.e. keratinocytes, Langerhans cells, Merkel cells, cornified cells, hornified cells, keratinized cells, corneocytes, sebaceous gland cells, hair follicle cells, sweat gland cells, fibroblasts, stem cells, mesenchymal cells, vascular endothelial cells, pilomotor muscular cells, vascular smooth muscle cells, adipocytes, etc., consequently, it may also be able to prevent aging-induced death and functional disorder of skin-constituting cells. As explained hereinabove, ginsenoside derivatives, especially dihydroginsenoside Rb₁, not only protect all cells constituting the skin but also facilitate regeneration and/or reconstruction of the cells even if once cells of skin are going to death or fall into dysfunction. Consequently, they can prevent, improve or reduce senile symptoms of the skin accompanied by aging (shrinkage of skin, atrophy, vulnerability to infection, easy infectivity, flabbiness, looseness, slackening, itching, roughness, cracks, rhagades, fissure, oligosteatosis, asteatosis, exfoliation or ablation of keratinocytes, hornified cells, cornified cells or keratinized cells, exfoliation or ablation of the stratum corneum, corneocyte ablation, horny layer ablation, chapped, chap, spots, blotches, ephelis, wrinkles, lines, furrows, freckle, alopecia, depilation, gray hair, poliosis, dandruff, scurf, pigmentation, sunburn, poor regeneration, aplasia, dryness, etc.). Namely, ginsenoside derivatives, especially dihydroginsenoside Rb₁, can be said to improve, prevent or reduce senile symptoms of the skin accompanied by aging through potent two actions : cytoprotective action and tissue and cell regeneration-promoting action. Moreover, as demonstrated in the experimental results of the present invention, ginsenoside derivatives, especially dihydroginsenoside Rb₁, exhibit cytoprotective action and tissue and cell regeneration- promoting action, when the extracellular fluid concentrations in lesioned tissues and cutaneous tissues are 100 ng/ml or less, preferably 10 pg/ml or less, more preferably 100 fg/ml or less. Of course, ginsenoside derivatives, especially dihydroginsenoside Rb₁, are, as like ginsenoside Rb₁, useful as a health drug composition(s) or a composition(s) for external application to mucosa in order to prevent, improve or reduce senile symptoms of mucosae (shrinkage, atrophy, epithelial ablation or exfoliation, mucosal ablation or exfoliation, aplasia, poor regeneration, chapping, chapped, cracks, rhagades, dryness, etc.).

Consequently, when trace amount of ginsenoside derivatives, especially dihydroginsenoside Rb₁, is admixed into every cosmetics or drug for health (cosmetic lotion (skin lotion), beauty liquid, agent for massage, agent for pack, emulsion, milky lotion, foundation cream, hand cream, cold cream, lotion, gel, emulsion, body milk, hair dye, hair manicure, eye shadow, cleansing cream, cleansing foam, night cream, beauty cream, troches, candy for cough, water for health, isotonic water, ice, sherbet, ice cream, sweet, candy, face powder, eye wash, cleansing liquid, collyrium, lipstick, cosmetic soap, gargle, shampoo, hair rinse, tooth paste, tooth powder, bath gel, lip cream, hair tonic, hair liquid, makeup base, UV liquid foundation, powder foundation, etc.) and used to maintain the extracellular fluid concentrations of ginsenoside derivatives, especially dihydroginsenoside Rb₁, in local region of skin or local region of mucosa at the low concentrations hereinbefore, excellent effectiveness can be exhibited on senile symptoms of the skin accompanied by aging (shrinkage, atrophy, easy infectivity, vulnerability to infection, looseness, slackening, flabbiness, itching, roughness, fissure, cracks, rhagades, asteatosis, oligosteatosis, epithelial exfoliation or ablation, mucosal exfoliation or ablation, corneocyte ablation, exfoliation or ablation of keratinocytes, cornified cells, hornified cells or keratinized cells, exfoliation or ablation of the stratum corneum, horny layer ablation, chapped, chap, spots, blotches, ephelis, chloasma, wrinkles, lines, furrows, freckle, alopecia, depilation, gray hair, poliosis, dandruff, scurf, pigmentation, sunburn, poor regeneration, aplasia, dryness, etc.). For example, only shortage or defect of skin fat (i.e. sebum) accompanied by aging or senility causes roughness, itching, fissure, cracks, rhagades, corneocyte ablation, exfoliation or ablation of keratinocytes, cornified cells, hornified cells or keratinized cells, exfoliation or ablation of the stratum corneum, horny layer ablation, chapped, chapping, dryness, etc., but as a result of applying every cosmetics admixed with ginsenoside derivatives, especially dihydroginsenoside Rb₁, at low concentrations, protection or regeneration and/or reconstruction of the sebaceous glands are promoted and the above-described senile symptoms of skin accompanied by aging are thought to be prevented, improved or reduced. Since any cosmetics comprising or containing low concentrations of ginsenosides derivatives, especially dihydroginsenoside Rb₁, not only protect epidermal cells (epidermal keratinocytes, cornified cells, hornified cells, keratinized cells, and corneocytes) but also facilitate regeneration thereof, as the results of promoting production and secretion of natural humectant factors and lipids between keritnocytes, hornified cells, cornified cells or keratinized cels, the cosmetics can suppress dryness and roughness of the skin to provide natural moisture in the skin. The low concentrations of ginsenoside derivatives, especially dihydroginsenoside Rb₁, can be admixed inone type or more than two types of reagents or agents for administration, which are used in the total process of chemical peeling (before, during and/or after chemical peeling) as a composition(s) for promoting regeneration and/or reconstruction of skin tissue in the chemical peeling. Ginsenoside derivatives, especially dihydroginsenoside Rb₁, are useful for prevention, treatment and/or improvement of senility or diseases of skin by using as bath gel in nursing facilities, hot spa health facilities, hospitals, etc. Mixing ginsenoside derivatives, especially dihydroginsenoside Rb₁, with dressing agent, antiseptics, washing lotion, plaster, coating agents, etc. promotes wound healing and prevents deterioration of wound. The base(s) for dihydroginsenoside Rb₁ and the composition(s) for external or topical application to skin, which can be used in combination with dihydroginsenoside Rb₁, are the same as in ginsenoside Rb₁.

Ginsenoside derivatives, especially dihydroginsenoside Rb₁, of the present invention can promote, as like ginsenoside Rb₁, rooting, budding, generation, growth, regeneration or reconstruction of plant tissues. When ginsenoside derivatives, especially dihydroginsenoside Rb₁, are used independently or together with other effective components, as a fertilizer composition(s) or a composition(s) for growth regulation in hydroponics, the concentrations of ginsenoside derivatives, especially dihydroginsenoside Rb₁, in solution of the hydroponics (i.e. extracellular fluid concentrations in plant tissue) are adjusted to 100 ng/ml or less, preferably 10 pg/ml or less, more preferably 100 fg/ml or less. Ginsenoside derivatives, especially dihydroginsenoside Rb₁, of the present invention promote sufficiently rooting, budding, growth, differentiation, regeneration, generation or reconstruction of plant tissues in the hydroponics, even in the concentration range about 0.0001 - 0.01 fg/ml, and are utilized for preservation, growth, cultivation or improvement of all farm products and plants including vegetables, fruits and fresh flowers. Further, when ginsenoside derivatives, especially dihydroginsenoside Rb₁, are added or admixed into any solid fertilizer, gel fertilizer, liquefied fertilizer, liquid fertilizer, powdery fertilizer (e.g. straight fertilizer, soil amendment matter or fertilizer, seedling fertilizer, fertilizer for paddy and barley, slow-release fertilizer, high-analysis compound fertilizer, low-analysis compound fertilizer, organic compound fertilizer, NK-PK-PM compound fertilizer, organic mixed fertilizer, liquid fertilizer, etc.), concentrations thereof are, as same in the cosmetics , preferably set to 1% by weight or less or lower, 10⁻²⁰% or more. The upper limit of concentrations of ginsenoside derivatives, especially dihydroginsenoside Rb₁, as the fertilizer composition(s) is 10% by weight or less.

As described hereinbefore, it can be said that the extracellular fluid concentrations of dihydroginsenoside Rb₁, which promotes generation, regeneration or reconstruction of plant tissues, are extremely low as like in case of regeneration and reconstruction of animal tissues (skin tissue). Consequently, the low dosages, low doses and/or low concentrations of ginsenoside derivatives, especially dihydroginsenoside Rb₁, can be applied for cultivation, growth or preservation of plant, preservation, cultivation or growth of fresh flower, hydroponics, cultivation or growth of farm products, cultivation or growth of vegetables, cultivation and/or growth of fruits, cultivation and/or growth of tobacco, cultivation of mushrooms, cultivation of medicinal plants or cultivation and/or growth of tea-leaves. The fertilizer composition comprising ginsenoside derivatives, especially dihydroginsenoside Rb₁, can be admixed to any fertilizers, preferably at low concentrations, or can be used as a promoter(s) of rooting, budding, generation, regeneration, growth or reconstruction of plant tissues independently.

The speculation that ginsenoside derivatives, especially dihydroginsenoside Rb₁, promote rooting, budding, generation, regeneration or reconstruction of plant tissues in addition to animal tissues (skin tissue and mouth mucosal tissue) as like ginsenoside Rb₁, indicates that ginsenoside derivatives including dihydroginsenoside Rb₁ promote generation, regeneration or reconstruction of all vital tissues or viable tissues. Consequently, ginsenoside derivatives, especially dihydroginsenoside Rb₁, can be used as a additive(s) for feeds for livestock, cultivation of fish and shellfish and pet animals, i.e. a feed composition(s) or a composition(s) for growth regulation. For example, in the cultivation of fish and shellfish, crustacean, eel, sea urchin, oyster, conger, pearl shell, pearl oyster, etc., the addition of low concentrations of ginsenoside derivatives, especially dihydroginsenoside Rb₁, to sea water or fresh water together with conventional feeds is thought to promote growth of these aquatic or marine resources. Of course, ginsenoside derivatives, especially dihydroginsenoside Rb₁, can protect marine and aquatic resources such as fish and shellfish, crustacean, eel, conger, etc. from trauma, wound, pathogenic microorganisms, biohazards, endocrine disrupters, environmental pollution, toxins, etc. through their cytoprotective actions. Namely, feed additives of the present invention will be essential for secure human from forthcoming food crisis.

Next, embodiments of the present invention are described in more details as follows.

The present invention provides a pharmaceutical composition(s) for therapy, prevention or treatment of diseases caused by injury or defect of skin or mucosa, or diseases causing histopathological changes of skin or mucosa comprising an agent(s) for intravenous administration, an agent(s) for external application to mucosa or an agent(s) for external application to skin, containing ginsenosides, especially ginsenoside Rb₁, at low concentrations. Examples of the diseases caused by injury or defect of skin are trauma, chilblain, congelation, frostbite, bruise, crushed injury, wound, burn, radiation injury, ultraviolet injury, electric injury, skin ulcer, incised wound, open wound, decubitus, bedsore, bullous skin diseases, xeroderma, etc. Further, examples of the diseases causing histopathological changes of skin are wound, burn, radiation injury, chilblain, congelation, frostbite, pernio, ultraviolet injury, electric injury, trauma, skin ulcer, decubitus, bedsore, bullous dermatitis, contact dermatitis., atopic dermatitis, stagnation dermatitis, xeroderma, autosensitization dermatitis, erythroderma, exfoliative dermatitis, epidermolysis bullosa, epidermal hydroa, photosensitivity, progressive pigmented purpuric dermatosis (Schamberg disease), strophulus, insect-bite, prurigo, erythema multiforme, erythema annulare, erythema nodosum, pemphigus, pemphigoid, herpetic dermatitis, palmoplantar pustulosis, psoriasis, lichen planus, ichthyosis, lichen pilaris, xanthomatosis, cutaneous amyloidosis, herpes simplex, viral wart, molluscum pendulum, molluscum contagiosum, pyoderma, skin tuberculosis, atypical mycobacteriosis of the skin, trichophytia, tinea, cutaneous or oral candidiasis, scabies, pediculosis pubis, syphilis, keloid, hypertrophic scar, hemangioma, lymphoma, nevus, vitiligo vulgaris, ephelides, chloasma, moth patches, melanosis, pompholyx, miliaria, acne vulgaris, rosacea, rosacea-like dermatitis, oral mucosa injury, stomatitis, perioral dermatitis, senile symptoms of skin, alopecia, depilation, perionychia, ingrown nail, etc. The above-described excellent preventive, treatment or therapeutic effect of ginsenosides, especially ginsenoside Rb₁ can be provided by skin tissue regeneration and reconstruction-promoting action or wound healing-promoting action. If ginsenosides, especially ginsenoside Rb₁, of the present invention are administered intravenously or extracutaneously before and/or after operation in patient with diabetes, aged person, patient with immune deficient disease, patient with AIDS, patient with low nutrition or patient with cancer, cure of operated wound is promoted and regeneration and reconstruction of tissue are promoted and insufficient suture is prevented, thereby early recovery will be expected. Of course, patients with good general condition may receive intravenous administration or local cutaneous administration of ginsenosides, especially ginsenoside Rb₁, before and/or after operation. Local or external administration of ginsenosides, especially ginsenoside Rb₁, during operation will be very useful. Examples of diseases caused by injury, morsus, wound, burn, trauma or defect of oral mucosa or diseases and pathological states causing histopathological changes of mucosal tissues are caries, pulpitis, marginal periodontitis, stomatitis, glossitis, recurrent aphtha, intraoral aphtha, halitosis, mouth odor, oral abnormal sensation, oral dysesthesia, odontogenic infection, oral mucosa morsus, lingual morsus, oral mucosa burn, lingual burn, lingual injury, oral mucosa injury, gingivitis, alveolar pyorrhea, catarrhal stomatitis, gangrenous stomatitis, Vincent stomatitis, aphthous stomatitis, acute herpetic gingival stomatitis, herpangina, herpes zoster, oral mucosal erosion, oral mucosal ulcer, decubitus ulcer, radiation stomatitis, pemphigus, oral candidiasis, mucosal defect, mucosal erosion, mucosal ulcer, lichen planus, Riga-Fede disease, bald tongue, red plain tongue, Sjbgren's syndrome, etc., and ginsenosides, especially ginsenoside Rb₁, are useful for prevention, therapy or treatment of all of these diseases and pathological states. Of course, ginseng, a ginseng extract(s), a crude saponin fraction(s) of ginseng and ginsenoside derivatives, especially dihydroginsenoside Rb₁, exhibit the same effects, usages or efficacy as those of ginsenosides, especially ginsenoside Rb₁ of the present invention.

Further, intravenous administration, external administration to mucosae or external administration to skin of ginsenosides, especially ginsenoside Rb₁, at low concentrations, low doses and/or low dosages is thought to expedite wound healing by promoting regeneration and/or reconstruction of skin tissue or oral mucosal tissue receiving injury, trauma, wound or defect, and thus based on this fact, intravenous administration of ginsenosides, especially ginsenoside Rb₁, is to exhibit effectiveness and efficacy, as same in case of injury, wound, trauma or defect of skin tissue, through tissue regeneration and/or reconstruction-promoting action or wound healing-promoting action, for diseases caused by injury, trauma, wound or defect of abdominal or thoracic visceral organs, brain and nerves, organs in head and neck, bones, joints, ligaments, muscles, blood vessels or nerves. Consequently, intravenous administration, nasal administration, eye drop administration, sublingual administration, inhalation, local administration or intrarectal administration, of ginsenosides, especially ginsenoside Rb₁, is effective for: promoting recovery of incision and suture of organ and prevention of incomplete suture in surgical operation; prevention, therapy or treatment of peptic ulcer lesion; promoting regeneration and/or reconstruction of organ after excision of liver, kidney, spleen, pancreas, lung, intestine, uterus, urinary bladder or gallbladder; promoting regeneration and/or reconstruction of tissue in reconstructive operations of bones, joints, ligaments, tendons, peripheral nerves (orthopedic surgery); and prevention, therapy or treatment of injury, wound, trauma or defect of organs such as liver, kidney, spleen, pancreas, lung, intestine, urogenital organs and endocrine organs. For example, in finger tip injury, it is possible for ginsenosides, especially ginsenoside Rb₁, to facilitate regeneration of skin, nail, connective tissue, bone, blood vessels or nerves. Of course, ginsenosides, especially ginsenoside Rb₁, exhibit effectiveness and efficacy for organic diseases causing histopathological changes in all organs and tissues through tissue regeneration and reconstruction-promoting action. Further, ginseng, a ginseng extract(s), a crude saponin fraction(s) of ginseng containing ginsenosides, especially ginsenoside Rb₁, and dihydroginsenoside Rb₁ have effectiveness, efficacy and usages similar to those of ginsenoside Rb₁.

The present invention is to report initiative in the world that low dosages and/or low concentrations of ginsenosides, especially ginsenoside Rb₁, regenerate and reconstruct epidermal tissue, epithelial tissue, connective tissue in the dermis, dermal papilla, subcutaneous tissue, lamina propria, pilomotor muscles, hair follicles, salivary glands, hair papillae, sweat glands, mixed glands, sebaceous glands, muscular tissues, peripheral nerves and blood vessels of skin or oral mucosa with incised wound, open wound (defect) and morsus rapidly and nearly to the condition prior to injury. The fact that ginsenosides, especially ginsenoside Rb₁, promote regeneration and/or reconstruction of epidermis (stratified squamous epithelium), connective tissue, peripheral nerves and glandular tissues indicates that ginsenoside Rb₁ is effective for regeneration and/or reconstruction of not only the skin but also the other tissues and organs (e.g. liver, kidney, spleen, hematopoietic tissue, brain, spinal cord, peripheral nerve, digestive tract, lung, pancreas, cornea, salivary gland, gonad, urinary organs, etc.). Namely, intravenous administration, local administration during operation, intrarectal administration, sublingual administration, inhalation administration, external or topical application to skin, external or topical application to mucosa, nasal drop administration or local administration to lesion of ginsenosides, especially ginsenoside Rb₁, appears to be effective for: regeneration and/or reconstruction of liver after hepatitis, hepatectomy and/or hepatic ischemia and reperfusion; regeneration and/or reconstruction of kidney after nephritis and/or acute tubular necrosis; regeneration and/or reconstruction of spleen after splenectomy; regeneration, reconstruction and/or attachment to the original position of amputated finger; reconstruction and/or successful graft of transplanted organ, tissue, cells and bone marrow; regeneration and/or reconstruction of tympanic membrane after injury; regeneration and/or reconstruction of cornea after injury; prevention, therapy or treatment of corneal erosion, corneal ulcer, corneal herpes or injury of nail; prevention, therapy or treatment of peptic ulcer; menopause symptoms; regeneration and/or reconstruction of organs or tissues injured by surgical operations (thoracic surgery, peritoneal surgery, orthopedic surgery, plastic surgery, gynecotocological surgery, urological surgery, vanity surgery, ophthalmological surgery, head and neck surgery, otorhinolaryngological surgery, veterinary surgery, dental oral surgery, neurological surgery, etc.). Especially, the preventive effect of ginsenosides, especially ginsenoside Rb₁, on insufficient suture is thought to be extremely large. Of course, the low concentrations, low doses and/or low dosages of ginsenosides, especially ginsenoside Rb₁, can be administered as a veterinary drug composition(s) for not only human but also pet, livestock and vertebrates with diseases hereinabove. Further, ginseng, a ginseng extract(s) or a crude saponin fraction(s) of ginseng containing ginsenosides and ginsenoside derivatives, especially dihydroginsenoside Rb₁, have also the same effectiveness, efficacy and usages as those of ginsenosides, especially ginsenoside Rb₁.

Further, in open wound (defect) of skin, quite naturally the hair follicles are rapidly exfoliated, but as a result of intravenous administration or external application onto the skin of ginsenosides, especially ginsenoside Rb₁, at low dosages and low concentrations, hair restoration, hair growth and/or pilatory in open wound are obviously promoted. Judging from this fact, intravenous administration or external application onto skin of ginsenosides, especially ginsenoside Rb₁, in low dosage and low concentration can be said to recover and repair the wound region nearly to the original healthy condition by promoting hair restoration, hair growth and/or pilatory action in the region of the open wound (defect of skin). Namely, the low dosages and/or low concentrations of ginsenosides, especially ginsenoside Rb₁, can be applied as a promoter(s) of hair restoration, hair growth and/or pilatory action or as an agent(s) for prevention of progress of depilation or alopecia. Concretely, intravenous administration, local administration, external application or external spray of ginsenosides, especially ginsenoside Rb₁, is effective for alopecia areata, male pattern alopecia, androgenic alopecia and/or diffuse depilation. Further, in the open wound (defect) of skin and morsus of mouth mucosa, although peripheral nerves and blood vessels distributed in the skin or mucosal defected region are destroyed and incised, these peripheral nerves and blood vessels can obviously be regenerated, reconstructed and recovered rapidly by intravenous administration or external application onto skin of ginsenosides, especially ginsenoside Rb₁, nearly to the original healthy condition. Consequently, ginsenosides, especially ginsenoside Rb₁, can be utilized as a pharmaceutical composition(s) for promoting regeneration and/or reconstruction of nervous tissues and vascular tissues. Namely, in any of pathological states of diabetic neuropathy, intervertebral disk hernia, spinal canal stenosis, spondylolysis, spondylolisthesis, spondylopathy, cervical spondylotic myelopathy, ossification of the posterior longitudinal ligament, facial nerve paralysis, spinal cord injury, head injury, neurotrauma, nerve injury, neurodegenerative diseases, peripheral nerve paralysis, neuralgia and cerebral apoplexy, intravenous administration, nasal administration, rectal administration or local administration of ginsenosides such as ginsenoside Rb₁ can exhibit effectiveness and efficacy by promoting regeneration and/or reconstruction of once injured nervous tissues. On the other hand, ginsenosides, especially ginsenoside Rb₁, appear to exhibit effectiveness and efficacy for diseases with a main symptom of blood flow failure or blood flow disorder (aortitis syndrome, collagen diseases, peripheral embolism, thromboangitis obliterans, arteriosclerosis obliterans, thrombophlebitis, diabetic retinopathy, diabetic nephropathy, occlusion of the central retinal artery or vein, Raynaud's disease, Raynaud's syndrome, myocardial infarction, bedsore, decubitus, peripheral circulation failure, angina pectoris, ischemia reperfusion injuries of liver, kidney and heart, pile, hemorrhoids, cerebrovascular diseases, etc.) through promoting regeneration and/or reconstruction of blood vessels.

Of course, as described in Japanese Patent Appln. No. Hei 10-365560 and PCT/JP99/02550 (Brain cell or nerve cell protective agents comprising ginsenoside Rb₁), it is the efficacy of ginsenosides, especially ginsenoside Rb₁, to suppress cell death in the tissue exposed to blood flow disturbance in diseases with a main symptom of blood flow disturbance. Consequently, in the diseases of either central or peripheral origin with blood flow disturbance, ginsenoside Rb₁ reduces damage of tissues or cells exposed to blood flow disturbance through at least two mechanisms of action. Further, ginseng, a ginseng extract(s) or a crude saponin fraction(s) of ginseng containing ginsenosides and ginsenoside derivatives, especially dihydroginsenoside Rb₁, have also the same effectiveness, efficacy and usages as those of ginsenosides, especially ginsenoside Rb₁.

As described in Japanese Patent Appln. No. Hei 10-365560, PCT/JP99/02550 (Brain cell or nerve cell protective agents comprising ginsenoside Rb₁), ginsenoside Rb₁ is thought to exhibit tissue and cell-protective action through an increase in the expression of a cell death suppressive gene product Bcl-x_{L}. Judging from this fact, the low concentrations of ginsenosides, especially ginsenoside Rb₁, are effective for protection, preservation and/or maintenance of cultured keratinocyte sheets for skin graft. Further, ginsenosides, especially ginsenoside Rb₁, appear to be useful for preservation, protection and/or maintenance of cells for preparation of cultured skin, preservation, protection and/or maintenance of stem cells for preparation of artificial organs, and preservation, protection and/or maintenance of organs, tissues or cells for transplantation (liver, kidney, heart, spleen, lung, meninx, bone, joint, ligament, digestive tract, cornea, skin, blood vessel, peripheral nerve, etc.). Further, in the cultured skin graft, if external application to skin of ginsenosides, especially ginsenoside Rb₁, is combined, result of transplantation is improved. Of course, as a result of any method of administration of ginsenosides, especially ginsenoside Rb₁, before, during or after transplantation operation to the recipient, regeneration, reconstruction or generation of organs, tissues or cells for transplantation can be promoted. Further, ginsenosides, especially ginsenoside Rb₁, are effective for protection, maintenance, regeneration and/or generation of blood cells and platelets for transfusion, preservation, protection, maintenance, regeneration, reconstruction and/or generation of frozen cells (sperms, ova, stem cells, etc.) or frozen cultured skin sheet. As such, ginsenosides, especially ginsenoside Rb₁, can be applied for promotion of proliferation, regeneration, differentiation or successful graft of organs, tissues or cells in tissue engineering aiming at regeneration medicine. Ginsenosides, especially ginsenoside Rb₁, having potent cytoprotective action and tissue regeneration-promoting action are useful for culture of marine products (fish and shellfish, crustacean, eel, conger, etc.) and cultivation of farm products. In this case, ginsenosides, especially ginsenoside Rb₁, are thought to protect marine resources and farm products from endocrine disrupters, toxins, trauma, microorganisms, microbes, biohazards, environmental pollution, etc. Further, the low concentrations and/or low dosages of ginsenosides, especially ginsenoside Rb₁, can be utilized as a heath drug(s) for prevention, improvement or treatment of senile symptoms of mucosa (atrophy, shrinkage, epithelial exfoliation, mucosal exfoliation, chapped, chapping, poor regeneration, aplasia, dryness, etc.). Ginseng, a ginseng extract(s) or a crude saponin fraction(s)of ginseng containing ginsenosides and ginsenoside derivatives, especially dihydroginsenoside Rb₁, have also the same effectiveness, efficacy and usages as those of ginsenosides, especially ginsenoside Rb₁.

Since ginsenosides, especially ginsenoside Rb₁ of the present invention can promote regeneration and/or reconstruction of degenerated and exfoliated skin tissue after wound, open wound, incised wound or injury, quite naturally, degenerated and exfoliated skin tissue after exposure to ultraviolet ray can also be regenerated and reconstructed to the original healthy condition by ginsenosides, especially ginsenoside Rb₁. Generally, senility of skin is known to be caused by exfoliation and/or functional failure of skin cells accompanied by UV light irradiation or aging; as a result, atrophy, shrinkage, vulnerability to infection, easy infectivity, looseness, slackening, flabbiness, cracks, rhagades, fissure, itching, roughness, oligosteatosis, asteatosis, keratic cell ablation, exfoliation or ablation of keratinocytes, cornified cells, hornified cells or keratinized cells, exfoliation or ablation of the stratum corneum, corneum ablation, chapped, chapping, spots, blotches, chloasma, ephelis, lines, furrows, wrinkles, freckle; gray hair, poliosis, dandruff, scurf, alopecia, depilation, pigmentation, sunburn, dryness, etc. are deteriorated with aging. Consequently, intravenous administration, external application or spray onto skin of ginsenosides, especially ginsenoside Rb₁, at low concentrations, low doses or low dosages promotes regeneration and/or reconstruction of degenerated and exfoliated skin tissues accompanied by UV irradiation or aging, as a result, is effective for prevention, treatment or therapy of atrophy, shrinkage, vulnerability to infection, easy infectivity, slackening, looseness, flabby, flabbiness, itching, roughness, oligosteatosis, asteatosis, keratic cell ablation, exfoliation or ablation of keratinocytes, cornified cells, hornified cells or keratinized cells, exfoliation or ablation of the stratum corneum, corneum ablation, chapped, chapping, spots, blotches, ephelis, lines, furrows, wrinkles, freckle, gray hair, poliosis, dandruff, scurf, alopecia, depilation, pigmentation, sunburn, poor regeneration, aplasia, dryness, etc. accompanied by aging or senility. Namely, the low concentrations of ginsenosides, especially ginsenoside Rb₁, can be. utilized as a raw material(s) and/or a composition(s) of all cosmetics. Further, intravenous administration, local administration, local application and local spray of low concentrations and low dosages of ginsenosides, especially ginsenoside Rb₁, are useful as a composition for promoting regeneration and/or reconstruction of skin tissue after chemical peeling. Ginseng, a ginseng extract(s) or a crude saponin fraction(s) of ginseng containing ginsenosides, and. ginsenoside derivatives, especially dihydroginsenoside Rb₁, have also the same effectiveness, efficacy and usages as those of ginsenosides, especially ginsenoside Rb₁.

Ginsenosides, especially ginsenoside Rb₁ of the present invention can be used as not only an agent(s) for intravenous administration but also an agent(s) for external application and for injection to local lesion as far as the extracellular fluid concentrations of ginsenosides, especially ginsenoside Rb₁, in lesioned tissues can be maintained at low levels. Further, method of administration of ginsenosides, especially ginsenoside Rb₁, can be selected as subcutaneous injection, intramuscular injection, eye drops, eye ointment, nasal drops, ear drops, ear ointment, inhalation, intrarectal administration, oral administration, sublingual administration, transcutaneous administration, etc. Proviso that, when ginsenosides, especially ginsenoside Rb₁, are used as an agent(s) for oral administration, effectiveness may not be expected by administration of ginsenosides per se. Therefore, after ginsenosides, especially ginsenoside Rb₁, are admixed or encapsulated with a carrier(s) which inhibits decomposition of ginsenosides in the digestive tract (e.g. shellac, chitosan capsule, gelatin, oil layer, etc.) or with a carrier(s) for promoting absorption of ginsenosides in the digestive tract, oral administration of ginsenosides, especially ginsenoside Rb₁, should be performed. Further, if among the metabolites of ginsenosides, especially ginsenoside Rb₁, a substance(s) as effective as or more effective and efficacious than ginsenosides is identified, such an active metabolite(s) can be administered against diseases hereinbefore described, for which application of ginsenosides, especially ginsenoside Rb₁, can be expected, by the conventional methods. Further, after preparing dispersion of ginsenosides, especially ginsenoside Rb₁, or metabolites thereof of the present invention with polymer compound, the dispersion is spray dried to select any administration route. Further, after coating micro-particles of polymer compound with ginsenoside Rb₁, any administration route can be selected. Ginseng, a ginseng extract(s) or a crude saponin fraction(s) of ginseng containing ginsenosides, and ginsenoside derivatives, especially dihydroginsenoside Rb₁, have also the same effectiveness, efficacy and usage as those of ginsenosides, especially ginsenoside Rb₁.

A Preparation(s) for intravenous administration, a preparation(s) for external application to mucosa or a preparation(s) for external application to skin comprising ginsenosides, especially ginsenoside Rb₁, exhibits an epoch-making effect, which has not been known in the history, for prevention, therapy and/or treatment of diseases caused by injury, wound, trauma or defect of skin though skin tissue regeneration and reconstruction-promoting action, oral mucosal tissue regeneration and reconstruction-promoting action or wound healing-promoting action. Surely, administration of ginsenosides, especially ginsenoside Rb₁, leads "to fast and sure therapy of wound". Consequently, as a result of using ginsenosides, especially ginsenoside Rb₁, or metabolites thereof as a leading compound, many remedies for diseases caused by injury, wound, trauma or defect of skin will be newly developed in future. In order to support this fact, it has been found in the present invention that dihydroginsenoside Rb₁, which is prepared by using ginsenoside Rb₁ as a leading compound, showed an excellent skin tissue regeneration and reconstruction-promoting action. As a result of identifying the target molecule of ginsenosides, especially ginsenoside Rb₁, synthesis of a novel compound(s), which modifies function of the target molecule, can be possible. Further, as a result of preparing a prodrug(s) by modifying a part(s) of the chemical structures of ginsenosides, especially ginsenoside Rb₁, any administration route and administration method can be selected as described hereinbefore. The pharmaceutical composition(s) of the present invention exhibits, as far as low concentrations and/or low dosages are used, almost no side effects, and the present invention provides a highly safe drug.

Prevention of senile symptoms of skin by ginsenosides, especially ginsenoside Rb₁, as a cosmetic composition(s) is explained hereinbelow in detail.

Senility of skin or mucosa accompanied by aging is caused mainly by a decrease in the number of cells constituting skin or mucosa (death), functional failure of the cells, aplasia, poor regeneration or UV irradiation, and it exhibits symptoms such as atrophy, shrinkage, vulnerability to infection, easy infectivity, flabbiness, slackening, looseness, itching, roughness, oligosteatosis, asteatosis, cracks, rhagade, fissure, keratic cell ablation, exfoliation or ablation of keratinocytes, cornified cells, hornified cells or keratinized cells, exfoliation or ablation of the stratum corneum, corneum ablation, chapped, chapping, spots, blotches, chloasma, ephelis, wrinkles, furrows, lines, crease, freckle, poliosis, gray hair, dandruff, scurf, depilation, alopecia, pigmentation, sunburn, dryness, etc. Ginsenoside Rb₁ promotes regeneration and/or reconstruction of skin tissue or mucosal tissues and constituting cells thereof when the extracellular fluid concentrations of ginsenoside Rb₁ in lesioned tissues are at 1 ng/ml or less, preferably 10 pg/ml or less, more preferably 100 fg/ml or less, as well as facilitating the expression of a cell death-suppressing gene product Bcl-x_{L} to protect cells of skin or mucosa including epidermal cells, epidermal keratinocytes, epithelial cells, sebaceous gland cells, hornified cells, corneocytes, cornified cells, keratinized cells, melanocytes, Langerhans cells, Merkel cells, hair folliclular cells, salivary gland cells, sweat gland cells, mucous gland cells, muscle cells, serous gland cells, pilomotor muscular cells, fibroblasts, stem cells, mesenchymal cells, adipocytes, etc. (JP Appln. No. Hei 10-365560, PCT/JP99/02550, Brain cell or nerve cell protective agents comprising ginsenoside Rb₁). Consequently, when trace amount of ginsenosides, especially ginsenoside Rb₁, is admixed into every cosmetics or drug for health (cosmetic lotion (skin lotion), beauty liquid, agent for massage, agent for pack, emulsion, milky lotion, foundation cream, hand cream, cold cream, lotion, gel, emulsion, body milk, hair dye, hair manicure, eye shadow, cleansing cream, cleansing foam, night cream, beauty cream, troches, candy for cough, water for health, isotonic water, ice, sherbet, ice cream, sweet, candy, face powder, eye wash, cleansing liquid, collyrium, lipstick, cosmetic soap, gargle, shampoo, hair rinse, tooth paste, tooth powder, bath gel, lip cream, hair tonic, hair liquid, makeup base, UV liquid foundation, powder foundation, etc.) to maintain the extracellular fluid concentratinos of ginsenosides, especially ginsenoside Rb₁, in local region of skin or local region of mucosa at the low levels hereinbefore, excellent effect can be exhibited on senile symptoms of the skin or mucosa accompanied by aging (atrophy, shrinkage, vulnerability to infection, easy infectivity, slackening, looseness, flabbiness, itching, roughness, cracks, rhagade, fissure, asteatosis, oligosteatosis, epithelial exfoliation or ablation, mucosal exfoliation or ablation, corneocyte ablation, exfoliation or ablation of keratinocytes, cornified cells, hornified cells or keratinized cells, exfoliation or ablation of the stratum corneum, horny layer ablation, chapped, chap, ephelis, chloasma, crease, lines, furrows, wrinkles, freckle, alopecia, depilation, gray hair, poliosis, dandruff, scurf, pigmentation, sunburn, poor regeneration, aplasia, dryness, etc.).

When ginsenosides, especially ginsenoside Rb₁, are admixed into liquid cosmetics such as conventional UV liquid foundation, concentration thereof is adjusted to be 1000 ng/ml or less, preferably 10 ng/ml or less, more preferably 0.01 fg/ml - 100 pg/ml, and if the mixture is applied externally or sprayed externally onto skin every day, the extracellular fluid concentrations of ginsenosides, especially ginsenoside Rb₁, in the local region of skin can be maintained at the low levels as described hereinbefore. Then it is useful for improvement, prevention of progress or prevention of deterioration of the senile symptoms of skin (atrophy, shrinkage, dermatrophia, vulnerability to infection, easy infectivity, slackening, flabbiness, looseness, itching, alopecia, depilation, dandruff, scurf, gray hair, poliosis, roughness, cracks, rhagades, fissure, asteatosis, oligosteatosis, keratic cell ablation, exfoliation or ablation of keratinocytes, cornified cells, hornified cells or keratinized cells, exfoliation or ablation of the stratum corneum, corneum ablation, chapped, chapping, dryness, spots, blotches, ephelis, chloasma, crease, lines, furrows, wrinkles, freckle, pigmentation, poor regeneration, aplasia, sunburn, etc.). When ginsenosides, especially ginsenoside Rb₁, is admixed into solid, gelled or creamy cosmetics, such as conventional makeup base or night cream, the amount of admixed ginsenosides, especially ginsenoside Rb₁, is controlled to be 1000 ng or less, preferably 10 ng or less, more preferably 0.01 fg - 100 pg per g of cream, then the mixture is applied or sprayed externally onto skin for consecutive days. Thereby, the extracellular fluid concentrations of ginsenosides, especially ginsenoside Rb₁, in the local region of skin are maintained at the low levels as described hereinbefore, then the mixture (i.e. cosmetics) is useful for improvement, prevention of progress or prevention of deterioration of the senile symptoms of skin (atrophy, shrinkage, dermatrophia, vulnerability to infection, easy infectivity, slackening, looseness, flabbiness, itching, roughness, cracks, rhagades, fissure, oligosteatosis, asteatosis, keratic cell ablation, exfoliation or ablation of keratinocytes, hornified cells, cornified cells or keratinized cells, exfoliation or ablation of the stratum corneum,corneum ablation, chapped, chapping, dryness, spots, blotches, chloasma, ephelis, wrinkles, crease, lines, furrows, freckle, poliosis, gray hair, scurf, dandruff, depilation, alopecia, pigmentation, poor regeneration, aplasia, sunburn, etc. of skin). The upper limit of ginsenosides, especially ginsenoside Rb₁, that are contained in any cosmetics for prevention, improvement or treatment of the senile symptoms of skin is 10 µg/ml or less or lower in case of liquid cosmetics and 10 µg/g or less or lower in case of solid, gelled or creamy cosmetics. In other words, ginsenosides, especially ginsenoside Rb₁, are admixed preferably in the above described cosmetics at concentrations of 0.001% by weight or less, or lower. If not, membrane of normal cells in the skin may be damaged. Namely, when ginsenosides, especially ginsenoside Rb₁, are admixed into the cosmetics or health drug(s), which is used for long term, to make the concentration lower than that in the agent for external application for prevention, treatment or therapy of skin disease and mucosal disease as previously described in the present invention is thought to be safe. As described hereinbefore, since external application of ginsenoside Rb₁ at a concentration of 0.00000001% by weight to open wound of skin can exhibit sufficient effectiveness, generally, the concentrations of ginsenosides, especially ginsenoside Rb₁, in cosmetics are preferably lower than that. Consequently, when ginseng, a ginseng extract(s) or a crude saponin fraction(s) of ginseng is used as a cosmetic composition(s), its preferable concentration is less than 0.001% by weight, preferably 0.00001% by weight or less, more preferably 0.0000001% by weight or less. Of course, as described hereinabove, the cosmetics comprising trace amount of ginsenosides, especially ginsenoside Rb₁, can be applied or sprayed externally onto the face and also applied or sprayed externally onto the other skin (e.g. extremities, trunk, neck, head, etc.) which are frequently irradiated by sun light. Ginsenosides, especially ginsenoside Rb₁, are used for prevention, treatment and/or improvement of the senile symptoms of skin by using as bath gel in nursing facilities, hot spa health facilities, hospitals, etc. and thereby skin diseases (bedsore, ulcer, etc.) of aged person and bedridden patients can be prevented, improved and/or treated. When ginsenosides, especially ginsenoside Rb₁, are used as a health drug composition(s) or a composition(s) for external application to mucosa in order to prevent, treat or improve the senile symptoms of mucosa, it is necessary to use the low concentrations of ginsenosides, especially ginsenoside Rb₁, as described hereinabove.

As described, when the cosmetics or health drugs containing or comprising low concentrations of ginsenosides, especially ginsenoside Rb₁, are used usually for long term, the symptoms accompanied by senility of skin and mucosa (shrinkage, atrophy, easy infectivity, vulnerability to infection, slackening, loosening, flabbiness, itching, roughness, fissure, cracks, rhagades, asteatosis, oligosteatosis, keratic cell ablation, exfoliation or ablation of keratinocytes, cornified cells, hornified cells, or keratinized cells, exfoliation or ablation of the stratum corneum, corneum ablation, chapped, chapping, ephelis, chloasma, spots, blotches, crease, furrows, lines, wrinkles, freckle, gray hair, poliosis, dandruff, scurf, depilation, alopecia., pigmentation, sunburn, poor regeneration, aplasia, dryness, etc.) or the senile symptoms of mucosa (shrinkage, atrophy, mucosal ablation or exfoliation, epithelial ablation or exfoliation, poor regeneration, aplasia, chapped, cracks, rhagades, dryness, etc.) can be prevented or improved. Of course, low concentrations of a crude saponin fraction(s) of ginseng, a ginseng extract(s) or ginseng are used in place of ginsenosides, especially ginsenoside Rb₁, and the senile symptoms of skin or mucosa can also be prevented, improved or reduced.

Ginsenosides, especially ginsenoside Rb₁, can be admixed to every known cosmetics and health drug as far as low concentrations thereof are used. Further, ginsenosides, especially ginsenoside Rb₁, can be admixed into the cosmetics together with all cosmetic compositions heretofore used. Cosmetic compositions, which can be used together with ginsenosides, especially ginsenoside Rb₁, are described in U.S. Patent 5,663,160, and WO 99/07338. Proviso that the concentrations of the compositions, which can be used together with ginsenosides, especially ginsenoside Rb₁, are preferably made lower than those described in U.S. Patent 5,663,160, and WO99/07338. Of course, in case that cosmetic base or health drug base containing any effective component is newly developed, various symptoms caused by senility of skin or mucosa can be prevented, treated or improved by admixing ginsenosides, especially ginsenoside Rb₁, at low concentrations into the cosmetic base or health drug base as describe hereinbefore. In case of chemical peeling to promote regeneration and/or reconstruction of skin tissue, low concentrations of ginsenosides, especially ginsenoside Rb₁, are admixed (0.001% by weight or less or lower, preferably 0.00001% by weight or less, more preferably 0.00000001% by weight (10⁻⁸% by weight) or less, 10⁻²⁰% by weight or more) in an agent(s) for chemical peeling, and used mainly immediately after chemical peeling. When any other cosmetic composition(s) is used together with the low concentrations of ginsenosides, especially ginsenoside Rb₁, in combination, the concentrations of the other cosmetic composition(s) should preferably be set at lower levels than before.

Further, ginsenosides, especially ginsenoside Rb₁, can be admixed in a known hair restorer(s), hair tonic or new hair restorer(s) or hair tonic containing any effective components as like in the case of the cosmetics, preferably at low concentrations (0.001% by weight or lower or less, preferably 0.00001% by weight or less, more preferably 0.00000001% by weight or less and 10⁻²⁰% by weight or more), and they can be used for promoting hair restoration, hair growth or hair nourishment, for prevention of deterioration of alopecia or depilation, or for prevention of progress of alopecia or depilation. Of course, in place of ginsenosides, especially ginsenoside Rb₁, any natural product, natural extract, natural extracted substance, ginseng, a ginseng extract(s) or a crude saponin fraction(s) of ginseng containing ginsenoside Rb₁ is admixed into all cosmetics, hair restorers, pilatory or agent(s) for prevention of depilation progress, and if final concentrations of ginsenosides, especially ginsenoside Rb₁, are maintained at the low levels as described before, it can be used for improvement, prevention or treatment of the senile symptoms of mucosa or improvement, prevention or treatment of depilation. Proviso that, the amount of ginsenosides, especially ginsenoside Rb₁, admixed into the agent(s) for external application to skin, cosmetics, hair restorer(s), pilatory or agent(s) for prevention of depilation progress is tentative value, and actually the admixed amount of ginsenosides, especially ginsenoside Rb₁, in the cosmetics, health drug(s) (including bath gel)), hair restorer(s), pilatories or agent(s) for prevention of depilation progress should be adjusted so that the extracellular fluid concentrations thereof in local skin are kept at 1 ng/ml or less, preferably 10 pg/ml or less, more preferably 100 fg/ml or less. As described hereinbefore, when ginsenosides, especially ginsenoside Rb₁, ginseng, a ginseng extract(s) and/or a crude saponin fraction(s) of ginseng are used for long term as a cosmetic composition(s), a composition for hair restoration, hair growth, pilatory or health drug, even if the concentration(s) thereof is reduced to 10⁻²⁰% by weight, effectiveness can be observed.

When ginsenosides, especially ginsenoside Rb₁, is injected into locally lesioned region of skin or its penumbra for treatment or therapy of bedsore, skin ulcer, wound or for promotion of skin tissue regeneration or reconstruction, the concentrations of ginsenosides, especially ginsenoside Rb₁, in the local injection(s) should be adjusted to 10 µg/ml or less, preferably 100 ng/ml or less, more preferably 1 fg - 1000 pg/ml. The upper limit of concentration of ginsenosides, especially ginsenoside Rb₁, in the local injection is 10 mg/ml or less, preferably 100 µg/ml or less. Examples of solvent for skin local injection comprising ginsenosides, especially ginsenoside Rb₁, can be selected from, as same in case of using ginsenoside Rb₁ as a preparation(s) for intravenous administration, any one of physiological saline, distilled water, phosphate buffer, glucose solution, liposome or fat emulsion. Of course, the above local injection(s) can be injected into not only skin but also all organs or tissues (including transplantation organs) and can be used as eye drops and eardrops. Further, the concentrations of ginsenosides in the local injection(s) are tentative value and actually the admixed amount of ginsenosides, especially ginsenoside Rb₁, to the local injection should be adjusted so that the extracellular fluid concentrations of ginsenosides, especially ginsenoside Rb₁, in local injection region are kept at 1 ng/ml or less, preferably 10 pg/ml or less, more preferably 100 fg/ml or less.

When ginsenosides, especially ginsenoside Rb₁, are comprised in an agent(s) for external or topical application to skin for treatment or therapy of bedsore, decubitus, skin ulcer, wound or for promotion of skin tissue regeneration etc., ginsenosides, especially ginsenoside Rb₁, can be admixed into any base, and concentration thereof should be adjusted to 100 mg (0.1% by weight) or less or lower, preferably 10 mg (0.01% by weight) or less or lower, more preferably 1 mg (0.001% by weight) or less or lower and 1 fg (10⁻¹⁵% by weight) or more, per base 100 g or 100 ml. The upper limit of concentration of ginsenosides admixed into the above agent(s) for external or topical application to skin for prevention, therapy or treatment of skin diseases is thought to be about 10% by weight or less, preferably 1% or less.

Proviso that, the upper limit of concentration of ginsenosides, especially ginsenoside Rb₁, in the agent(s) for external or topical application to skin for treatment or therapy of skin diseases is tentative value, and actually the admixed amount of ginsenosides should be adjusted by using the extracellular fluid concentrations of ginsenosides in local skin region as an index.

It will be explained by introducing experimental results of the inventors in detail that crude saponin fraction described in PCT/JP00/04102 exhibits effectiveness and efficacy on skin tissue regeneration and reconstruction as same in ginsenoside Rb₁. The inventor (Sakanaka) had already found the superior effectiveness and efficacy that continuous intravenous administration of ginsenoside Rb₁ (60 µg/day) could stand up bed-ridden rats (weighing about 300 g) with spinal cord injury (JP Appln. No. Hei 11-243378, PCT/JP99/06804, Cerebrovascular regeneration/reconstruction promoters and nervous tissue secondary degeneration inhibitors comprising ginsenoside Rb₁). Further, the present inventors (Sakanaka and Nakata) had found that continuous intravenous administration of a low dose of crude saponin fraction of ginseng (870 µg/day), like continuous intravenous administration of ginsenoside Rb₁ (60 µg/day), could stand up bed-ridden rats with spinal cord injury (PCT/JP00/04102, Brain cell or nerve cell protecting agents comprising ginseng).

As described in the experimental results using rats with spinal cord injury, continuous intravenous administration of the crude saponin fraction of ginseng (870 µg/day) shows similar effectiveness to continuous intravenous administration of ginsenoside Rb₁ (60 µg/day). This indicates that if the crude saponin fraction about 14.5-times larger in amount than ginsenoside Rb₁ is continuously administered, an effective extracellular fluid concentration(s) of the crude saponin fraction can be maintained in the lesioned tissue. One of the inventors (Sakanaka) have demonstrated that ginsenoside Rb₁ exhibits effectiveness and efficacy when its extracellular fluid concentrations in lesioned tissues are 1 ng/ml or less, preferably 10 pg/ml or less, more preferably 100 fg/ml or less (Japanese Patent Appln. No. Hei 10-365560 and PCT/JP99/02550, Brain cell or nerve cell-protective agents comprising ginsenoside Rb₁). Consequently, the low dosage(s) of crude saponin fraction is likely to exhibit superior neuroprotective action when its extracellular fluid concentrations in lesioned tissues are 14.5 ng/ml or less, preferably 145 pg/ml or less, more preferably 1450 fg/ml or less. Further, since ginsenoside Rb₁ is able to exhibit sufficient effect even when its extracellular fluid concentrations in lesioned tissues are 1 - 100 fg/ml or less (e.g. 0.01 fg/ml), the pharmaceutical composition or preparation comprising crude saponin fraction appears to exhibit sufficient effect even when its extracellular fluid concentrations in lesioned tissues are 14.5 - 1450 fg/ml or 1/100 thereof.

As demonstrated by one of the inventors (Sakanaka) in Japanese Patent Appln. No. Hei 10-365560 and PCT/JP99/02550 (Brain cell or nerve cell-protective agents comprising ginsenoside Rb₁), continuous intravenous administration of ginsenoside Rb₁ (60 µg/day) exhibited superior therapeutic effect for cerebral apoplexy and cerebral infarction. Further, based on the above described experimental results using rats with spinal cord injury, it is demonstrated that continuous intravenous administration of a crude saponin fraction (870 µg/day) exhibits, in the same manner as of the continuous intravenous administration of ginsenoside Rb₁ (60 µg/day), superior therapeutic effect for spinal cord injury. On the basis of these facts, continuous intravenous administration of the crude saponin fraction (870 µg/day) can be estimated to exhibit, in the same manner as of the continuous intravenous administration of ginsenoside Rb₁ (60 µg/day), superior therapeutic effect for cerebral apoplexy and cerebral infarction. Further, since continuous intravenous administration of ginsenoside Rb₁ at a dose of 6 µg/day also exhibits superior therapeutic effect for cerebral infarction and cerebral apoplexy (Japanese Patent Appln. No. Hei 10-365560 and PCT/JP99/02550, Brain cell or nerve cell-protective agents comprising ginsenoside Rb₁), continuous intravenous administration of the crude saponin fraction at a dose of 87 µg/day is thought to exhibit superior therapeutic effect for cerebral infarction and cerebral apoplexy. Namely, continuous intravenous administration of the crude saponin fraction of ginseng at doses of 87 µg/day - 870 µg/day, in rats weighing approximately 300 g can exhibit superior brain cell or nerve cell-protecting action. Consequently, as a result of continuous intravenous administration of the crude saponin fraction at dosages of 2.9 mg/kg/day - 0.29 mg/kg/day, superior brain cell-protecting effect or nerve cell-protecting effect is obtained. However, this is merely a tentative value for amount of administration of the crude saponin fraction to rats weighing about 300 g, and when the crude saponin fraction(s) of ginseng is intravenously administered to human, the amount of administration per kg of body weight should be set about 1/2 - 1/20 of the above described value. Namely, when the crude saponin fraction(s) of ginseng is intravenously administered to human, although depending-on pathological states of patients and individual differences, the amount of administration is preferably set at 1450 µg/kg/day or less and 14.5 µg/kg/day or more.

Further, since in Japanese Patent Appln. No. Hei 10-365560 and PCT/JP99/02550 (Brain cell or nerve cell-protective agents comprising ginsenoside Rb₁) one of the inventors (Sakanaka) have found that continuous intravenous administration of ginsenoside Rb₁ (60 µg/day) caused an increase in the expression of. a cell death-suppressing factor in brain and nervous tissues, i.e. Bcl-x_{L} gene, continuous intravenous administration of a crude saponin fraction of ginseng (870 µg/day) is also considered to upregulate the expression of Bcl-x_{L}. Namely, the crude saponin fraction(s) of ginseng can promote the expression of Bcl-x_{L} gene in nerve cells or neurons when its extracellular fluid concentrations in lesioned tissues are 14.5 ng/ml or less, preferably 145 pg/ml or less, more preferably 1450 fg/ml or less.

Further, the fact that continuous intravenous administration of low dosages of the crude saponin fraction(s) of ginseng is effective for prevention, therapy or treatment of spinal cord injury, cerebral infarction or cerebral apoplexy, any one of components in the crude saponin fraction(s) obviously shows superior effectiveness and efficacy against brain and nervous diseases. Of course, plurality of components in the crude saponin fraction(s) of ginseng may show superior effectiveness and efficacy for brain and nervous diseases. Purified saponins or ginsenosides having similar chemical structures in the crude saponin fraction(s) are: ginsenoside Ro, ginsenoside Rb₁, ginsenoside Rb₂, ginsenoside Rc, ginsenoside Rd, ginsenoside Re, ginsenoside Rf, ginsenoside Rg₁, ginsenoside Rg₂, ginsenoside Rg₃, ginsenoside Rh₁, ginsenoside Rh₂, ginsenoside Ra₁, ginsenoside Ra₂, ginsenoside Ra₃, notoginsenoside R₄, cinquenosideR₁, malonylginsenoside Rb₁, ginsenoside RS₁, malonylginsenoside Rb₂, ginsenoside RS₂, malonylginsenoside Rc, malonylginsenoside Rd, ginsenoside Rb₃, (20S)-ginsenoside Rg₃, 20-glucoginsenoside Rf, notoginsenoside R₁, (20R)-ginsenoside Rg₂, (20R)-ginsenoside Rh₁, etc. Among them, it is known that amount of content of ginsenoside Rb₁ is 2 times or more of the other purified saponins. Considering the fact that ginsenoside Rb₁ exhibits nerve cell or brain cell-protecting action when its extracellular fluid concentrations in lesions are 1 ng/ml or less, preferably 10 pg/ml or less, more preferably 100 fg/ml or less, the other purified saponins, i.e. ginsenosides, are likely to protect brain cells or nerve cells in the same concentration range or in the concentration ranges from 1/10 to 1/100 thereof. Proviso that, components in the crude saponin fraction(s) of ginseng are not limited within the above mentioned purified saponins, i.e. ginsenosides.

As explained hereinabove, the crude saponin fraction(s) of ginseng or any one of components thereof can exhibit, in the same manner as in ginsenoside Rb₁, superior brain cell or nerve cell-protecting action and therapeutic effect for spinal cord injury, head injury or neurotrauma. Consequently, low concentrations and/or low dosages of the crude saponin fraction(s) of ginseng or any one of components thereof can exhibit effectiveness, efficacy or usages of ginsenoside Rb₁, which is described by one of the inventors (Sakanaka) in (Japanese Patent Appln. No. Hei 10-365560, PCT/JP99/02550, Brain cell or nerve cell-protective agents comprising ginsenoside Rb₁; Japanese Patent Appln. No. Hei 11-340850, PCT/JP99/06804, Cerebrovascular regeneration and reconstruction promoter and nervous tissue secondary degeneration inhibitor comprising ginsenoside Rb₁). Namely, low concentrations and/or low dosages of the crude saponin fraction(s) of ginseng or any one of components thereof, like ginsenoside Rb₁, can increase the expression of a cell death-suppressing gene product Bcl-x_{L}, suppress neuronal apoptosis or apoptosis-like cell death, and exhibit superior cyto-protective action in the peripheral tissues.

In addition to prior patent applications (JP Appln. No. Hei 10-365560, PCT/JP99/02550; JP Appln. No. Hei 11-243378, PCT/JP99/06804) which indicate that continuous intravenous administration of ginsenoside Rb₁ (60 µg/day) is extremely effective for prevention, treatment or therapy of brain and nervous diseases including cerebral apoplexy and neurotrauma, as well as exhibiting superior cyto-protective effect, it is found in the present invention that continuous intravenous administration of ginsenoside Rb₁ (60 µg/day or 12 µg/day) exhibits superior therapeutic effect for skin diseases through skin tissue regeneration and reconstruction-promoting action or wound healing-promoting action. Consequently, based on the above described speculation that continuous intravenous administration of ginsenoside Rb₁ (60 µg/day) exhibits effectiveness and efficacy similar to continuous intravenous administration of the crude saponin fraction(s) of ginseng (870 µg/day), continuous intravenous administration of the crude saponin fraction(s) of ginseng (870 µg/day) is likely to exhibit superior therapeutic effect for skin diseases through skin tissue regeneration-promoting action or wound healing-promoting action. Namely, the effectiveness, efficacy and usages of ginsenosides, especially ginsenoside Rb₁, which have been newly found in the present invention, are all in common with the effectiveness, efficacy and usages of low doses and low concentrations of the crude saponin fraction(s) of ginseng and components thereof. In fact, as explained hereinabove, in the experiments using cuttings of pothos, it was found that a crude saponin fraction of ginseng promoted regeneration, generation and/or reconstruction of plant tissues as like ginsenoside Rb₁.

Further, with regard to a method for administration of the crude saponin fraction(s) of ginseng or components thereof, as like ginsenoside Rb₁, if the extracellular fluid concentrations in lesioned tissues can be maintained at the low levels as described hereinbefore, any administration route can be selected. Concretely, low concentrations and/or low dosages of the crude saponin fraction(s) of ginseng or components thereof can be used not only as an agent(s) for intravenous administration but also as an agent(s) for external application or an agent(s) for local lesion administration. Further, with regard to a method for administration of the crude saponin fraction(s) of ginseng or any one of components thereof, any optional route such as subcutaneous administration, intramuscular injection, eye drops, nasal drops, ear drops, inhalation, intrarectal administration, oral administration, sublingual administration, percutaneous administration, etc. can be selected. The crude saponin fraction(s) of ginseng can be used as a sustained release agent(s). Of course, solvent and base for formulation of the crude saponin fraction(s) of ginseng are the same as those for ginsenoside Rb₁. Proviso that, when the crude saponin fraction(s) of ginseng or any one of components thereof is used as an agent(s) for oral administration, there is a possibility for not exhibiting so favorable effect if the crude saponin fraction(s) of ginseng or any one of components thereof is administered alone. In that case, the crude saponin fraction(s) of ginseng or any one of components thereof is admixed, encapsulated or bound with a carrier which inhibits decomposition in the digestive tract or a carrier which promotes absorption in the digestive tract, thereafter administered orally. Further, among any one of metabolites of the crude saponin fraction(s) of ginseng or components thereof, if a substance(s) having effectiveness and efficacy equal to or higher than those of the crude saponin fraction(s) of ginseng or components thereof is identified, such the active metabolite product can be administered by the conventional methods to patients with the above mentioned diseases for which application of the crude saponin fraction(s) of ginseng or components thereof at low concentrations and low dosages is expected. Further after preparing dispersion of low concentrations and low dosages of the crude saponin fraction(s) of ginseng or components thereof with polymer compound, said dispersion is sprayed out, dried and then administered via optional route. Furthermore, micro-particles of polymer compound are coated with the crude saponin fraction(s) of ginseng or any one of components thereof, thereafter any route of administration is selected. Of course, after preparing a prodrug(s) by using the crude saponin fraction(s) of ginseng or any one of components thereof, any route of administration may be selected.

Further, low concentrations and low dosages of the crude saponin fraction(s) of ginseng or any one of components thereof can be used as a health drug(s) for improving or ameliorating hypofunction of immunity, hypofunction of skin, hypofunction of circulation, hypofunction of digestion, hypofunction of bone metabolism, decrease of muscle force, hypofunction of joint or hypogonadism accompanied by aging. Further, low concentrations and/or low dosages of the crude saponin fraction(s) of ginseng or any one of components thereof is used as a cosmetic composition(s), and is able to applied for prevention, therapy or treatment of senile symptoms accompanied by aging (atrophy, shrinkage, vulnerability to infection, easy infectivity, slackening, looseness, flabbiness, itching, roughness, fissure, cracks, rhagades, oligosteatosis, asteatosis, keratic cell ablation, exfoliation or ablation of keratinocytes, hornified cells, cornified cells or keratinized cells, exfoliation or ablation of the stratum corneum, corneum ablation, chapped, chapping, blotches, spots, ephelis, chloasma, crease, lines, furrows, wrinkles, freckle, poliosis, gray hair, scurf, dandruff, depilation, alopecia, pigmentation, sunburn, poor regeneration, aplasia, dryness, etc. of skin).

The crude saponin fraction(s) of ginseng as a cosmetic composition(s) is explained in detail as follows. Generally, a crude saponin fraction of ginseng is obtained by extracting red ginseng with methanol, suspending the extract (yield 35 - 45%) in water, washing with ether and by shaking with water-saturated butanol; yield is about 8%. Proviso that the crude saponin fraction(s) of ginseng in the present invention is not limited within one obtained from red ginseng, but includes substances obtained from other ginseng (e.g. sanshichi ginseng, denshichi ginseng, Himalayan ginseng, American ginseng, chikusetsu ginseng, etc.).

Senility of skin accompanied by aging is mainly produced by ultraviolet irradiation, death of skin cells or dysfunction thereof, and presents atrophy, shrinkage, dermatrophia, vulnerability to infection, easily infectivity, looseness, flabbiness, slackening, itching, roughness, cracks, rhagades, fissure, asteatosis, oligosteatosis, keratic cell ablation, exfoliation or ablation of keratinocytes, hornified cells, cornified cells or keratinized cells, exfoliation or ablation of the stratum corneum, corneum ablation, chapped, chapping, blotches, spots, ephelis, chloasma, crease, furrows, wrinkles, freckle, gray hair, poliosis, alopecia, depilation, dandruff, scurf, pigmentation, sunburn,poor regeneration, aplasia, dryness, etc. Since the crude saponin fraction(s) of ginseng is thought to exhibit skin and mucosal cell-protecting action and regeneration and reconstruction-promoting action of said cells including epidermal cells, epidermal keratinocytes, Langerhans cells, melanocytes, Merkel cells, keratinocytes, salivary gland cells, mixed gland cells, sebaceous gland cells, hair follicle cells, mucous gland cells, muscle cells, pilomotor muscle cells, sweat gland cells, fibroblasts, stem cells, mesenchymal cells, adipose cells, etc. at extracellular fluid concentrations in lesioned tissues of 14.5 ng/ml or less, preferably 145 pg/ml or less, more preferably 1450 fg/ml or less, if trace amount of the crude saponin fraction(s) of ginseng is admixed into every cosmetics or drugs for health, e.g. cosmetic lotion (skin lotion), beauty liquid, agent for massage, agent for pack, emulsion, milky lotion, foundation cream, hand cream, cold cream, lotion, gel, emulsion, body milk, hair dye, hair manicure, eye shadow, cleansing cream, cleansing foam, night cream, beauty cream, troches, candy for cough, water for health, isotonic water, ice, sherbet, ice cream, sweet, candy, face powder, eye wash, cleansing liquid, collyrium, lipstick, cosmetic soap, gargle, shampoo, hair rinse, tooth paste, tooth powder, bath gel, lip cream, hair tonic, hair liquid, makeup base, UV liquid foundation, powder foundation, etc. and used to keep the extracellular fluid concentrations in local region of skin low as described hereinbefore, excellent effects can be exhibited on senile symptoms of the skin accompanied by aging. Base for the crude saponin fraction(s) of ginseng and compositions for external application to skin, which can be used in combination, are the same as in case of ginsenoside Rb₁.

When a crude saponin fraction(s) of ginseng is admixed into liquid cosmetics such as conventional UV liquid foundation, concentration thereof is adjusted at 14.5 µg/ml or less, preferably 145 ng/ml or less, more preferably 0.145 fg/ml - 1450 pg/ml, and the mixture is applied externally or sprayed externally onto skin every day to keep the extracellular fluid concentrations of the crude saponin fraction(s) of ginseng in the local skin region at the low levels as described hereinbefore, then it is useful for improvement, prevention of progress or prevention of deterioration of the senile symptoms of skin (atrophy, shrinkage, dermatrophia, vulnerability to infection, easy infectivity, flabbiness, looseness, slackening, itching, depilation, alopecia, dandruff, scurf, poliosis, gray hair, roughness, fissure, cracks, rhagades, oligosteatosis, asteatosis, keratic cell ablation, exfoliation or ablation of keratinocytes, hornified cells, cornified cells or keratinized cells, exfoliation or ablation of the stratum corneum, corneum ablation, chapped, chapping, dryness, spots, bloches, ephelis, crease, lines, furrows, wrinkles, freckle, chloasma, pigmentation, poor regeneration, aplasia, sunburn, etc.). When the crude saponin fraction(s) of ginseng is admixed into solid, gelled or creamy cosmetics, such as conventional makeup base or night cream, amount of the admixed crude saponin fraction(s) of ginseng is controlled to 14.5 µg or less, preferably 145 ng or less, more preferably 0.145 fg - 1450 pg per g of cream. Then, the mixture is applied or sprayed externally onto skin for consecutive days for prevention, therapy or treatment of the senile symptoms of skin (atrophy, shrinkage, dermatrophia, vulnerability to infection, easy infectivity, flabbiness, slackening, looseness, itching, roughness, cracks, rhagades, fissure, oligosteatosis, asteatosis, keratic cell ablation, exfoliation or ablation of keratinocytes, cornified cells, hornified cells or keratinized cells, exfoliation or ablation of the stratum corneum, corneum ablation, chapped, chapping, dryness, spots, blotches, ephelis, crease, wrinkles, lines, furrows, chloasma, freckle, poliosis, gray hair, scurf, dandruff, depilation, alopecia, pigmentation, poor regeneration, aplasia, sunburn, etc. of skin). The upper limit of concentration of the crude saponin fraction(s) of ginseng, which is admixed in the above cosmetics for the purpose of prevention, improvement or treatment of the senile symptoms of skin, is 145 µg/ml in liquid cosmetics, and 145 µg/g in solid, gelled or creamy cosmetics. In other words, the crude saponin fraction(s) of ginseng is admixed preferably in the above-described cosmetics at concentration of 0.0145% by weight or less. If not, membrane of normal cells in the skin may be damaged. Namely, when the crude saponin fraction(s) of ginseng is admixed into the cosmetics or health drug, which is used for long term, it is safe to lower its concentration. Judging from the fact that ginsenoside Rb₁ exhibits superior wound healing effect at a low concentration of 0.00000001% by weight (10⁻⁸% by weight), the concentration of a crude saponin fraction(s) of ginseng and a ginseng extract(s) or ginseng, which will be explained later, is preferably, in the same manner as ginsenosides, 0.001% by weight or less or lower. Of course, as described hereinabove, the cosmetics comprising or containing trace amount of a crude saponin fraction(s) of ginseng can be applied or sprayed externally onto the face and also applied or sprayed externally to the other skin regions (e.g. extremities, trunk, neck, head, etc.) which are frequently irradiated by sun light. As described, when the cosmetics or preparations for external application to skin comprising a crude saponin fraction(s) of ginseng at low concentrations are used usually for long term, the symptoms accompanied by senility of skin (shrinkage, atrophy, easy infectivity, vulnerability to infection, slackening, looseness, flabbiness, itching, roughness, fissure, cracks, rhagades, asteatosis, oligosteatosis, keratic cell ablation, exfoliation or ablation of keratinocytes, cornified cells, hornified cells or keratinized cells, exfoliation or ablation of the stratum corneum, corneum ablation, chapping, chapped, ephelis, spots, blotches, chloasma, crease, lines, furrows, wrinkles, freckle, gray hair, poliosis, scurf, dandruff, alopecia, depilation, pigmentation, sunburn, poor regeneration, aplasia, dryness, etc.) can be prevented, reduced or improved. The crude saponin fraction(s) of ginseng can be used at the above-described low concentration as a health drug composition(s) in order to prevent, reduce or improve senile symptoms of mucosa (shrinkage, chapped, chapping, dryness, etc.). It has already been described that low concentrations of a crude saponin fraction(s) of ginseng, a ginseng extract(s) or ginseng can be utilized as like ginsenosides, especially ginsenoside Rb₁, for cultivation of marine products, cultivation of farm products and/or bath gel in nursing facilities and hot spring nursing facilities.

In place of the crude saponin fraction(s) of ginseng, if a component(s) of the crude saponin fraction(s) of ginseng in almost equal amount of ginsenosides, especially ginsenoside Rb₁, is admixed in cosmetics or an agent(s) for external application to skin, various symptoms accompanied by senility of skin can be prevented. Ginsenosides, especially ginsenoside Rb₁, a crude saponin fraction(s) of ginseng, or components thereof other than ginsenosides, can be admixed in conventional cosmetics and an agent(s) for external application to skin, even in use at low concentration. Of course, in case of developing a novel base for cosmetics containing any effective component, various symptoms caused by senility of skin and mucosa can be prevented, treated and improved by mixing the base with low concentrations of ginsenosides, ginsenoside Rb₁, a crude saponin fraction(s) of ginseng, or components thereof other than ginsenosides. Further as described hereinbefore, multiple combination of low concentrations of ginsenosides, ginsenoside Rb₁, a crude saponin fraction(s) of ginseng, or a component(s) of the crude saponin fraction(s) of ginseng is admixed in conventional cosmetics, health drug or agent for external application to skin, or admixed in novel cosmetic base containing any effective component, then cosmetics, health drug or agent for external application to skin for preventing, treating or improving various symptoms caused by aging can be prepared. When multiple combination of low concentrations of ginsenosides, especially ginsenoside Rb₁, a crude saponin fraction(s) of ginseng, or a component(s) of the crude saponin fraction(s) of ginseng is admixed in cosmetics, health drug or agent for external application to skin, any concentration can be selected, as long as each concentration in cosmetics and agent for external application to skin is low. Further, in case of promoting regeneration and/or reconstruction of skin tissue in chemical peeling, as described hereinbefore, low concentrations of a crude saponin fraction(s), a crude saponin fraction component(s), ginseng, a ginseng extract(s) or ginsenosides, especially ginsenoside Rb₁ are admixed in a chemical peeling agent(s).

Further, ginsenosides, especially ginsenoside Rb₁, a crude saponin fraction(s) of ginseng, or a component(s) of the crude saponin fraction(s) of ginseng can be used for hair growth, hair restoration or pilatory, prevention of depilation progress or for prevention of deterioration of depilation or alopecia by admixing them at low concentrations in conventional hair restorers and pilatories or novel hair restorers or pilatories containing any effective component in the same manner as of the method in the cosmetics. Further, in place of the crude saponin fraction(s) of ginseng, which is admixed into the cosmetics, hair-restorers or pilatories, a ginseng extract(s) or ginseng in an amount almost equal to that of the crude saponin fraction(s) or 5 - 6 times larger amount thereof may be used. Of course, a crude saponin fraction(s) of ginseng and a ginseng extract(s) are used in combination and are admixed into the above described cosmetics, hair restorers, pilatories or agents for preventing depilation, as long as using them at low concentrations. Furthermore, any natural product or natural composition, which comprises or contains ginsenosides, especially ginsenoside Rb₁, or other ginseng components, is admixed in all kinds of cosmetics, hair-restorers, pilatories or agents for preventing depilation; as a result, if the final content of ginsenosides, especially ginsenoside Rb₁, or other ginseng component can be maintained at the low concentrations as described hereinbefore, it can be utilized for improvement, prevention or treatment of senility of skin or depilation. It has already been described that a ginseng extract(s) or a component(s) of the crude saponin fraction(s) of ginseng can be utilized, as long as it is used at low concentrations similar to ginsenoside Rb₁, for cultivation of marine products, cultivation of farm products and for a bath gel in nursing facilities or hot spring resort facilities.

When a crude saponin fraction(s) of ginseng is injected into a local lesion of skin or its penumbra for treatment or therapy of bedsore, decubitus, skin ulcer, wound or for promotion of skin tissue regeneration or reconstruction, the concentrations of the crude saponin fraction(s) in the local injection(s) should be adjusted to 145 µg/ml or less, preferably 1.45 µg/ml or less, more preferably 0.145 fg/ml-14.5 ng/ml. The upper limit of concentration of the crude saponin fraction(s) in the local injection is 14.5 mg/ml or less, preferably 1.45 mg/ml or less. Examples of solvent for local skin injection comprising a crude saponin fraction(s) of ginseng can be selected from, in the same manner as of using ginsenoside Rb₁ as a local injection(s) to skin or a preparation(s) for intravenous administration, any one of physiological saline, distilled water, phosphate buffer, glucose solution, liposome or fat emulsion. Of course, the above local injection(s) can be injected into not only skin but also all organs or tissues and can be used as eye drops and ear drops. Further, the concentrations of the crude saponin fraction(s) of ginseng in the local injection(s) are tentative value and actually the admixed amount of the crude saponin fraction(s) of ginseng in the local injection should be adjusted so that the extracellular fluid concentrations of the crude saponin fraction(s) of ginseng in local injection region are kept at 14.5 ng/ml or less, preferably 145 pg/ml or less, more preferably 1450 fg/ml or less.

When a crude saponin fraction(s) of ginseng is used as a preparation(s) for external application to skin for therapy of bedsore, decubitus, skin ulcer or wound, or for promotion of skin tissue regeneration and/or reconstruction, the crude saponin fraction can be admixed with any base, but its concentration should be adjusted to 145 mg (0.145% by weight) or less, preferably 1.45 mg (0.00145% by weight) or less, more preferably 0.145 mg (0.000145% by weight) or less per 100 g or 100 ml of base. The upper limit of concentration of the crude saponin fraction(s) admixed in the agent(s) for external or topical application to skin in order to prevent, treat or cure the above described skin diseases is approximately 1.45%. Proviso that as described hereinbefore, since ginsenoside Rb₁ can treat or cure open wound at the concentration of 0.00000001% (10⁻⁸% by weight) by weight, the concentration of ginseng, a ginseng extract(s) or a crude saponin fraction(s) of ginseng in the agent(s) for external or topical application to skin can be preferably set at levels less than 0.001% by weight.

Of course, the content of the above crude saponin fraction(s) of ginseng in the agent(s) for external or topical application to skin is tentative value, and actually, the amount of the crude saponin fraction(s) of ginseng to be admixed should be adjusted with the extracellular fluid concentrations of the crude saponin fraction(s) in the local region of the skin as an index.

Finally, ginsenosides, especially ginsenoside Rb₁ or a crude saponin fraction(s) of ginseng used in the present invention is known as a component(s) of ginseng and is a pharmaceutical composition(s), a composition(s) for external or topical application to mucosa, a health drug composition(s), a composition(s) for hair growth, hair restoration or pilatory, a veterinary drug composition(s), a composition(s) for chemical peeling, a composition(s) for growth regulation or a cosmetic composition(s) with extremely low adverse effects. Further, it has already been described that the effectiveness, efficacy and usages of ginsenosides, especially ginsenoside Rb₁ described in the present invention are also in common with those of prosaposin-related peptides having Bcl-x_{L} expression-upregulating action (JP Appln No. Hei 11-185155) and the other compounds showing brain cell-protective action by intracerebroventricular administration.

As described hereinabove, the pharmaceutical composition(s), cosmetic composition(s), composition(s) for chemical peeling, health drug composition(s), fertilizer composition(s), feed composition, composition(s) for external application to mucosa, composition(s) for growth regulation, veterinary drug composition(s) and composition(s) for hair growth, hair restoration or pilatory of the present invention exhibit superior effects on regeneration and/or reconstruction of the vial or viable tissues (animal tissues and plant tissues) and lesioned tissues. Consequently, the present invention provides a method for treating or curing organic diseases through regeneration and/or reconstruction of cells or tissues comprising using the pharmaceutical composition or veterinary drug composition(s) of the present invention. Further, the present invention provides a method for make up comprising using the cosmetic composition(s) of the present invention, a method for chemical peeling comprising using the composition(s) for chemical peeling, and a method for hair growth, hair restoration or pilatory comprising using the composition(s) for hair growth, hair restoration or pilatory of the present invention. Further, the present invention provides a method for increased production of foodstuff comprising using the fertilizer composition(s) or feed composition(s) of the present invention.

Furthermore, the present invention provides use of ginsenosides, especially ginsenoside Rb₁ for production of medicinal drug preparations or veterinary drug preparations comprising the pharmaceutical composition(s) or veterinary drug composition(s) of the present invention. Further, the present invention provides use of ginsenoside derivatives, especially dihydroginsenoside Rb₁ having effectiveness, efficacy and usages similar to those of ginsenoside Rb₁.

### Examples

The present invention will be explained in detail by concrete experimental examples, but the present invention is not limited by these concrete examples.

### Example 1 (Incised wound healing by intravenous infusion of ginsenoside Rb₁)

Male wistar rats, weighing about 300 g, were used. Animals were bred in a room furnished with a 12:12 hour light-dark cycle and water and feeds were supplied ad libitum. Incised wound, about 3 cm in length, was made on the dorsal region of each animal under inhalation anesthesia, sutured by using nylon thread, and one hour later, ginsenoside Rb₁ (60 µg) dissolved in physiological saline was intravenously infused once. Thereafter, ginsenoside Rb₁ was continuously infused intravenously for 7 days (60 µg/day) through an Alza osmotic minipump.

Control animals, which underwent operation of similar incisional wound and were sutured with nylon thread, received intravenous administration of an equal amount of physiological saline alone.

Two days after finishing continuous intravenous administration of ginsenoside Rb₁ or physiological saline alone, the animals were anesthetized with pentobarbital and fixed transcardialy with perfusion of 0.1 M phosphate buffer containing 4% paraformaldehyde. Thereafter, the skin tissue including the sutured region of incised wound was collected, post-fixed and embedded in paraffin. Paraffin sections with 5 µm thickness were cut and stained with hematoxylin-eosin (HE). Result is shown in Fig. 1. Fig. 1A shows a case of ginsenoside Rb₁ administration and Fig. 1B shows a case of physiological saline administration. In the figure, "s" indicates scar or granulation.

As shown in Fig. 1A, in the case of ginsenoside Rb₁ administration, as compared with the case of physiological saline administration in Fig. 1B, scar or granulation formation was obviously little and many skin appendages such as sweat glands, sebaceous glands and hair follicles were observed in close proximity to the local region of incised wound. Further, in case of ginsenoside Rb₁ administration, which differs from the case of physiological saline administration, except for the local region of wound, the epidermis, dermis and subcutaneous tissue were regenerated, reconstructed or recovered to the nearly normal condition.

### Example 2 (Open wound healing or skin defect healing by intravenous infusion of ginsenoside Rb₁)

Male wistar rats weighing about 300 g were used. The punch biopsy with diameter 6 mm was performed in the dorsal region of animals under inhalation anesthesia to make open wound and the animals were allow to leave as they were. About 1 hour later, ginsenoside Rb₁ (12 µg) dissolved in physiological saline was administered intravenously once, then ginsenoside Rb₁ was continuously infused intravenously for 7 days by using an Alza osmotic minipump (12 µg/day).

An equal amount of physiological saline was administered intravenously in the control animals, which received the same open wound and were allow to leave as they were.

Two days after finishing continuous intravenous administration of ginsenoside Rb₁ or physiological saline alone, the animals were anesthetized with pentobarbital and fixed transcardialy with perfusion of 0.1 M phosphate buffer containing 4% paraformaldehyde. Thereafter, the skin tissue including the open wound was dissected out, post-fixed and embedded in paraffin. Paraffin sections 5 µm thick were cut and stained with hematoxylin-eosin (HE). Result is shown in Fig. 2. Fig. 2A shows a case of ginsenoside Rb₁ administration and Fig. 2B shows a case of physiological saline administration. Left side from the arrow in Fig. 2A and B, indicates healthy region; right side from the arrow in Fig. 2A, indicates regenerated skin tissue; and right side from the arrow in Fig. 2B, indicates mainly scar or granulation. In the regenerated skin tissue in Fig. 2A, many hair follicles and hair papillae as well as associated sebaceous glands and pilomotor muscles are observed in the subepidermal connective tissue (dermis or subcutaneous tissue), and a small amount of scar or granulation was observed under the regenerated and reconstructed skin tissue.

As shown in Fig. 2A, in the ginsenoside Rb₁-administered case, as compared with the physiological saline-administered case in Fig. 2B, scar or granulation formation was obviously little and sufficient epithelization occurred, and regeneration and/or reconstruction of the subcutaneous tissue and the connective tissue of dermis with papillae proceeded to the condition close to that of normal tissue. Further, in ginsenosrde Rb₁-administered case, which is different from the physiological saline-administered case, the skin appendages such as hair follicles, hair papillae, pilomotor muscles, sweat glands, sebaceous glands, etc. were observed abundantly in the skin tissue regenerated after open wound, and the blood vessel networks were regenerated and reconstructed to the condition close to that of the normal tissue.

### Example 3 (Improving effect of open wound or skin defect by intravenous preinfusion of ginsenoside Rb₁)

Intravenous administration of physiological saline solution containing ginsenoside Rb₁ (12 µg) was conducted once in male Wistar rats weighing about 300 g under inhalation anesthesia, subsequently, ginsenoside Rb₁ was continuously infused intravenously for 4 days through an Alza osmotic minipump at a dose of 12 µg/day. Thereafter, the punch biopsy with diameter 6 mm was performed in the dorsal region of animals under inhalation anesthesia to make open wound, and the continuous intravenous infusion of, ginsenoside Rb₁ further lasted 3 days.

An equal amount of physiological saline was administered intravenously in the control animals, which were subjected to the same open wound and allowed to leave as they were.

Two days after finishing continuous intravenous administration of ginsenoside Rb₁ or physiological saline alone (i.e. on day 5 after making the open wound), the animals were anesthetized with pentobarbital and fixed transcardialy with perfusion of 0.1 M phosphate buffer containing 4% paraformaldehyde. Thereafter, the skin tissue including the open wound was dissected out, post-fixed and embedded in paraffin. Paraffin sections with 5 µm thickness were cut and stained with hematoxylin-eosin (HE). Result is shown in Fig. 3. Fig. 3A shows a case of ginsenoside Rb₁ administration and Fig. 3B shows a case of physiological saline administration. "i" indicates incrustation or eschar, "ep" indicates stratified squamous epithelium of epidermis, and "bv" indicates blood vessel.

As shown in Fig. 3A, in the ginsenoside Rb₁-administered case, on day 5 after making the open wound, obvious epidermis (stratified squamous epithelial tissue) was regenerated and reconstructed under the eschar, and thick regenerated blood vessels or generated blood vessels filled with erythrocytes were distributed just beneath the epidermis (stratified squamous epithelium). Further, relatively thin blood vessels, which dissociated from the thick blood vessel, were observed densely in the connective tissue of the dermis or in the subcutaneous tissue. On the other hand, as shown in Fig. 3B, in the physiological saline-administered case, even on day 5 after making the open wound, regeneration of the epidermis (stratified squamous epithelium) under eschar was extremely incomplete, and regenerated blood vessels just beneath the very thin epidermis was obviously thin as compared with the case of ginsenoside Rb₁ administration. For that reason, a small number of extremely thin blood vessels was observed in the subepidermal connective tissues, which might be scar or granulation in future. Consequently, it was elucidated that as a result of intravenous administration of ginsenoside Rb₁, regeneration and reconstruction of skin tissue were obviously promoted, and regeneration, generation and reconstruction of once ruptured and excised blood vessels were also facilitated by intravenous administration of ginsenoside Rb₁.

According to the above example, ginsenoside Rb₁ can be said to exhibit superior skin tissue regeneration and reconstruction-promoting action, even if intravenously administered before making open wound or intravenously administered after making open wound.

### Example 4 (Prevention, therapy or treatment of incomplete suture by ginsenosides, especially ginsenoside Rb₁)

Since patients with diabetes mellitus, immunodeficiency diseases, malnutrition, malignant tumor, etc. or the elders develop frequently incomplete suture after surgical operation, it is thought essential to prevent it in advance. Consequently, in addition to the usual therapy, if ginsenosides, especially ginsenoside Rb₁, are intravenously infused once for every day or continuously at doses of 0.02 mg or more/day, preferably 0.2 mg or more/day, more preferably 10 mg or more/day for consecutive days preoperatively or postoperatively, incidence of incomplete suture after the operation is significantly decreased and surgical wound is recovered quickly. Further, in combination with intravenous administration of ginsenosides, especially ginsenoside Rb₁, low concentrations of ginsenoside Rb₁ can be admixed to optional base such as water-soluble base, ointment base, fat-soluble base, etc. to prepare an agent(s) for external or topical application to skin (cream, gel, spray or ointment, etc.), and it can be applied to the local region of the surgical wound and its penumbra until wound is cured. Further, ginsenosides, especially ginsenoside Rb₁, may preferably be administered locally during surgical operations. In that occasion, the admixed amount of ginsenoside Rb₁ to the base and agent for local administration is adjusted so that the extracellular fluid concentrations of ginsenosides, especially ginsenoside Rb₁, in the local region are kept at 1 ng/ml or less, preferably 10 pg/ml or less, more preferably 100 fg/ml or less. Namely, the amount of ginsenoside Rb₁ to the agent(s) for local administration is preferably set at levels less than 0.001% by weight.

### Example 5 (Therapy or treatment of radiation injury or burn by ginsenosides, especially ginsenoside Rb₁)

In patients with severe radiation injury and patients with severe burn, skin tissues are broadly degenerated and exfoliated, and no sufficient effect can be obtained even by transplantation of cultured skin sheets and vital prognosis of the patients may be jeopardized. For such patients, in order to promote skin tissue regeneration from the transplanted cultured sheets and regeneration of lesioned tissue by division, proliferation, lesion-oriented migration and differentiation of cells in the intact skin tissue, ginsenosides, especially ginsenoside Rb₁, at a dose of 0.02 mg or more/day, preferably 0.2 mg or more/day, more preferably 10 mg/day or more, are intravenously infused once for every day or in a continuous manner for consecutive days until improvement of the symptoms is observed. Of course, in combination with intravenous administration of ginsenosides, especially ginsenoside Rb₁, ginsenosides, especially ginsenoside Rb₁, can be admixed to water-soluble base or fat-soluble base to prepare an agent(s) for external or topical application to skin (cream, gel, lotion, spray or ointment, etc.), and it can be applied to the lesioned skin region and its penumbra until the lesion is improved and cured. In that occasion, the admixed amount of ginsenosides, especially ginsenoside Rb₁, to the base is adjusted so that the extracellular fluid concentrations of ginsenosides, especially ginsenoside Rb₁, in the local lesion are kept at 1 ng/ml or less, preferably 10 pg/ml or less, more preferably 100 fg/ml or less. The amount of ginsenosides, especially ginsenoside Rb₁, in the agent(s) for external or topical application to skin is preferably set at levels less than 0.001% by weight. If radiation injury or burn is relatively mild, the above-mentioned agent(s) for external application to skin may also be only applied.

### Example 6 (Prevention, therapy or treatment of bedsore by ginsenosides, especially ginsenoside Rb₁)

Bedsore or decubitus of the bedridden patients and elders is a skin disease which may deteriorates the systemic condition and QOL (quality of life). At early stages of bedsore, flare of skin lesion is observed, and even at this time, there are neither agents for external application, which are applied on the local lesion and its penumbra to exhibit effectiveness and efficacy, nor agents for intravenous administration to exhibit effectiveness and efficacy. This is the large problem in the dermatological field. Of course, treatment of decubitus lesion with defected skin tissue is extremely difficult.

Ginsenosides, especially ginsenoside Rb₁, can be admixed to water-soluble base or fat-soluble base with or without glucose to prepare an agent(s) or a preparation(s) for external or topical application to skin (cream or ointment). The preparation(s) was continuously applied on local decubital lesion and its penumbra until the decubital lesion was cured or reduced. The concentrations of ginsenosides, especially ginsenoside Rb₁, in the agent(s) or preparation(s) for external or topical application to skin are preferably set at levels less than 0.001% by weight. In that occasion, the admixed amount of ginsenosides, especially ginsenoside Rb₁, in the base is adjusted so that the extracellular fluid concentrations of ginsenosides, especially ginsenoside Rb₁, in the local region are kept at 1 ng/ml or less, preferably 10 pg/ml or less, more preferably 100 fg/ml or less. If necessary, intravenous administration of ginsenosides, especially ginsenoside Rb₁, is used in combination as described in example 4 and example 5. As described in Japanese Patent Appln. No. Hei 10-365560, PCT/JP99/02550 (Brain cell or nerve cell-protective agents comprising ginsenoside Rb₁), ginsenoside Rb₁ suppresses expansion of decubital lesion through a potent cytoprotective action, and exhibits an excellent therapeutic effect against once occurred defect of skin tissue in patients with decubitus by promoting skin tissue regeneration and/or reconstruction.

### Example 7 (Therapy of peptic ulcer by ginsenosides, especially ginsenoside Rb₁)

With regard to means for pharmacotherapy of gastric ulcer and duodenal ulcer, H2 receptor inhibitors, proton pump inhibitors, gastric mucosa-protecting agents, etc. are mainly used, however even if ulcer lesion is temporarily cured by a drug, if administration of the drug is terminated, frequently ulcer lesion recurs. Further, ulcer lesion is frequently observed in Crohn's disease and ulcerative colitis, which are specified as intractable diseases, and are causes for deteriorating the patients' prognosis. After onset of gastric ulcer, duodenal ulcer, ulcerative colitis or Crohn's disease, intravenous infusion, intrarectal administration, ear-drop administration or endoscopic extramucosal administration of ginsenosides, especially ginsenoside Rb₁, is conducted as early as possible while applying conventional therapeutic means, then the treatment is continued until cure or improvement of the lesion is confirmed endoscopically.

### Example 8 (Therapy of diabetic skin ulcer by ginsenosides, especially ginsenoside Rb₁)

Diabetic skin ulcer is an intractable disease accompanied by blood flow failure and skin tissue defect in lesion, however if ginsenosides, especially ginsenoside Rb₁, having action for promoting regeneration and/or reconstruction of blood vessels and skin tissues are intravenously administered, locally infused or externally applied to the skin, effectiveness can be obtained. Namely, for patients with diabetic skin ulcer, ginsenosides, especially ginsenoside Rb₁, in a dose of 0.02 mg or more/day, preferably 0.2 mg or more/day, more preferably 10 mg or more/day, are intravenously infused once for every day or in a continuous manner for consecutive days, while applying conventional therapeutic means. If necessary, an agent(s). for external application to skin comprising ginsenoside Rb₁ may be applied to the lesion and its penumbra as described in example 4, or physiological saline solution comprising ginsenosides, especially ginsenoside Rb₁, may be infused or injected into the local lesion. In that occasion, the amount of ginsenosides, especially ginsenoside Rb₁, admixed in the base or the amount of local injection of physiological saline solution (solvent) comprising ginsenoside Rb₁ is adjusted so that the extracellular fluid concentrations of ginsenosides, especially ginsenoside Rb₁, in the lesion are kept at 1ng/ml or less, preferably 10 pg/ml or less, more preferably 100 fg/ml or less.

### Example 9 (Therapy of open wound or skin defect by an agent for external application to skin comprising ginsenoside Rb₁)

Male Wistar rats weighing about 300 g were used. Punch biopsies with diameter 6 mm were performed in the dorsal region of animals after shaving their hair under inhalation anesthesia to make 3 regions of open wound. Among them, 0.1 g of ophthalmic white Vaseune (Propet) containing 0.01% by weight or 0.001% by weight of ginsenoside Rb₁ was spread onto two regions once for every day, and onto the remaining open wound, the equal amount of ophthalmic white Vaseline (propet) alone was spread. On day 9 after making open wound, the skin including wound regions was photographed. Further, we (the present inventors) have examined the effect of extracutaneous spread of 0.0001% by weight, 0.00001% by weight or 0.000001% by weight of ginsenoside Rb₁ on open wound by the same procedure. Experimental animals were euthanized by anesthetia immediately before photographing, then wound regions were dissected out after photographing or after dissecting out wound regions, photographing was performed. Thereafter, the tissues containing wound region tissues were stored in the fixative. Result is shown in Fig. 5 and Fig. 6.

In Fig. 5, the first wound from the top is a case of external administration (external spread) of only propet after making the open wound, showing obvious red colored open wound (in the photograph, black open wound). In Fig. 5, the second wound from the top is a case of external spread (external administration) of propet containing 0.001% by weight of ginsenoside Rb₁, and the open wound area is slightly reduced as compared with the first wound which is externally spread or administered with only propet. The third open wound which is externally spread or administered with propet containing 0.01% by weight of ginsenoside Rb₁ shows no difference as compared with the control of the first wound. As shown in Fig. 6, on the second and the third from the top, propet containing 0.00001% by weight (10⁻⁵% by weight) or 0.000001% by weight (10⁻⁶% by weight) of ginsenoside Rb₁ shows superior effectiveness as compared with propet containing 0.0001% by weight of ginsenoside Rb₁. Further, as a result of extracutaneous administration of 0.000001% by weight of ginsenoside Rb₁, obvious hair restoration or hair growth from the regenerated open wound was observed. This demonstrates that in case that an agent(s) for external application to skin comprising low concentrations of ginsenosides, especially ginsenoside Rb₁, is externally spread or externally sprayed on open wound, effectiveness and efficacy almost identical with those of continuous intravenous administration of low dosages of ginsenosides, especially ginsenoside Rb₁, can be obtained.

### Example 10 (Therapy of morsus of human oral mucosa by ginsenoside Rb₁: No. 1)

Next, one of the present inventors (Sakanaka) had confirmed by himself whether propet containing low concentrations of ginsenoside Rb₁ was effective for morsus of mouth mucosa or not. On May 2nd, 2000, at about 2:00 p.m. after dental treatment, Sakanaka had started to have late lunch before awakening from infiltration anesthesia of the left third division of the trigeminal nerve and periodontal anesthesia due to heavy hunger. He had bitten himself three times his own left lip mucosa and he felt iron taste (blood) in his oral cavity. As a result of confirmation of his morsus by a mirror, he found at least five regions of erosion or defect of mouth mucosa, further hematoma was found at one spot. Since he concluded that if the morsus was allowed to leave as it was, aphthous stomatitis would be developed as a complication to require a week to ten days for complete cure, a small amount of propet containing a low concentration (0.00001% by weight) of ginsenoside Rb₁, which had been confirmed to show effectiveness and efficacy by animal experiments of the present inventors, was topically or externally applied to the five regions with erosion or defect of oral mucosa and to one hematoma region. External or topical application was performed before and after meal and before and after eating between meals. Namely, propet containing 0.00001% by weight of ginsenoside Rb₁ was applied externally or topically onto morsus regions of the labial mucosa 6 - 10 times a day. A photograph of the labial mucosa at 96 hours after morsus is shown in Fig. 7.

As shown in the photograph of Fig. 7, although hematoma remained at 96 hours after morsus as indicated in the white arrow, the morsus regions of labial mucosa (i.e. erosive or defected mouth mucosa) indicated by black arrowheads were only slightly flared with almost complete epithelization, and wound was thought to be obviously cured. Furthermore, after applying externally or topically propet containing 0.00001% by weight of ginsenoside Rb₁ to the morsus regions, pain in the wounded regions was markedly reduced.

### Example 11 (Therapy of morsus of human oral mucosa by ginsenoside Rb₁: No. 2)

Next, one of the inventors of the present invention (Sakanaka) had bitten again the left lower labial mucosa on May 26, 2000 during lunch, and propet containing 0.00001% by weight of ginsenoside Rb₁ was applied externally or topically onto the morsus region. A photograph just after morsus is shown on the left side of Fig. 8, and a photograph at 72 hours after morsus is shown on the right side of Fig. 8.

As shown in Fig. 8, if low concentrations of ginsenoside Rb₁ was externally or topically applied onto the mucosa, morsus was rapidly cured without developing aphthous stomatitis as a complication.

### Example 12 (Promotion of generation and/or regeneration of cuttings of pothos by ginsenoside Rb₁)

We have examined whether ginsenosides, especially ginsenoside Rb₁, could promote generation, regeneration or reconstruction of not only skin tissue or mouth mucosal tissue but also plant tissues. For that purpose, one of foliage plants, pothos (Epipremunum aureum, golden pothos) was selected. Six cuttings resembled with each other from the parent plant of pothos in the room of one of the inventors (Tanaka) were collected. Among them, 3 cuttings were cultured by hydroponics in water alone and the remaining three were cultured in water containing ginsenoside Rb₁ at a concentration of 100 fg/ml. A photograph of cuttings on day 13 of culture is shown in Fig. 9. The left photograph in Fig. 9 is the cutting (stem and branch of pothos) cultured with only water and the right photograph in Fig. 9 is the cutting cultured with water containing ginsenoside Rb₁ at a concentration of 100 fg/ml. In case that the cutting of pothos is cultured with water containing the low concentration of ginsenoside Rb₁ (100 fg/ml) in hydroponics, growth of root is promoted.

Next, we have further continued cultivation of the above cuttings with hydroponics and on day 22, again photographs were taken. Result is shown in Fig. 10. The left photograph in Fig. 10 is the cuttings of pothos cultured with only water for 22 days and the right photograph in Fig. 10 is the cuttings cultured with water containing the low concentration of ginsenoside Rb₁ (100 fg/ml). Waters with or without ginsenoside Rb₁ for hydroponics were exchanged once a week.

As shown in Fig. 10, when the cuttings were cultured with water containing the low concentration of ginsenoside Rb₁ (100 fg/ml) for 22 days, many roots were generated or regenerated to grow to contact glass vessel for hydroponics. Consequently, as the results of the present experiments, it was demonstrated that low concentrations and low dosages of ginsenosides, especially ginsenoside Rb₁, promoted generation, regeneration or reconstruction of not only skin tissue and human mouth mucosal tissue but also plant tissues.

### Example 13 (Generation and regeneration-promoting effect of a crude saponin fraction of ginseng on cuttings of pothos)

Next, we have examined whether crude saponin fraction of ginseng can promote, as same manner in ginsenoside Rb₁, generation, regeneration or reconstruction of the cutting of pothos. For that purpose, two cuttings resembled with each other from the parent plant of pothos were collected. Among them, one cutting was cultured with water alone and the remaining one was cultured in water containing a crude saponin fraction of ginseng at a concentration of 1450 fg/ml. A photograph of cuttings on day 14 of culture is shown in Fig. 11. The left photograph in Fig. 11 is the cutting cultured with only water and the right photograph in Fig. 11 is the cutting cultured with water containing the crude saponin fraction of ginseng (1450 fg/ml). In case that the cutting of pothos was cultured with water containing the low concentration of the crude saponin fraction of ginseng (1450 fg/ml) in hydroponics, as compared with cultivation only with water, growth of root was promoted. Namely, the crude saponin fraction(s) of ginseng, in the same manner as in ginsenoside Rb₁, promotes generation, regeneration or reconstruction of plant tissues. Quite naturally, a ginseng extract(s) and ginseng containing crude saponin fraction are also to have the same action. Namely, it can be said that ginseng, a ginseng extract(s), a crude saponin fraction(s) of ginseng and ginsenosides, especially ginsenoside Rb₁, can promote regeneration, generation and/or reconstruction of all vital tissues or viable tissues (animal and plant tissues).

### Example 14 (Therapy of open wound by propet comprising 10⁻⁴% by weight - 10⁻⁸% by weight of ginsenoside Rb₁)

The punch biopsy with diameter 6 mm was performed in the dorsal region of animals under inhalation anesthesia to make open wound. Thereafter, 0.1 g of propet containing ginsenoside Rb₁ at a concentration of 0.0001% by weight (10⁻⁴% by weight), 0.00001% by weight (10⁻⁵% by weight), 0.000001% by weight (10⁻⁶% by weight), 0.0000001% by weight (10⁻⁷% by weight) or 0.00000001% by weight (10⁻⁸% by weight), respectively, was applied externally or topically once a day for 9 days onto each open wound. The equal amount of propet alone was applied externally or topically in the control animals. Then, immediately after euthanasia by anesthetization, the skin including the open wound was dissected out and photographed. The collected skin tissue was preserved in a fixative. Results are shown in Fig. 12.

As shown in Fig. 12, even propet containing from 10⁻⁶% by weight to 10⁻⁸% by weight of ginsenoside Rb₁ (i.e. concentration of ginsenoside Rb₁ from 10 ng/g to 100 pg/g) was externally or topically applied to the open wounds, as same in propet containing 10⁻⁵% by weight of ginsenoside Rb₁, wound healing was obviously promoted as compared with the open wounds externally or topically applied with propet alone. Consequently, in case that ginsenosides, especially ginsenoside Rb₁, are used as an agent(s) for external or topical application to skin, concentration thereof in the agent(s) for external or topical application can be set preferably around 10⁻⁸% by weight or less. Consequently, in case that ginsenosides, especially ginsenoside Rb₁, a crude saponin fraction(s) of ginseng, a ginseng extract(s) or ginseng is used as a composition(s) for cosmetics or a composition(s) for health drug, concentration thereof in cosmetics, an agent(s) for chemical peeling or health drug should be set at levels less than 0.001% by weight, preferably at 0.00001% by weight (10⁻⁵% by weight) or less, more preferably at 0.00000001% by weight (10⁻⁸% by weight) or less.

In the experimental cases hereinbefore explained, the area of open wound applied topically or externally with propet alone is set as a denominator and the area of open wound applied topically or externally with propet containing ginsenoside Rb₁ at concentrations from 10⁻⁴% by weight to 10⁻⁸% by weight, respectivery, is set as a numerator, and ratio thereof is calculated. Result is shown in Fig. 13. As shown in Fig. 13, in case that low concentrations of ginsenoside Rb₁ were externally or topically applied onto open wound, wound healing was significantly promoted. Statistical analysis was conducted by ANOVA + Scheffe's post hoc test. *: P < 0.05, **: P < 0.01. Since the topical or external application of ginsenoside Rb₁ at concentrations around 10⁻⁸% by weight reduces area of the open wound to about 1/4 of the control group, the volume of the open wound appears to be reduced to about 1/8 by external or topical administration of low concentrations of ginsenoside Rb₁.

### Example 15 (Therapy of oral mucosal burn or aphthous stomatitis by propet containing low concentrations of ginsenoside Rb₁)

When hot food or drink is put into oral cavity in haste, lingual mucosa, labial mucosa, hard palate mucosa or soft palate mucosa is subjected to burn that causes frequently exfolition of mucosal epithelia and erosion or flare of mucosa. Of course, such burn is usually accompanied with pain. Further, after morsus or burn of oral mucosa, or due to an unknown cause(s), aphthous stomatitis occurs frequently, as a result, pain is always felt in the oral cavity for about 1 week to 10 days and it is quite uneasy to take meal. If ointment containing or comprising ginsenoside Rb₁ at concentrations less than 10⁻³% by weight, preferably at concentrations of 10⁻⁵% by weight or less, more preferably at concentrations of 10⁻⁷% by weight or less, is applied to the local lesion of burn or aphthous stomatitis in oral mucosa 3 - 10 times a day, especially before or after the meal, pain will be reduced and mucosal defect or wound healing is promoted. With regard to base used for ginsenosides, especially ginsenoside Rb₁, in an agent(s) for external or topical application to oral mucosa, the same base as that of dexaltin ointment or kenalog can be used. The optimum concentration of ginsenosides, especially ginsenoside Rb₁, in an agent(s) for external or topical application to oral mucosa appears to be 10 - 1000 times higher than that of ginsenosides, especially ginsenoside Rb₁, in an agent(s) for external or topical application to skin.

### Example 16 (Therapy of open wound by an agent for external or topical application to skin comprising dihydroginsenoside Rb₁)

Next, we have examined whether low concentrations or low dosages of ginsenoside derivatives can promote tissue regeneration and/or reconstruction in the same manner as in ginsenoside Rb₁. For that purpose, we have selected dihydroginsenoside Rb₁ as one of ginsenoside derivatives and examined the open wound healing effect of the said compound. Details of dihydroginsenoside Rb₁ is described in the specification of PCT/JP00/04102 (Brain cell or nerve cell protecting agents comprising ginseng). Punch biopsy with diameter 6 mm was performed at 5 places in the dorsal region of rats (n = 4) under inhalation anesthesia to make open wound. Thereafter, 0.1 g of propet containing dihydroginsenoside Rb₁ at a concentration of 0.0001% by weight (10⁻⁴% by weight), 0.00001% by weight (10⁻⁵% by weight), 0.000001% by weight (10⁻⁶% by weight) or 0.0000001% by weight (10⁻⁷% by weight), respectively, was applied topically or externally, once a day for 9 days onto each open wound. Propet alone was applied externally or topically in the control group. Then, immediately after euthanasia by anesthetization, the skin including the open wound was dissected out and photographed. The collected skin tissue was preserved in a fixative. Results are shown in Fig. 14.

As shown in Fig. 14, when propet containing dihydroginsenoside Rb₁ at concentrations from 0.00001% by weight (10⁻⁵% by weight) to 0.0000001% by weight (10⁻⁷% by weight)(i.e. concentration of dihydroginsenoside Rb₁ from 100 ng/g to 1 ng/g) was externally or topically applied to the open wounds, wound healing was obviously promoted as compared with the open wounds topically or externally applied with propet alone. In case of external or topical administration of the low concentrations of dihydroginsenoside Rb₁, obvious hair growth was observed in wound healing region. Consequently, in case that ginsenoside derivatives, especially dihydroginsenoside Rb₁, are used as an agent(s) for external or topical application to skin, concentration thereof in the agent(s) for external or topical application is thought to be set preferably around 0.0000001% by weight (10⁻⁷% by weight) or less. Consequently, in case that ginsenoside derivatives, especially dihydroginsenoside Rb₁, is also used as a composition(s) for cosmetics, concentration thereof in cosmetics or health drug should be set at levels less than 0.001% by weight, preferably at levels of 0.00001% by weight (10⁻⁵% by weight) or less, more preferably at levels of 0.0000001% by weight (10⁻⁷% by weight) or less.

In the experimental cases hereinbefore explained, the area of open wound (flare region) applied externally only with propet is set as a denominator and the area of open wound applied externally with propet containing dihydroginsenoside Rb₁ at concentrations from 0.0001% by weight (10⁻⁴% by weight) to 0.0000001% by weight (10⁻⁷% by weight) is set as a numerator, and ratio thereof is calculated. Result is shown in Fig. 15. As shown in Fig. 15, external or topical administration of dihydroginsenoside Rb₁ at concentrations of 0.00001% by weight (10⁻⁵% by weight) or less to the open wound promoted regeneration and/or reconstruction of skin and facilitated significantly wound healing. Especially, the fact that external or topical administration of dihydroginsenoside Rb₁ at concentrations of 0.00001% by weight (10⁻⁵% by weight) or less, i.e. 100 ng/g or less or 100 ng/ml or less of dihydroginsenoside Rb₁, reduced significantly the open wound, strongly supports that when the extracellular fluid concentrations of dihydroginsenoside Rb₁ in lesioned tissues are 100 ng/ml or less, generation, regeneration or reconstruction of vital or viable tissues is promoted. Statistical analysis was conducted according to ANOVA + Fisher's PLSD. *: P < 0.05.

### Example 17 (Protection of cultured nerve cells by dihydroginsenoside Rb₁)

Next, we have performed experiments using cultured nerve cells in order to confirm that dihydroginsenoside Rb₁ had the same effectiveness, efficacy and usages as those of ginsenoside Rb₁.

We (Sakanaka and Tanaka) had reported that when cultured nerve cells or neurons were exposed to sodium nitroprusside for a short time, apoptosis or apoptosis-like cell death of nerve cells is induced (Toku, K. et al., J. Neurosci. Res., 53, 415-425, 1998). Using this culturing experimental system, we have already found that ginsenoside Rb₁ at extracellular fluid concentrations of 1 ng/ml or less inhibits apoptosis or apoptosis-like cell death of nerve cells (Japanese Patent Appln. No. Hei 10-365560, Brain cell or nerve cell-protective agents comprising ginsenoside Rb₁). We have examined the nerve cell-protective action of dihydroginsenoside Rb₁ using the same experimental system.

Nerve cells were isolated from cerebral cortices of fetal rats on gestation day 17 by using trypsin EDTA and seeded onto a 24 well plate(s) coated with poly-L-lysine. After incubating the cells in Dulbecco's modified Eagle's medium (DMEM) containing 10% fetal calf serum for 16 hours, the culture medium was replaced by serum free medium for nerve cell culture containing insulin, transferrin, etc. and the cells were further incubated for 3 or 4 days. On day 3 or 4 of incubation, sodium nitroprusside (SNP) at the concentration of 300 µM was added to the neuronal culture and the cells were incubated for 10 minutes. Thereafter, the culture medium was replaced by Eagle's minimum essential medium (MEM) containing dihydroginsenoside Rb₁ (0 - 1 ng/ml) and bovine serum albumin. Sixteen hours after SNP loading, nerve cells were lysed with Laemmli's sample buffer for electrophoresis, and polyacrylamide electrophoresis was performed. Electrophoresed proteins were transferred to nitrocellulose membrane, and immunoblotting was performed by using antibody against neuron specific protein MAP 2. In order to quantify survival rate of nerve cells, immunostained MAP2 band was analyzed with densitometry. Results are shown in Fig. 16 and Fig. 17. For reference, NMR chart of dihydroginsenoside Rb₁ is shown in Fig. 18 (400 MHz, CD₃OD).

Fig. 16 is a photograph showing result of immunoblotting of microtuble-associated protein 2 (MAP 2) in place of drawing. The first lane from left indicates the result of control nerve cells, showing a clear MAP 2 band (i.e. a band of nerve cell marker). When SNP treatment was performed, a large number of nerve cells entered apoptosis or apoptosis-like cell death and the band of MAP 2 was clearly weakened as observed in the second lane from left. When dihydroginsenoside Rb₁ is added to the culture medium at the concentrations from 0.01 fg/ml (lane 3) to 1 ng/ml (lane 7), apoptosis or apoptosis-like cell death of neurons caused by SNP is obviously inhibited, as a result, intense bands of MAP 2, which is a survival marker of nerve cells, was observed.

The above-mentioned MAP2 immunoblotting experiments were repeated 5 times and the results were analyzed by densitometry (Fig. 17). As shown in Fig. 17, dihydroginsenoside Rb₁ at the concentrations from 0.01 fg/ml to 1 ng/ml significantly inhibited apoptosis or apoptosis-like cell death of nerve cells or neurons. Namely, dihydroginsenoside Rb₁ exhibits preferable effects on cells, especially nerve cells, in the slightly wider concentration range than that of ginsenoside Rb₁. Consequently, ginsenoside derivatives, especially dihydroginsenoside Rb₁, can exhibit a superior cytoprotective action through inhibition of apoptosis or apoptosis-like cell death, when its extracellular fluid concentrations in lesioned tissues are 100 ng/ml or less, preferably 10 pg/ml or less, more preferably 0.0001 fg/ml - 100 fg/ml. *: P < 0.001. **: P < 0.0001.

Judging from the experimental results hereinbefore that the agent for external or topical application to skin comprising 0.00001% by weight (10⁻⁵% by weight) of dihydroginsenoside Rb₁ shows superior open wound-healing effect, ginsenoside derivatives, especially dihydroginsenoside Rb₁, exhibits regenerative and reconstructive action on vital or viable tissues, when extracellular fluid concentrations thereof in the lesioned tissues are 100 ng/ml or less, preferably 10 pg/ml or less, more preferably 0.0001 fg/ml - 100 fg/ml.

### Industrial Applicability

The present invention provides a pharmaceutical composition(s); a composition(s) for external or topical application to skin, a composition(s) for external or topical application to mucosa, a health drug composition(s), a composition for chemical peeling, a cosmetic composition(s), a fertilizer composition(s), a feed composition(s), a composition(s) for growth regulation, or a composition(s) for hair restoration, hair growth or pilatory with extremely low side effects comprising ginsenosides, especially ginsenoside Rb₁, which are known as components of ginseng. In the present invention, by using ginsenosides, especially ginsenoside Rb₁, at lower concentrations than before, excellent actions thereof for promoting regeneration and/or reconstruction of viable or vital tissues have been newly invented. These excellent actions could not be found out in case of the conventional composition containing ginsenoside Rb₁. Low concentrations and/or low dosages of ginsenosides, especially ginsenoside Rb₁, are useful for prevention, therapy or treatment of all organic diseases causing histopathological changes and for cultivation, growth or farming of farm products or marine products, through promoting regeneration and/or reconstruction of vital or viable tissues. Further, in the present invention, it is found that ginsenoside derivatives, especially dihydroginsenoside Rb₁, ginseng, a ginseng extract(s) or a crude saponin fraction(s) of ginseng can exhibit the same effectiveness, efficacy and usages as those of ginsenosides, especially ginsenoside Rb₁. The effectiveness, efficacy and usages of ginsenosides, especially ginsenoside Rb₁, described in the present invention can be applied uniformly to prosaposin-related peptides (JP Appln. No. Hei 11-185155) and the other compounds showing brain cell-protective action by intracerebroventricular administration.

## Claims

1. A pharmaceutical composition(s) or a veterinary drug composition(s) for prevention, treatment or therapy of organic diseases causing histopathological changes of the vital or viable tissues comprising ginsenosides, metabolites thereof or salts thereof.

2. The pharmaceutical composition(s) or the veterinary drug composition(s) according to claim 1, wherein the content of ginsenosides, metabolites thereof or salts thereof is less than 0.001% by weight in the whole composition.

3. The pharmaceutical composition(s) or the veterinary drug composition(s) according to claim 1 or claim 2, wherein the extracellular fluid concentrations of ginsenosides, metabolites thereof or salts thereof in lesioned tissues are adjusted to 1 ng/ml or less.

4. The pharmaceutical composition(s) or the veterinary drug composition(s) according to claim 3, wherein the extracellular fluid concentrations of ginsenosides, metabolites thereof or salts thereof in lesioned tissues are 0.01 - 100 fg/ml or 1 - 10,000 fg/ml.

5. The pharmaceutical composition(s) or the veterinary drug composition(s) according to any one of claims 1 - 4, wherein ginsenosides, metabolites thereof or salts thereof are ginsenoside Rb₁ or dihydroginsenoside Rb₁.

6. The pharmaceutical composition(s) or the veterinary drug composition(s) according to any one of claims 1 - 5, wherein ginsenosides, metabolites thereof or salts thereof are present in an extract(s) of a natural product(s), fraction(s) thereof or in purified fraction(s) thereof.

7. The pharmaceutical composition(s) or the veterinary drug composition(s) according to any one of claims 1 - 4, wherein ginsenosides, metabolites thereof or salts thereof are chemically modified derivatives from the natural product(s) by chemical means.

8. The pharmaceutical composition(s) or the veterinary drug composition(s) according to claim 7, wherein the chemical means is reduction.

9. The pharmaceutical composition(s) or the veterinary drug composition(s) according to claim 7 or claim 8, wherein the chemically modified derivative is dihydroginsenoside Rb₁.

10. The pharmaceutical composition(s) or the veterinary drug composition(s) according to claim 6, wherein the extract(s) of the natural product(s), fraction(s) thereof or purified fraction(s) thereof is ginseng, a ginseng extract(s) or a crude saponin fraction(s) of ginseng.

11. The pharmaceutical composition(s) or the veterinary drug composition(s) according to claim 10, wherein the content of the ginseng, the ginseng extract(s) or the crude saponin fraction(s) of ginseng is less than 0.001 % by weight.

12. The pharmaceutical composition(s) or the veterinary drug composition(s) according to claim 10 or claim 11, wherein the extracellular fluid concentrations of the ginseng, the ginseng extract(s) or the crude saponin fraction(s) of ginseng in lesioned tissues are adjusted to 14.5 ng/ml or less.

13. The pharmaceutical composition(s) or the veterinary drug composition(s) according to claim 12, wherein the extracellular fluid concentrations of the ginseng, the ginseng extract(s) or the crude saponin fraction(s) of ginseng in lesioned tissues are 0.145 - 1,450 fg/ml or 14.5 - 145,000 fg/ml.

14. A preparation(s) for intravenous administration or intravenous injection comprising the pharmaceutical composition(s) or the veterinary drug composition(s) according to any one of claims 1 - 13.

15. The preparation(s) for intravenous administration or intravenous injection according to claim 14, wherein the preparation(s) for intravenous administration or intravenous injection is a preparation(s) for single injection or a preparation(s) for continuous intravenous administration.

16. A preparation(s) for external or topical application to skin comprising the pharmaceutical composition(s) or the veterinary drug composition(s) according to any one of claims 1 - 13.

17. A preparation(s) for external or topical application to mucosa comprising the pharmaceutical composition(s) or the veterinary drug composition(s) according to any one of claims 1 - 13.

18. The pharmaceutical composition(s) or the veterinary drug composition(s) according to any one of claims 1 - 17, wherein the histopathological changes are caused by wound.

19. The pharmaceutical composition(s) or the veterinary drug composition(s) according to claim 18, wherein the wound is incised wound, open wound, morsus or defect.

20. The pharmaceutical composition(s) or the veterinary drug composition(s) according to claim 18 or claim 19, wherein the wound is incomplete suture after surgical operations.

21. The pharmaceutical composition(s) or the veterinary drug composition(s) according to any one of claims 1 - 20, wherein the prevention, the treatment or the therapy of the vital or viable tissues with histopathological changes comprises promoting regeneration and/or reconstruction of cells.

22. The pharmaceutical composition(s) or the veterinary drug composition(s) according to any one of claims 1 - 20, wherein the prevention, the treatment or the therapy of the vital or viable tissues with histopathological changes is caused by regeneration and/or reconstruction of said tissues.

23. The pharmaceutical composition(s) or the veterinary drug composition(s) according to any one of claims 1 - 22, wherein the composition(s) comprises promoting cure of organic diseases.

24. The pharmaceutical composition(s) or the veterinary drug composition(s) according to any one of claims 1 - 23, wherein the vital or viable tissues are the cutaneous tissue or the mucosal tissues including the mouth mucosa.

25. The pharmaceutical composition(s) or the veterinary drug composition(s) according to claim 24, wherein prevention, treatment or therapy of the cutaneous tissue or the mucosal tissues including the mouth or oral mucosa with histopathological changes is caused by regeneration and/or reconstruction of epidermis, epithelium, dermis (corium), dermal papillae, subcutaneous tissue, connective tissues, lamina propria, muscular tissues, salivary glands, mixed glands, sweat glands, sebaceous glands, mucous glands, serous glands, hair papillae, hair follicles, pilomotor muscles, blood vessels or peripheral nerves in the cutaneous tissue or mucosal tissues including the mouth or oral mucosa.

26. The pharmaceutical composition(s) or the veterinary drug composition(s) according to claim 24 or claim 25, comprising promoting epithelization of the cutaneous tissue or the mucosal tissues including the mouth or oral mucosa with histopathological changes.

27. The pharmaceutical composition(s) or the veterinary drug composition(s) according to any one of claims 24 - 26, wherein prevention, treatment or therapy of the cutaneous tissue or the mucosal tissues including the mouth or oral mucosa with histopathological changes is caused by regeneration and/or reconstruction of epidermal cells, epidermal keratinocytes, epithelial cells, Merkel cells, melanocytes, Langerhans cells, keratinized cells, hornified cells, cornified cells, stem cells, mesenchymal cells, fibroblasts, sebaceous gland cells, salivary gland cells, myoepithelial cells, sweat gland cells, smooth muscle cells, mucous gland cells, serous gland cells, mixed gland cells, muscle cells, vascular endothelial cells, adipocytes, hair follicular cells, collagen fibers, elastic fibers, reticular fibers or extracellular matrix (matrices) in the cutaneous tissue or mucosal tissues including the mouth or oral mucosa.

28. The pharmaceutical composition(s) or the veterinary drug composition(s) according to any one of claims 24 - 26, wherein the prevention, the treatment or the therapy of the cutaneous tissue or the mucosal tissues including the mouth or oral mucosa with histopathological changes is caused by hair growth or hair nourishment in the cutaneous tissue with the histopathological changes.

29. The pharmaceutical composition(s) or the veterinary drug composition(s) according to any one of claims 24 - 26, wherein the prevention, the treatment or the therapy of the cutaneous tissue or the mucosal tissues including the mouth or oral mucosa with histopathological changes is for prevention, treatment or improvement of senile symptoms of the skin or the mucosa.

30. The pharmaceutical composition(s) or the veterinary drug composition(s) according to any one of claims 1 - 29, wherein the vital or viable tissues are organs or tissues for transplantation.

31. A composition(s) for external application to skin or a composition(s) for external application to mucosa, comprising containing ginsenosides, metabolites thereof or salts thereof at concentrations less than 0.001 % by weight in the composition.

32. The composition(s) for external application to skin according to claim 31, wherein the composition(s) for external application to skin is a cosmetic composition(s).

33. The composition(s) for external application to skin according to claim 31, wherein the composition(s) for external application to skin is a composition(s) for chemical peeling.

34. The composition(s) for external application to skin according to claim 31, wherein the composition(s) for external application to skin is a composition(s) for hair growth and/or hair nourishment.

35. The composition(s) for external application to skin or the composition(s) for external application to mucosa according to any one of claims 31 - 34, wherein the extracellular fluid concentrations of ginsenosides, metabolites thereof or salts thereof in the skin tissue or the mucosal tissues are 1 ng/ml or less.

36. The composition(s) for external application to skin or the composition(s) for external application to mucosa according to claim 35, wherein the concentrations of ginsenosides, metabolites thereof or salts thereof are 0.01 - 100 fg/ml or 1 - 10,000 fg/ml.

37. The composition(s) for external application to skin or the composition(s) for external application to mucosa according to claim 36, wherein the ginsenosides are ginsenoside Rb₁.

38. The composition(s) for external application to skin or the composition(s) for external application to mucosa according to any one of claims 31- 36, wherein ginsenosides, metabolites thereof or salts thereof are present in an extract(s) of a natural product(s), fraction(s) thereof or in purified fraction(s) thereof.

39. The composition(s) for external application to skin or the composition(s) for external application to mucosa according to claim 38, wherein the extract(s) of the natural product(s), fraction(s) thereof or purified fraction(s) thereof is ginseng, a ginseng extract(s) or a crude saponin fraction(s) of ginseng.

40. The composition(s) for external application to skin or the composition(s) for external application to mucosa according to claim 39, wherein the extracellular fluid concentrations of the crude saponin fraction(s) of ginseng, the ginseng extract(s), ginseng or metabolites thereof in the skin tissue or the mucosal tissues are 14.5 ng/ml or less.

41. The composition(s) for external application to skin or the composition(s) for external application to mucosa according to claim 40, wherein the concentrations of ginseng, the ginseng extract(s) or the crude saponin fraction(s) of ginseng are 0.145 - 1,450 fg/ml or 14.5 - 145,000 fg/ml.

42. The composition(s) for external application to skin or the composition(s) for external application to mucosa according to any one of claims 31 - 36, wherein ginsenosides, metabolites thereof or salts thereof are chemically modified derivatives from the natural product(s) by chemical means.

43. The-composition(s) for external application to skin or the composition(s) for external application to mucosa according to claim 42, wherein the chemically modified derivative is dihydroginsenoside Rb₁.

44. The composition(s) for external application to skin or the composition(s) for external application to mucosa according to any one of claims 31 - 43, wherein the composition(s) for external application to skin or the composition(s) for external application to mucosa is for protection of the skin cells or mucosal cells, or for prevention, treatment or improvement of senile symptoms of the skin or the mucosa.

45. The composition(s) for external application to skin or the composition(s) for external application to mucosa according to claim 44, wherein the senile symptoms of the skin or the mucosa are atrophy, shrinkage, dermatrophia, vulnerability to infection, easily infectivity, slackening, looseness, flabbiness, scurf, dandruff, depilation, alopecia, itching, roughness, gray hair, poliosis, cracks, rhagades, asteatosis, oligosteatosis, keratic cell ablation, exfoliation or ablation of keratinocytes, cornified cells, hornified cells or keratinized cells, exfoliation or ablation of the stratum corneum, corneum ablation, chapped, chapping, chloasma, lines, wrinkles, furrows, crease, spots, blotches, ephelides, sunburn, poor regeneration, aplasia, pigmentation, dryness of the skin or shrinkage, atrophy, exfoliation, ablation, epithelial cell exfoliation or ablation, poor regeneration, aplasia, chapped, cracks, rhagades, chapping or dryness of the mucosa.

46. A method for exploring or screening an active composition(s) for prevention, treatment or therapy of diseases of skin tissue or mucosal tissues, comprising applying ginsenosides or metabolites thereof as a leading compound(s).

47. The method according to claim 46, wherein the diseases of skin tissues or mucosal tissues are causing histopathological changes of skin tissue or mucosal tissues.

48. The method according to claim 46 or claim 47, wherein the diseases of skin tissue or mucosal tissues are incised wound, open wound or defect of the skin, or morsus of the mouth mucosal tissue.

49. A method for exploring or screening novel substances for promoting regeneration or reconstruction of tissues comprising using a target molecule of ginsenosides.

50. The method according to any one of claims 46 - 49, wherein ginsenosides are ginsenoside Rb₁ or dihydroginsenoside Rb₁.

51. A pharmaceutical composition(s) or a veterinary drug composition(s) for prevention, treatment or therapy of diseases of skin tissue or mucosal tissues, comprising containing substances explored by the methods according to claims 46 - 50.

52. Use of ginsenosides or metabolites thereof as a leading compound(s) for exploring or screening effective components or compounds for prevention, treatment or therapy of diseases of skin or mucosa.

53. Use of ginsenosides or metabolites thereof as a leading compound(s) for exploring or screening an agent(s) for promoting regeneration of tissues or an agent(s) for promoting reconstruction of tissues with histopathological changes.

54. Use according to claim 53, wherein the vital or viable tissues with histopathological changes are the skin with incised wound or open wound, or the mouth mucosa with morsus.

55. Use of ginsenosides, metabolites thereof or salts thereof for producing a pharmaceutical composition(s) or a veterinary drug composition for prevention, treatment or therapy of the organic diseases causing histopathological changes of the skin tissue or the mucosal tissues.

56. Use according to any one of claims 52 - 55, wherein ginsenosides are ginsenoside Rb₁ or dihydroginsenoside Rb₁.

57. A method for mass production of ginsenosides or metabolites thereof, comprising using cultured cells or plant stocks which produce ginsenosides.

58. A composition(s) for growth-regulation for promoting generation, regeneration, growth, reconstruction, differentiation, preservation, stock, nourishment or cultivation of tissues or cells of plants or animals, comprising ginseng, an extract(s) of ginseng, a crude saponin fraction(s) of ginseng, ginsenosides or salts thereof.

59. The composition(s) for growth-regulation according to claim 58, wherein the composition(s) for growth-regulation is a composition(s) for growth-regulation of plants for promoting generation, regeneration, growth, reconstruction, differentiation, preservation, stock, nourishment or cultivation of tissues or cells of plants or animals.

60. The composition(s) for growth-regulation of plants according to claim 59, wherein the composition(s) for growth-regulation of plants is a composition(s) of fertilizer.

61. The composition(s) for growth-regulation of plants according to claim 59 or claim 60, wherein tissues or cells of plants are hydroponic cultured plants.

62. The composition(s), for growth-regulation of plants according to claim 61, wherein the hydroponic cultured plant is a cutting of pothos.

63. The composition(s) for growth-regulation according to claim 58, wherein the composition(s) for growth-regulation is a composition(s) for growth-regulation of animals for nourishment, protection or culture of marine products, marine resources, aquatic products, fisheries resources, marine animals, aquatic animals, pets or livestock.

64. The composition(s) for growth-regulation according to claim 63, wherein the composition(s) for growth-regulation of animals is a composition(s) of feed.

65. The composition(s) for growth-regulation according to any one of claims 58 - 64, wherein ginseng, the ginseng extract(s), the crude saponin fraction(s) of ginseng or ginsenoside(s) is a natural liquid extract(s) or solid extract(s).

66. The composition(s) for growth-regulation according to any one of claims 58 - 65, wherein ginsenosides are ginsenoside Rbi or dihydroginsenoside Rb₁.

67. A method for exploring or screening novel substances or compounds for promoting regeneration, generation, rooting, budding, growth, differentiation or reconstruction of the vital or viable tissues, comprising assaying protective action of an intracerebroventricularly administered substance(s) to be tested on brain cells or nerve cells.

68. The method according to claim 67, wherein the protective action on the brain cells or the nerve cells is assayed when the extracellular fluid concentrations of the intracerebroventricularly administered substance(s) to be tested are 1 ng/ml or less or 0.5 nM or less.

69. The method according to claim 67, wherein the extracellular fluid concentrations of the intracerebroventricularly administered substance(s) to be tested are 0.01 - 100 fg/ml or 0.005 - 50 fM, or 1 - 10,000 fg/ml or 0.5 - 5,000 fM.

70. A pharmaceutical composition(s) or a veterinary drug composition(s) for promoting regeneration, generation, growth, differentiation or reconstruction of the vital or viable tissues comprising containing a compound(s) or salt thereof, which exhibited protective action on brain cells or nerve cells by intracerebroventricular administration performed by the method according to any one of claims 67 - 69.

71. A composition(s) for external application to skin for promoting regeneration, generation, growth, differentiation or reconstruction of the vital or viable tissues, comprising containing a compound(s) or salt thereof which exhibited protective action on brain cells or nerve cells by intracerebroventricular administration performed by the method according to any one of claims 67 - 69.

72. A composition(s) for growth-regulation for promoting regeneration, generation, rooting, budding, growth, differentiation or reconstruction of the vital or viable tissues, comprising containing a compound(s) or salt thereof which exhibited protective action on brain cells or nerve cells by intracerebroventricular administration performed by the method according to any one of claims 67 - 69.
